# EUROPEAN PATENT APPLICATION

(11) **EP 2 298 350 A2**
(43) Date of publication of application: **23.03.2011**
(21) Application number: 10184726.7
(22) Date of filing: 28.07.1997
(51) Int. Cl.: A61K 45/06, A61K 31/52, A61K 31/195, A61K 31/70, A61K 31/19

(54) **Composition comprising an inducing agent and an anti-viral agent for the treatment of viral disorders**

(30) Priority: 26.07.1996 US 687670; 26.07.1996 US 687671
(62) Divisional of application: 07014281.5
(71) Applicant: Perrine, Susan P., Weston, MA 02193 (US); Faller, Douglas V., Weston, MA 02193 (US); White, Brian F., Fresno, CA 93772 (US)
(72) Inventor: Perrine, Susan P., Weston, MA 02193 (US); Faller, Douglas V., Weston, MA 02193 (US); White, Brian F., Fresno, CA 93772 (US)
(74) Representative: Wilson, Justin Scott

(57) **Abstract**

The invention relates to novel compositions and to novel methods for the administration of compositions to a patient or to cells *in vitro* for the treatment of human disorders. In one embodiment, compositions contain chemical compounds that stimulate the expression of fetal hemoglobin and/or stimulate the proliferation of red blood cells, white blood cells and platelets in patients and *ex vivo* for reconstitution of hematopoiesis *in vivo*. These methods are useful to treat or prevent the symptoms associated with anemia, sickle cell disease, thalassemia and other blood disorders. In another embodiment, compositions contain an inducing agent and an anti-viral agent. The inducing agent induces the expression of a cellular or viral product, such as viral thymidine kinase, increasing the sensitivity of proliferating cells to the anti-viral agent. Typical anti-viral agents include nucleoside analogs such as ganciclovir that inhibit viral replication. Methods involve administration of therapeutically effective amounts of the inducing agent with the anti-viral agent to destroy virus-infected cells. Viral infections that can be treated include infections by herpes viruses such as Kaposi's-associated herpes virus and Epstein-Barr virus, HIV infections and HTLV infections. These compositions and methods are particularly effective against episomal and latent infections in proliferating cells. The invention also relates to novel methods for the pulsed administration of compositions to patients for the treatment and prevention of cell proliferative disorders including deficiencies such as cytopenia and malignancies such as viral-induced tumors, other forms of neoplasia and for expansion of cells for hematopoietic transplantation.

## Description

### Background of the Invention

### 1. Field of the Invention

The invention relates to pharmaceutical compositions that contain novel chemical compounds and to novel methods for administering these compositions. Compositions are useful for for the treatment and prevention of blood disorders virus-associated disorders and cellular disorders such as neoplastic malignancies. Compositions stimulate the expression of a specific gene such as a globin gene or a virus-associated gene that renders the infected-cell susceptible to further therapeutic treatment. Such compositions can be administered in predetermined pulses which increases therapeutic effectiveness.

### 2. Description of the Background

### Blood Disorders

The major function of red blood cells is to transport oxygen to tissues of the body. Minor functions include the transportation of nutrients, intercellular messages and cytokines, and the absorption of cellular metabolites. Anemia, or a loss of red blood cells or red blood cell capacity, can be grossly defined as a reduction in the ability of blood to transport oxygen. Anemia can be measured by determining a patient's red blood cell mass or hematocrit. Hematocrit values are indirect, but fairly accurate measures of the total hemoglobin concentration of a blood sample. Anemia, as measured by a reduced hematocrit, may be chronic or acute. Chronic anemia may be caused by extrinsic red blood cell abnormalities, intrinsic abnormalities or impaired production of red blood cells. Extrinsic or extra-corpuscular abnormalities include antibody-mediated disorders such as transfusion reactions and erythroblastosis, mechanical trauma to red cells such as micro-angiopathic hemolytic anemias, thrombotic thrombocytopenic purpura and disseminated intravascular coagulation. In addition, infections by parasites such as *Plasmodium,* chemical injuries from, for example, lead poisoning, and sequestration in the mononuclear system such as by hypersplenism can result in red blood cell disorders and deficiencies.

Impaired red blood cell production can occur by disturbing the proliferation and differentiation of the stem cells or committed cells. Some of the more common diseases of red cell production include aplastic anemia, hypoplastic anemia, pure red cell aplasia and anemia associated with renal failure or endocrine disorders. Disturbances of the proliferation and differentiation of erythroblasts include defects in DNA synthesis such as impaired utilization of vitamin B₁₂ or folic acid and the megaloblastic anemias, defects in heme or globin synthesis, and anemias of unknown origins such as sideroblastic anemia, anemia associated with chronic infections such as malaria, trypanosomiasis, HIV, hepatitis virus or other viruses, and myelophthisic anemias caused by marrow deficiencies.

Intrinsic abnormalities include both hereditary and acquired disorders. Acquired disorders are those which have been induced through, for example, a membrane defect such as paroxysmal nocturnal hemoglobinuria. Hereditary disorders include disorders of membrane cytoskeleton such as spherocytosis and elliptocytosis, disorders of lipid synthesis such as an abnormally increased lecithin content of the cellular membrane, red cell enzyme deficiencies such as deficiencies of pyruvate kinase, hexokinase, glutathione synthetase and glucose-6-phosphate dehydrogenase. Although red blood cell disorders may be caused by certain drugs and immune system disorders, the majority are caused by genetic defects in the expression of hemoglobin. Disorders of hemoglobin synthesis include deficiencies of globin synthesis such as thalassemia syndromes and structural abnormalities of globin such as sickle cell syndromes and syndromes associated with unstable hemoglobins.

Mammalian globin gene expression is highly regulated during development. The basic structure of the α and β globin genes are similar as are the basic steps in synthesis of a and β globin. There are at least five human a globin genes located on chromosome 16 including two adult α globin genes of 141 amino acids that encode identical polypeptides which differ only in their 3'-untranslated regions, one embryonic α gene, zeta (ζ), and at least two pseudo-alpha genes, psi zeta (ψζ) and omega alpha (ωα). The human β globin gene cluster includes one embryonic gene, epsilon (ε), two adult beta globin genes, beta (β) and delta (δ), two fetal beta globin genes G-gamma (G-γ) and A-gamma (Ay), which differ by only one amino acid, and at least one pseudo-beta gene, psi beta (ψβ). All are expressed from a single 43 kilobase segment of human chromosome 11 (E.F. Fritsch et al., Nature 279:598-603, 1979).

Hemoglobin A comprises four protein chains, two alpha chains and two beta chains (α₂ β₂), interwoven together, each with its own molecule of iron and with a combined molecular weight of about 68 kD. The hemoglobin macromolecule is normally glycosylated and upon absorbing oxygen from the lungs transforms into oxyhemoglobin (HbO₂). There are at least six distinct forms of hemoglobin, each expressed at various times during development. Hemoglobin in the embryo is found in at least three forms, Hb-Gower 1 (ζ₂ε₂), Hb-Gower 2 (α₂ε₂), and Hb-Portand (ζ₂γ₂). Hemoglobin in the fetus comprises nearly totally HbF (α₂γ₂), whereas hemoglobin in the adult contains about 96% HbA (α₂ β₂), about 3% HbA₂ (α₂ δ₂) and about 1% fetal HbF (α₂ γ₂). The embryonic switch of globin expression from ζ to α and from e to γ begins in the yolk sac. However, chains of embryonic ζ and e have been found in the fetal liver and complete transition to the fetal form does not occur until late in fetal development. The fetal switch from γ to P begins later in erythropoiesis with the amount of γ globin produced increasing throughout gestation. At birth, β globin accounts for about 40% of non-α globin chain synthesis and thereafter continues to rapidly increase. Neither the switch from embryonic to fetal or fetal to adult appears to be controlled through cell surface or known cytokine interactions. Control seems to reside in a developmental clock with the switch occurring at times determined only by the stage of fetal development.

Defects or mutations in globin chain expression are common. Some of these genetic mutations pose no adverse or only minor consequences to the person, however, most mutations prevent the formation of an intact or normal hemoglobin molecule through a functional or structural inability to effectively bind iron, an inability of the chains or chain pairs to effectively or properly interact, an inability of the molecule to absorb or release oxygen, a failure to express sufficient quantities of one or more globin chains or a combination of these malfunctions. For example, substitutions of valine for glutamic acid at the sixth position of the P chain produces HbS and was found to occur in about 30% of black Americans. In the HbS heterozygote, only about 40% of total hemoglobin is HbS with the remainder being the more normal HbA.

Upon deoxygenation, HbS molecules undergo aggregation and polymerization ultimately leading to a morphological distortion of the red cells which acquire a sickle or holly-leaf shape. Sickling has two major consequences, a chronic hemolytic anemia and an occlusion of small blood vessels that results in ischemic damage to tissues. Further, when exposed to low oxygen tensions, polymerization converts HbS hemoglobin from a free-flowing liquid to a viscous gel. Consequently, the degree of pathology associated with sickle cell anemia can be correlated with the relative amount of HbS in the patient's system.

The course of sickle cell disease is typically punctuated with a variety of painful crises called vaso-occlusive crises. These crises represent episodes of hypoxic injury and infarction in the organs, abdomen, chest, extremities or joints. Leg ulcers are an additional manifestation of the vaso-occlusive tendency of this disease. Central nervous system involvement is common producing seizures and even strokes. Aplastic crises, also common, represent a temporary cessation of bone marrow activity and may be triggered by infections, folic acid deficiency or both. Crises are episodic and reversible, but may be fatal. Damage from crisis episodes tends to be cumulative and even in those individuals with milder forms of sickle cell disorders, life-spans can be greatly reduced. Absent alternative intervention, patients typically die before the age of 30.

The thalassemia syndromes are a heterogenous group of disorders all characterized by a lack of or a decreased synthesis of the globin chains of HbA. Deficiencies of β-globin expression are referred to as β-thalassemias and deficiencies of α-globin, α-thalassemias. The hemolytic consequences of deficient globin chain synthesis result from decreased synthesis of one chain and also an excess of the complementary chain. Free chains tend to aggregate into insoluble inclusions within erythrocytes causing premature destruction of maturing erythrocytes and their precursors, ineffective erythropoiesis, and the hemolysis of mature red blood cells. The underlying defects of hemoglobin synthesis have been elucidated over the years and largely reside in the nucleic acid sequences which express or control the expression of α or β globin protein.

Fetal β-type globin, or γ globin, is expressed in the earliest stages of mammalian development and persists until about 32 to 34 weeks of gestation. At this stage, the adult forms of β globin begin to be expressed and substitute for the fetal proteins. Studies correlating clinical hematological results with the locations of various mutations that correspond to switching indicate that a region located upstream of the 5'-end of the δ-gene may be involved in the cis suppression of γ-gene expression in adults (E.F. Fritsch et al., Nature 279:598-603, 1979). The reason for this switch from fetal to adult protein is unknown and does not appear to provide any significant benefit to the adult.

Genotypic variation in healthy individuals have been identified wherein adult P globin is not formed, but severe complications are avoided. These patients constituitively express fetal or γ globin protein in amounts sufficient to substitute for the missing β globin protein. This hereditary persistence of fetal hemoglobin (HPFH) may involve one or both of the fetal β-globin genes, A-γ and G-γ. Apparently, consistent production of either γ-globin protein accomplishes the necessary functions, at least in the short term, of the abnormal or missing β-globin protein (R. Bernards et al., Nuc. Acids Res. 8:1521-34, 1980).

A variety of small molecules have been shown to effect hemoglobin or fetal globin expression. Early experiments demonstrated that acetate (CH₃COOH), propionate (CH₃CH₂COOH), butyrate (CH)CH₂CH₂COOH) and isobutyrate (CH₃CH(CH₃)COOH) all induced hemoglobin synthesis in cultured Friend leukemia cells (E. Takahashi et al., Gann 66:577-80, 1977). Additional studies showed that polar compounds, such as acid amides, and fatty acids could stimulate the expression of both fetal and adult globin genes in murine erythroleukemia cells (U. Nudel et al., Proc. Natl. Acad. Sci. USA 74:1100-4, 1977).

One of the major breakthroughs in the treatment of hemoglobinopathies was made when it was discovered that butyric acid (butanoic acid; CH₃CH₂CH₂COOH) accurately and specifically stimulated transcription of the human fetal (γ) globin gene (G.A. Partington et al., EMBO J. 3:2787-92, 1984). These findings were quickly confirmed *in vivo* wherein it was shown that pharmacological doses of butyric acid greatly increased expression of fetal globin in adult chickens rendered anemic by injections with phenylhydrazine (G.D. Ginder et al., Proc. Natl. Acad. Sci. USA 81:3954-58, 1984). Although promising in pilot clinical studies, treated patients were unable to maintain adequate levels of fetal globin in their system. It was later determined that many of forms of butyric acid had extremely short-half lives. Oxidation in the serum, clearance by hepatocytes and filtration through the kidneys rapidly eliminated these agents from the patient's system. Patients also rapidly developed tolerance or metabolites of compounds had the opposite desired effect.

Other methods to increase fetal globin expression have focused on recruitment and reprogramming of erythroid progenitor cells to increase total globin expression. For example, the hematopoietic growth factor erythropoietin (EPO) was found to be a potent, although not a fetal-specific, reticulocyte stimulator (Al-Khatti et al., Trans. Assoc. Am. Physicians 101:54, 1988; G.P. Rodgers et al., N. Engl. J. Med. 328:73-80, 1993). In one experiment, animals were treated with EPO following a specific course of therapy (U.S. 4,965,251). According to this experiment, a high dose of erythropoietin was administered in a first time period followed by a second time period wherein erythropoietin was withheld. Following this regimen of treatment, typical for a cytokine, F-reticulocyte colonies cultured from samples obtained from two chronically-anemic baboons increased from 6-8% and 20% pre-treatment to 23% and 50% post-treatment, respectively.

These methods were somewhat advantageous to artificially phlebotomized baboons, but would be entirely counter-productive to patients with a hemoglobinopathy. As thalassemic patients express high levels of EPO, supplemental treatments with EPO would have little to no effect. Sickle cell patients and other patients with unstimulated levels would also not benefit from supplemental EPO treatments because absolute amounts of both α-globin and non α-globin would increase. Defective non-α globin such as thalassemic forms of β-globin, would seriously interfere with formation of hemoglobin proteins and, consequently, oxygen transport. Any beneficial effects attributable to increased fetal hemoglobin would be negated as supplemental treatments with EPO would increase both the frequency and number of sickle cell crises, both of which are to be avoided in such patients.

Other hematopoietic growth factors, such as granulocyte/macrophage-colony stimulating factor (GM-CSF) and interleukin 3 (IL-3), were also tested *in vivo* or *in vitro* for the ability to stimulate F-reticulocytes (M. Giabbianelli et al., Blood 74:2657, 1989; A.R. Migliaccio et al., Blood 76:1150, 1990). Both of these factors were found to non-specifically increase fetal globin synthesis in tissue culture cells.

### Viral and Cellular Disorders

A growing number of cellular disorders such as neoplastic malignancies have been found to contain viral genetic sequences or virus particles in the anomalous cells. For a large number of these disorders, the presence of the virus is believed to be causative or at least contributory instrument. Representative members of many of the known families of viruses have been found in such cells including members of the herpes family of viruses, the polyomaviruses and the hepatitis viruses.

Epstein-Barr virus (EBV), a 172 kb herpes virus, is often found intimately associated with both mature and immature B cells and is believed to be involved to some degree in infectious mononucleosis, African Burkitt's lymphoma (BL) and nasopharyngeal carcinoma. EBV undergoes lytic replication after initial infection of oropharyngeal epithelia. The linear form genome is duplicated, packaged into the viral capsid and extruded from the cell by budding or lysis. One hundred viral proteins are synthesized during this lytic stage of the virus life cycle. In contrast, normal B cells incubated with EBV *in vitro* are efficiently immortalized and develop into continuously growing lymphoblastoid cell lines (LCLs). The cellular events that regulate these distinct outcomes are as yet unclear.

In immortalized cells, the genome circularizes, amplifies and replicates coordinate with, and dependent upon, cell division. Because no viral particles are produced, infection is considered to be latent and EBV persists in the cells for life. Outgrowth of latently infected B cells is prevented by T cell immune surveillance. In immortalizing latent infection, only 11 gene products are detected, including 6 nuclear antigens (EBNA-1, -2, -LP, -3A, -3B, -3Q), 3 membrane proteins (LMP-1, LMP-2A, LMP-2B) and two small, non-poly(A) RNAs (EBER-1 and EBER-2) (G. Miller et al., Epstein-Barr Virus: Biology, Pathogenesis and Medical Aspects, Raven Press, N. Y. 1990). In EBV(+) tumors such as Burkitt's lymphoma, neoplastic genetic events have often superseded the requirement for viral immortalizing functions, and gene expression may be limited to EBNA-1 (M. Rowe et al., EMBO J. 6:2743-51, 1987). Virus tropism is determined by complement receptor type 2 which mediates attachment of the envelope protein gp350/2204 to B and some T lymphocytes, follicular dendritic cells and epithelial cells.

EBV is a common and worldwide pathogen. Childhood infection is asymptomatic. About 50% of individuals with delayed exposure develop a self-limited, lymphoproliferative syndrome referred to as infectious mononucleosis. EBV is also detected in 2 endemic tumors, African Burkitt's lymphoma (BL) (G. Henle et al., Proc. Natl. Acad. Sci. USA 58:94-101, 1985) and nasopharyngeal carcinoma (NPC) (W. Henle et al., Adv. Viral Onco. 5:201-38, 1985), as well as gastric carcinoma, breast cancers and sarcomas. Recently, some T-cell and B-cell lymphomas, as well as about 50% of Hodgkin's lymphomas have been found to contain EBV (L.M. Weiss et al., N. Engl. J. Med. 320:502, 1989).

African Burkitt's lymphoma is characterized by rapid growth of the tumor at non-lymphoid sites such as the jaw or the retroperitoneum. The tumor is of B cell origin and is closely related to the small noncleaved cells of normal lymphoid follicles. Biopsy specimens from African Burkitt's lymphoma invariably contain the EBV genome and are positive for EBNA (I. Magrath, Epstein-Barr Virus and Associated Diseases, pp. 631-43, M. Ninjhoff Publishing, Boston, 1986). This contrasts with the non-African Burkitt's lymphoma, in which only 15% to 20% of the tumors contain the EBV genome. EBV has a worldwide distribution and infects most (more than 90%) individuals before adulthood. The clustering of Burkitt's lymphoma in the equatorial belt of East Africa remains unexplained. It has been hypothesized that alterations of the immune system, possibly due to hyperstimulation by endemic malaria, may play an important role in the outcome of an EBV infection to individuals in this region (D.J. Moss et al., Int. J. Cancer 31:727-32, 1983). Individuals from this region show impairment in virus-specific cytotoxic T-cell activity. Normally, it is the T-cell response to EBV infection that limits B-cell proliferation, and this T-cell response is directly stimulated by EBV (H. zur Hausen et al., Nature 228:1056-58, 1970). It has been postulated that the failure of the T-cell immune response to control this proliferation could lead to excessive B-cell proliferation and, as such, provide a suitable background for further mutation, oncogenic transformation, and lymphomagenesis.

A scenario has been suggested for the involvement of EBV in the etiology of African Burkitt's lymphoma (G. Klein, Proc. Natl. Acad. Sci. USA 76:2442-46, 1979). The first step involves the EBV-induced immortalization of B lymphocytes in a primary infection. The second step involves the stimulated proliferation of EBV(+) B cells. This step is facilitated in the geographic areas where Burkitt's lymphoma is endemic (presumably because of the presence of malaria), through B-cell triggering and the suppression of T-cells involved in the control of the proliferation of EBV-infected cells. This pool of cells becomes increased in size as a target cell population for random chromosomal rearrangements. The third and final step is the reciprocal translocation involving a chromosomal locus with an immunoglobulin gene and the *c-myc* gene on chromosome 8. This leads to the deregulation of the *c-myc* gene, to the development of the malignant clone and to the appearance of a tumor mass (G. Klein et al., Nature 315:190, 1985) Alternative scenarios have been proposed in which the order of the steps are rearranged such that the B-cell activation by malaria precedes the chromosomal translocation and is followed by EBV infection. Regardless, the components of these two scenarios each account for the geographic distribution of Burkitt's lymphoma, the critical involvement of EBV in lymphomagenesis, and the eventual selection and clonal outgrowth of a population of cells with the critical translocation involving the deregulation of the *c-myc* gene on chromosome 8.

For more than 20 years, a role for EBV in the pathogenesis of Hodgkin's disease (HD) was postulated based on epidemiologic evidence linking Hodgkin's patients with EBV seropositivity and elevated EBV titers (A.S. Evans et al., Int. J. Cancer 34:149, 1984). A number of studies have found an increase (2-5 fold) in the incidence of HD after infectious mononucleosis. However, some Hodgkin's patients were seronegative for EBV and the association between EBV and Hodgkin's disease remained speculative until 1987. In that year, molecular genetic analysis demonstrated that some Hodgkin's tissues contained monoclonal EBV DNA and that the virus was localized to Reed-Sternberg (RS) cells (the malignant cells in HD). Subsequent immunohistochemical and serologic data support an association between EBV and Hodgkin's disease and confirm the localization of the virus to cytologically malignant-appearing RS cells and variants (N.M. Jiwa et al., Histopathology 21:51, 1992). EBV also infects variable numbers of small B and T lymphocytes in the reactive inflammatory cell infiltrate that composes the bulk of Hodgkin's tissues (L.M. Weiss et al., Am. J. Path. 139:1259, 1991).

Clonal and non-clonal EBV genomes are present in Hodgkin's disease. Expression of the oncogene LMP (latent membrane protein) is seen in RS cells. In HD, the region of the (viral) BNLF1 oncogene coding for the amino terminal and transmembrane domains (associated with oncogenic function) of LW appears to be homogeneous, whereas the region coding for the intracytoplasmic (carboxyl terminal) domain of LMP is heterogeneous. Cytological similarities between RS cells and immunoblasts of known EBV-induced infectious mononucleosis and EB V induced AIDS-related lymphomas are consistent with the hypothesis that the EBV-BNLFI, oncogene is an inducer of morphological features of RS cells. Whether chromosomal integration of EBV DNA is an important factor in activation of such a transforming activity remains to be elucidated. Therefore the RS cells appear to be derived from lymphocytes beyond the pre-B-cell or common thymocyte stage, which may or may not subsequently become infected by EBV.

The high prevalence of EBV in Hodgkin's disease implies an etiologic role for the virus in Hodgkin's tumorigenesis. This pathogenetic theory is supported by the monoclonality of EBV DNA in these tumors (M.L. Gulley et al., Blood 83:1595-602, 1994). In one series, monoclonal EBV DNA was detected in all 17 cases having EBNAI-positive RS cells. Because tumor-associated viral DNA is monoclonal, it is likely that virus infection preceded clonal expansion. This reinforces the hypothesis that the virus is not an innocent bystander, but rather plays a role in the pathogenesis of the Hodgkin's disease and the other tumor types in which it is found (A. Neri et al., Blood 77:1092, 1991). The observation of EBNA1 expression in the RS cells of clonally-infected cases indicates that the clonal virus is localized to these cells and suggests that Hodgkin's disease results from the transformation of an EBV-competent cell. Other studies suggest that this virus is modulating rather than an etiologic agent in a considerable proportion of HD cases.

Investigations into the biology of EBV infection have shown that only one viral particle successfully infects a given cell. Once the viral DNA is established inside the cell, it circularizes and reproduces itself to yield multiple identical copies of viral DNA (E.A. Hurley et al., J. Exp. Med. 168:2059, 1988). In this way, tumors derived from infected cells can have multiple copies of EBV per cell, while maintaining clonal viral DNA structure. The average amount of clonal EBV DNA in Hodgkin's disease tissues varied from 0.5 to 5 copies per cell. Because RS cells comprised only a small fraction (<1%) of all HD tissue cells, the content of EBV DNA in each RS cell is estimated to be at least 100 times higher than the measured average copy number per cell, or at least 50 copies of viral DNA per RS cell. This is comparable with, or greater than, the viral burden in infected non-Hodgkin's lymphomas. The high copy number of EBV in RS cells may relate to the pathobiology of this complex lymphomatous disorder. In agreement with these studies, EBV DNA is abundant and monoclonal in infected RS cells. The presence of EBV in RS cells was strongly and independently linked to mixed cellularity histology and Hispanic ethnicity.

Clonally-integrated EBV is found in association with T-cell lymphomas as well as B-cell lymphomas. Currently, three populations of tissue-restricted T lymphocytes have been recognized, mucosa-associated, cutaneous and nodal T lymphocytes. T-cell lymphomas arising from different sites, but with similar morphology may show differences in lymphomagenesis and in expression of oncogenies, adhesion molecules, presence of certain DNA/RNA viral sequences and in clinical presentation and behavior.

Primary cutaneous CD30(+) large cell, T-cell lymphomas often remain localized to the skin for a long time, express a unique cutaneous lymphocyte antigen (CLA), known as the skin-homing receptor, have been postulated to be associated with the presence of human T-cell leukemia/lymphoma virus type I (HTLV 1), and have a good clinical course (R.C. Beljaards et al., Cancer 71:2097, 1993). In contrast, morphologically similar T-cell lymphomas of nodal origin often behave more aggressively, are CLA-negative, and have been associated with the presence of EBV (P.C. de Bruin et al., Histopathology 23:127, 1993). There was no relation between primary cutaneous T-cell lymphoma and EBV.

Infection of T cells by EBV most likely occurs via CR2 or CR2-like receptors (C.D. Tsoukas et al., Immunol. Today 14:56, 1993). The close contact between T cells and the upper respiratory tract epithelium, known for its reservoir function for EBV, probably make T cells in this region more vulnerable for EBV infection. The finding that EBV can be found in almost all tumor cells in most cases of primary extra-nodal, and especially nasal, T-cell lymphoma, in contrast to primary nodal T-cell lymphoma, where the number of EBV-infected neoplastic cells varies greatly between the cases argues for an etiologic role for EBV in these cases. Moreover, these cases often express LMP-1, known for its transforming and oncogenic properties *in vitro* and are reported to be monoclonal for EBV (I. Su et al., Blood 77:799, 1991). Thus, there are site-restricted etiologic differences between morphologically identical T-cell lymphomas, of which EBV might be one of many factors.

EBV-induced lymphoproliferative disease or lymphoma has an immunodeficiency incidence in U.S. of about 10,000 B-cell, EBV(+) lymphoma patients per year. EBV is very commonly associated with lymphomas in patients with acquired or congenital immunodeficiencies. These lymphomas can be distinguished from the classical Burkitt's lymphomas in that the tumors may be polyclonal. Tumors also do not demonstrate the characteristic chromosomal abnormalities of Burkitt's lymphoma described earlier. The pathogenesis of these lymphomas involves a deficiency in the effector mechanisms needed to control EBV-transformed cells. The rototypic model for this disease has been the X-linked lymphoproliferative (XLP) syndrome (D.T. Purtilo et al., Am. J. Med. 73:49-56, 1982). Patients with XLP who develop acute infectious mononucleosis exhibit the usual atypical lymphocytosis and polyclonal elevation of serum immunoglobulins and increases in specific antibody to VCA and to EA. During these infections, patients with XLP fail to mount and sustain an anti-EBNA response after acute EBV infection. The unique vulnerability of males with XLP to EBV infection is most likely due to an inherited immune regulatory defect that results in the failure to govern the cytotoxic T cells and NK cells required to cope with EBV.

Patients with iatrogenic immunodeficiencies, such as organ transplant recipients, are also at an increased risk for lymphomas, and these lymphomas often contain EBV DNA and EBNA. Also, patients with AIDS are at a higher risk for developing polyclonal lymphomas associated with EBV.

EBV-induced lymphomas are associated with immunosuppression. EBV-associated lymphoproliferative disease (EBV-LPD) is characterized by actively proliferating EBV(+) B-cells, frequently without overt malignant change. These immunoblastic lymphoma-like lesions have been identified in a variety of transplant patients, in patients with congenital immunodeficiency, and in patients infected with HTV (J.I. Cohen, Medicine 70:137-60, 1991). These so called post-transplant lymphoproliferative disorders (PTLD) are observed after all transplants, including kidney, bone marrow, heart, liver and lung transplantation. This increased incidence of EBV-LPD in this setting is likely due to the aggressive immunosuppression required after these transplants.

### Summary of the Invention

The invention overcomes the problems and disadvantages associated with current strategies and designs and provides novel compositions containing novel chemical agents and novel methods for the treatment and prevention of blood disorders, virus-asociated disorders, cellular disorders and neoplasia.

One embodiment of the invention is directed to pharmaceutical compositions that comprise novel inducing agents. Inducing agents include cytokines or chemicals such as arginine butyrate or isobutyramide. These agents induce expression of certain gene products and the proliferation of specific cell populations. Gene products that can be induced by agents of the invention include fetal hemaglobin and viral proteins such as viral thymidine kinase of EBV-infected cells. Cell populations that can be induced include, for example, hemaglobin-expressing cells, myeloid cells and megakaryocytes. Expression of these products and cell populations can be used in the treatment and prevention of blood, cellular and viral disorders. Compositions may further comprise anti-viral agents which, for example, in combination with certain inducing agents destroy virus-infected cells. Effective anti-viral agents which can be used include substrate analogs such as nucleoside analogs, polymerase inhibitors and transcriptase inhibitors. Cells that demonstrate induced expression or proliferation are targeted and destroyed.

Another embodiment of the invention is directed to methods for the treatment of viral infections by treating infected mammals with a combination of an inducing agent and an anti-viral agent. The inducing agent induces a uniquely viral-process and the anti-viral agent targets induced cells for destruction. These methods are counter to conventional therapies that require either an antigenic viral expression, for elimination by the host immune system, or a suppression of viral activity in all forms. Disorders that can be successfully treated include mononucleosis, CMV retinitis and pulmonary disease.

Another embodiment of the invention is directed to methods for the treatment of cell-proliferative disorders resulting from viral infections. Treatment comprises administering a combination of an inducing agent and an anti-viral agent to infected mammals. The inducing agent induces a uniquely viral-process in the proliferating cells and the anti-viral agent specifically targets those cells for destruction. Disorders that can be successfully treated include viral-induced neoplasia such as certain B and T cell lymphoproliferative disorders, Burkitt's lymphoma, leukemias and other cell malignancies.

Another embodiment of the invention is directed to methods for the treatment of viral disorders including viral infections and virally-induced cell proliferative disorders. Treatment comprises administering a combination of an activator and an anti-viral agent to infected mammals. Activators activate latent virus integrated into the infected or proliferating cells and the anti-viral agent specifically targets those cells for destruction. Disorders that can be successfully treated include latent infections such as infection by Kaposi's-associated human herpes virus, or human herpes virus type 8, human immunodeficiency virus and human T-cell leukemia/lymphoma virus.

Another embodiment of the invention is directed to methods for the treatment of blood disorders and other maladies such as neoplasia by administering compositions to a patient in pulses. Pulse therapy according to the methods of the invention is much more effective than continuous therapy. The effective dose as well as the total amount of composition needed by the patient to be therapeutically effective is decreased as compared to amounts required for similar effect with continuous therapy. Further, as most chemical compositions are non-toxic at all effective doses, pulsed administration can be continued for very long periods with no adverse effects to the patient.

Another embodiment of the invention is directed to methods for the stimulation of cell proliferation by the administration of erythropoietin or other cell stimulatory agent to a patient and the administration of a chemical composition of the invention in pulses. Such a treatment regimen prepares bone marrow cells for stimulation and increases overall hemoglobin expression and production in the body.

Another embodiment of the invention is directed to novel chemical compounds such as 2,2-dimethyl and 2,2-diethyl butyrate, o-benzoyl lactate, n-dimethylbutyrate glycine amide, o-dimethyl butyrate lactate, 3-phenyl butyrate, 4-chloro-2-phenoxy-2-propionic acid and choline salts of these and other compounds, and to methods for administering these compositions that can be used, either with or without pulsing, for the treatment of blood and other disorders such as neoplasia.

Other objects and advantages of the invention are set fourth in part in the description which follows, and in part, will be obvious from this description, or may be learned from the practice of the invention.

### Description of the Drawings

- Figure 1: Comparison of fetal globin produced in (A) normal and (B) sickle cell erythroid progenitor colonies developing in the presence or absence of test compounds.
- Figure 2: Effects of tested compounds on erythroid colony (Bfu-e) proliferation in cultures from (A) a normal individual, (B) a sickle cell patient, and (C) fetal liver stem cells.
- Figure 3: Comparison of (A) pulse treatment of a baboon with phenoxyacetic acid verses (B) continuous treatment with EPO.
- Figure 4: Comparison of (A) conventional verses (B) pulse treatments.
- Figure 5: Sustained response observed comparing (A) continuous verses (B) pulse therapy.
- Figure 6: Total hemoglobin levels in a patient on continuous therapy.
- Figure 7: Total hemoglobin levels in a patient switched from continuous to pulse therapy.
- Figure 8: α, β and γ globin levels in a patient (A) before treatment, (B) at moderate dose treatment, and (C) at high dose treatment.
- Figure 9: Ferritin levels of a patient under therapy.
- Figure 10: EBV-TK gene induction by arginine butyrate.
- Figure 11: Inhibition of growth of EBV+tumor lines by (a) 0.5 mM and (b) 2.0 mM arginine butyrate and GCV.
- Figure 12: Growth curve of the Akata cell line in different doses of arginine butyrate.
- Figure 13: Growth curve of the Akata cell line in the presence of GCV.
- Figure 14: Comparison of the growth curves of the Akata cell line in the presence of arginine butyrate, GCV and both arginine butyrate and GCV.
- Figure 15: HIV p24 levels, assayed by radio-immuno assay, in PBMCs and Cil ls isolated 72 hours post-cultivation in arginine butyrate.
- Figure 16: Pharmakinetics of arginine butyrate infusion.
- Figure 17: Inhibition of EBV+ tumor cell proliferation in cells freshly isolated from a patient with LPD.
- Figure 18: Prior cell death with combination therapy on day 3 in cells freshly isolated from a patient with LPD.

### Description of the Invention

As embodied and broadly described herein, the present invention is directed to novel chemicals and pharmaceutical compositions comprising these and other chemical compositions that can be used in the treatment and prevention of diseases and disorders. The invention is further directed to methods for the administration of pharmaceutical compositions of the invention to patients for the treatment and prevention of cell proliferative disorders such as malignancies and cytopenias, blood disorders such as an anemia, sickle cell syndrome and thalassemia, and virus-associated disorders such as latent virus infections.

### Compositions of the Invention

One embodiment of the invention is directed to pharmaceutical compositions comprising one or more novel chemical agents. Agents of the invention include chemicals of the structure R₁-R₂-R₃ or, preferably, R₁-C(O)-R₂-R₃ wherein R, is CHₓ, Hₓ, NHₓ, OHₓ. SHₓ, COHₓ, CONHₓ, COOH or COSHₓ; R₂ is CHₓ or a branched or linear alkyl chain; R₃ is CONHₓ, COSHₓ, COOH, COOR₄, COR₄ or OR₄; R₄ is CHₓ, Hₓ, NHₓ, OHₓ, SHₓ or a branched or linear alkyl chain; phenyl-R₅-R₆-R₇ wherein phenyl is a six carbon benzyl ring or a hydrogenated, hydroxylated or halogenated six carbon ring; R₅ is CHₓ, NHₓ, OHₓ or SHₓ; R₆ is CHₓ, NHₓ, OHₓ, SHₓ or a branched or linear alkyl chain; R₇ is CHₓ, Hx, NHₓ, OHₓ, SHₓ, CONHₓ, COOH, COSHₓ, COOR₈, COR, or OR₈; R₈ is CHₓ, Hₓ, NHₓ, OHₓ, SHₓ or a branched or linear alkyl chain; and phenyl-R₉-R₁₀ wherein R₉ is CHₓ, NHₓ, OHₓ, SHₓ or a branched or linear alkyl chain; R₁₀ is CHₓ, Hₓ, NHₓ, OHₓ, SHₓ, CONHₓ, COOH, COSHₓ, COOR₁₁, COR₁₁ or OR₁₁; and R₁₁ is CHₓ, Hₓ, NHₓ, OHₓ, SHₓ or a branched or linear alkyl chain; wherein x is 0, 1, 2 or 3. Preferably, R₄ comprises between 1 to 8 carbon atoms and more preferably 1, 2, 3 or 4 carbon atoms. Preferably, R₆ comprises between 1 to 8 carbon atoms and more preferably 1, 2, 3 or 4 carbon atoms. Preferably, R₈ comprises between 1 to 8 carbon atoms and more preferably 1, 2, 3 or 4 carbon atoms.

Examples of chemical compounds of the structure R₁-R₂-R₃ or R₁-C(O)-R₂-R₃ include acids, amines, monoamides and diamides of butyric acid (H₃C-CH₂-CH₂-COOH); butyric acid ethyl ester (CH₃CH₂CH₂COCH₂CH₃), 4,4,4-trifluorobutyric acid (CF₃CH₂CH₂COOH), 2,2-dimethyl butyric acid (C₂H₅C(CH₃)₂CO₂H), 2,2-diethyl butyric acid, 3,3-dimethyl butyric acid (C₆H₁₂O₂), 3,3-diethyl butyric acid, fumaric acid (HOOCCH=CHCOOH), fumaric acid monomethyl and monoethyl ester, fumaric acid monoamide (C₄H₅O₂N), fumaramide (H₂NCOCHCHCONH₂), succinic acid (HOOC CH₂CH₂COOH) (succinamic acid and succinamide), 2,3-dimethyl succinic acid and methoxy acetic acid (CH₃tH₂OCH₃).

Examples of chemical compounds of the structure phenyl-R₅-R₆-R₇ include acids, amines and amides of phenoxyacetic acid (C₆H₅OCH₂ COOH; C₆H₅OCH₂COONH₃), 2- and 3-thiophenoxy propionic acid (C₆H₅SCH(CH₃) COOH; C₆H₅SCH₂CH₂COOH), 2- and 3-phenoxy propionic acid (C₆H₅OCH(CH₃) COOH; C₆H₅OCH₂CH₂COOH), 2- and 3-phenyl propionic acid (C₆H₅CH(CH₃)COOH; C₆H₅CH₂CH₂COOH), 4-chlorophenoxy-2-propionic acid (ClC₆OCH₂CH₂CO₂H), methoxy acetic acid (H₃COCH₂CO₂H), and 2-thiophenoxy acetic acid (C₆H₅SCH₂COOH).

Examples of chemical compounds of the structure phenyl-R₉-R₁₀ include acids, amines and amides of cinnamic acid (C₆H₅CH=CHCOOH), hydrocinnamic acid, dihydro cinnamic acid (C₆H₅CH₂CH₂COOH), α-methyl hydrocinnamic acid or dihydrocinnamic acid, 2,3-dimethyl hydrocinnamic or dihydrocinnamic acid, phenyl acetate ethyl ester (C₆H₅CH(CH)CH₂COCH₂CH₃), 2-phenoxypropionic acid (C₆H₅OCH₂CO₂H), phenoxy acetic acid (CH₃CH(OC₆H₅)CO₂H), and 3-phenyl butyric acid (C₆H₅CH(CH₃) CH₂COOH). Additional chemical compounds which may or may not be included in the above classification scheme include monobutyrin, tributyrin (CH₂(OCOCH₂CH₂CH₃)CH (OCOCH₂CH₂CH₃)CH₂(OCOCH₂CH₂CH₃), ethyl-phenyl acetic acid (CH₃CH₂C₆H₅CH₂ COOH), indol-3-propionic acid, indol-3-butyric acid, 1- and 2-methyl cyclopropane carboxylic acid (C₅H₈O₂ and C₆H₈O₂), mercaptoacetic acid (C₂H₄O₂S), N-acetylglycine (C₄H₇O₃N), squaric acid (C₄H₂O₄), 4-trifluorobutanol (C₄H₇OF₃), chloropropionic acid (ClCH₂CH₂CO₂H), 3-trimethyl silyl-1-pmposulfonic acid sodium (C₆H₁₅O₃SS), 2-oxopantansane (C₅H₈O₃), isobutly hydroxylamine HCl (C₄H₁₂OCl), 2-methyl butanoic acid (C₅H₁₀O₂), o-benzoyl lactate, n-dimethylbutyric acid glycine amide, o-dimethyl butyric acid lactate, and diethyl butyric acid.

Agents are useful in pharmaceutical compositions for the treatment of blood and virus-associated disorders. Preferred agents in such compositions include, for example, propionic acid, butyric acid, succinic acid, fumaric acid monoethyl ester, dimethyl butyric acid, trifluorobutanol (C₄H₇OF₃), chloropropionic acid (ClCH₂CH₂COOH), isopropionic acid, 2-oxypentasane (CH₃CH₂CH₂C(O)COOH), 2,2- or 3,3-dimethyl butyric acid (C₆H₁₂O₂), 2,2- or 3,3-diethyl butyric acid (C₈H₁₆O₂), butyric acid ethyl ester, 2-methyl butanoic acid (C₅H₁₀O₂), fumaric acid (C₄H₄O₃) and amides and salts thereof. Other examples include methoxy acetic acid (H₃C(O)CH₂COOH), dimethyl butric acid, methoxy propionic acid, N-acetylglycine (H₃CC(O)NCH₂COOH), mercaptoacetic acid (HSCH₂COOH), 1- or 2-methyl cyclopropane carboxylic acid (C₅H₈O₂), squaric acid (C₄H₂O₄), 2- or 3-phenoxy propionic acid, methoxy butyric acid, phenoxy acetic acid, 4-chloro-2-phenoxy 2-propionic acid, 2- or 3-phenoxy butyric acid, phenyl acetic acid, phenyl propionic acid, 3-phenyl butyric acid, ethyl-phenyl acetic acid, 4-chloro-2-phenoxy-2-propionic acid, n-dimethyl butyric acid glycine amide, o-benzoyl lactic acid, o-dimethyl butyric acid lactate, cinnamic acid, dihydrocinnamic acid (C₆H₅CHCH₃COOH), α-methyl-dihydrocinnamic acid, thiophenoxy acetic acid, and amines, amides and salts of these chemicals.

Useful amines and amides include isobutylhydroxylamme:HCl (C₄H₁₂OCl), fumaric acid monoamide (C₄H₅O₂N), fumaramide (H₂NCOCHCHCONH₂), succinamide and isobutyramide (C₄H₉ON). Salts can be sodium, potassium, calcium, ammonium, lithium or choline such as sodium 3-trimethyl silyl-1-proposulfonic acid (C₆H₁₅O₃SiS:Na). Reagents which may be electrostatically or covalently bonded with the inducing agent include amino acids such as arginine (arginine butyrate), glycine, alanine, asparagine, glutamine, histidine or lysine, nucleic acids including nucleosides or nucleotides, or substituents such as carbohydrates, saccharides, lipids, fatty acids, proteins or protein fragment. Combinations of these salts with the inducing agent can also produce useful new compounds from the interaction of the combination.

Chemical compounds are preferably optically pure with a specific conformation (plus {+} or minus {-}), absolute configuration (R or S), or relative configuration (D or L). Particular salts such as sodium, potassium, magnesium, calcium, choline, amino acid, ammonium or lithium, or combinations of salts may also be preferred, however, certain salts may be more advantageous than others. For example, chemical compounds that require high doses may introduce too much of a single salt to the patient Sodium is generally an undesirable salt because at high doses, sodium can increase fluid retention resulting in tissue destruction. In such instances, lower doses or combinations of different or alternative salts can be used. For example, compounds of the invention may be substituted with one or more halogens such as chlorine (Cl), fluorine (F), iodine (I), bromine (Br) or combinations of these halogens. As known to those of ordinary skill in the art, halogenation can increase the polarity, hydrophilicity or lipophilicity or a chemical compound which can be a desirable feature, for example, to transform a chemical compound into a composition which is more easily tolerated by the patient or more readily absorbed by the epithelial lining of the gastrointestinal tract. Such compositions could be orally administered to patients.

Therapeutically effective chemical compounds may be created by modifying any of the above chemical compounds so that after introduction into the patient, these compounds metabolize into active forms, such as the forms above, which have the desired effect on the patient. Compounds may also be created which are metabolized in a timed-release fashion allowing for a minimal number of introductions which are efficacious for longer periods of time. Combinations of chemical compounds can also produce useful new compounds from the interaction of the combination. Such compounds may also produce a synergistic effect when used in combination with other known or other compounds.

Compositions may also comprise proteinaceous agents such as cytokines that will increase the extent or magnitude of hematopoiesis, increase the proliferation of hemoglobin expressing cells, increase or balance the expression of hemoglobin macromolecules or increase or stimulate the specific expression of alternate globin genes such as γ-globin. Such proteinaceous agents include steel factor, insulin, erythropoietin (EPO), interferon (IFN), insulin growth factor (IGF), stem cell factor (SCF), macrophage-colony stimulating factor (M-CSF), granulocyte-colony stimulating factor (G-CSF), GM-CSF, growth factors such as fibroblast-derived growth factor (FGF), epidermal growth factor (EGF) and platelet-derived growth factor (PDGF), nerve growth factor (NGF), vascular endothelial growth factor (VEGF), bone morphogenic proteins (BMPs), the interleukins (IL) IL-1, IL-2, IL-3, IL-4, IL-5, IL-6, IL-7, IL-8, etc., activin also referred to as erythroid differentiation factor (EDF) or follicle-stimulating hormone releasing protein (FRP), inhibin, stem cell proliferation factor (SCPF) and active fragments, subunits, derivatives and combinations of these proteins. Erythropoietin, activin and SCF all stimulate the proliferation of stem cells, committed cells and erythroid progenitor cells, and can also stimulate the expression of embryonic globin, fetal globin or partly functional pseudo-globin expression. The hematopoietic factor, steel factor, also referred to as kit ligand, mast cell growth factor and stem cell factor, recruits and stimulates the proliferation of hemoglobin expressing cells and the specific expression of embryonic or fetal globin. Proteinaceous agents of the invention may also be aminated, glycosylated, acylated, neutralized, phosphorylated or otherwise derivatized to form compositions which are more suitable for the method of administration to the patient or for increased stability during shipping or storage.

Compositions are preferably physiologically stable at therapeutically effective concentrations. Physiological stable compounds are compounds that do not break down or otherwise become ineffective upon introduction to a patient prior to having a desired effect. Compounds are structurally resistant to catabolism, and thus, physiologically stable, or coupled by electrostatic or covalent bonds to specific reagents to increase physiological stability. Such reagents include amino acids such as arginine, glycine, alanine, asparagine, glutamine, histidine or lysine, nucleic acids including nucleosides or nucleotides, or substituents such as carbohydrates, saccharides and polysaccharides, lipids, fatty acids, proteins, or protein fragments. Useful coupling partners include, for example, glycol such as polyethylene glycol, glucose, glycerol, glycerin and other related substances.

Physiological stability can be measured from a number of parameters such as the half-life of the compound or the half-life of active metabolic products derived from the compound. Certain compounds of the invention have *in vivo* half lives of greater than about fifteen minutes, preferably greater than about.one hour, more preferably greater than about two hours, and even more preferably greater than about four hours, eight hours, twelve hours or longer. Although a compound is stable using this criteria, physiological stability can also be measured by observing the duration of biological effects on the patient Clinical symptoms which are important from the patient's perspective include a reduced frequency or duration, or elimination of the need for transfusions or chelation therapy. Preferably, a stable compound of the invention has an *in vivo* half-life of greater than about 15 minutes, a serum half-life of greater than about 15 minutes, or a biological effect which continues for greater than 15 minutes after treatment has been terminated or the serum level of the compound has decreased by more than half.

Preferably, compositions are also not significantly biotransformed, degraded or excreted by catabolic processes associated with metabolism. Although there may be some biotransformation, degradation or excretion, these function are not significant if the composition is able to exert its desired effect.

Compositions are also preferably safe at effective dosages. Safe compositions are compositions that are not substantially toxic (*e.g*. cytotoxic or myelotoxic), or mutagenic at required dosages, do not cause adverse reactions or side effects, and are well tolerated. Although side effects may occur, compositions are substantially safe if the benefits achieved from their use outweigh disadvantages that may be attributable to side effects. Unwanted side effects include nausea, vomiting, hepatic or renal damage or failure, hypersensitivity, allergic reactions, cardiovascular problems, gastrointestinal disturbances, seizures and other central nervous system difficulties, fever, bleeding or hemorrhaging, serum abnormalities and respiratory difficulties.

Compositions useful for treating blood disorders preferably do not substantially affect the viability of a cell such as a normal mammalian cell, the cell being treated or effected by the chemical compound. Normal cell viability, the viability of an untransformed or uninfected cell, can be determined from analyzing the effects of the composition on one or more biological processes of the cell. Detrimental interference with one or more of these cellular processes becomes significant when the process becomes abnormal. Examples of quantitatable and qualifiable biological processes include the processes of cell division, protein synthesis, nucleic acid (DNA or RNA) synthesis, nucleic acid (principally DNA) fragmentation and apoptosis. Others processes include specific enzyme activities, the activities of the cellular transportation systems such as the transportation of amino acids by system A (neutral), system B (acidic) or system C (basic), and the expression of a cell surface protein. Each of these parameters is easily determined as significantly detrimental, for example, in tissue culture experiments, in animal experiments or in clinical studies using techniques known to those of ordinary skill in the art. Abnormal cell division, for example, can be mitosis which occurs too rapidly, as in a malignancy, or unstably, resulting in programmed cell death or apoptosis, detected by increased DNA degradation. The determination of abnormal cell viability can be made on comparison with untreated control cells. Compositions preferably increase normal cell viability. Increased cell viability can be determined by those of ordinary skill in the art using, for example, DNA fragmentation analysis. A decreased amount of fragmentation indicates that cellular viability is boosted. Determination of increased or decreased viability can also be concluded from an analysis of the results of multiple different assays. Where multiple tests provide conflicting results, accurate conclusions can still be drawn by those of ordinary skill based upon the cell type, the correctness or correlation of the tests with actual conditions and the type of composition.

Compositions can be prepared in solution as a dispersion, mixture, liquid, spray, capsule or as a dry solid such as a powder or pill, as appropriate or desired. Solid forms may be processed into tablets or capsules or mixed or dissolved with a liquid such as water, alcohol, saline or other salt solutions, glycerol, saccharides or polysaccharide, oil or a relatively inert solid or liquid. Liquids, pills, capsules or tablets administered orally may also include flavoring agents to increase palatability. Additionally, all compositions may further comprise agents to increase shelf-life, such as preservatives, anti-oxidants and other components necessary and suitable for manufacture and distribution of the composition. Compositions further comprise a pharmaceutically acceptable carrier. Carriers are chemical or multi-chemical compounds that do not significantly alter or effect the active ingredients of the compositions. Examples include water, alcohols such as glycerol and polyethylene glycol, glycerin, oils, salts such as sodium, potassium, magnesium and ammonium, fatty acids, saccharides or polysaccharides. Carriers may be single substances or chemical or physical combinations of these substances.

Another embodiment of the invention is directed to combinations of compositions comprising a chemical compound in combination with an agent known to positively affect hemoglobin expression or hemoglobin expressing cells. The agent may be a chemical compound such as acetic acid, butyric acid, D- or L-amino-n-butyric acid, α- or β-amino-n-butyric acid, arginine butyrate or isobutyramide, all disclosed in U.S. Patent Nos. 4,822,821 and 5,025,029. Others include butyrin, 4-phenyl butyrate (C₆H₅CH₂CH₂CH₂COOH), phenylacetate (C₆H₅CH₂COOH), phenoxy acetic acid, all of which and more are disclosed in U.S. Patent No. 4,704,402, and U.S. Patent Application Ser. No. 08/398,588 (entitled "Compositions for the Treatment of Blood Disorders" filed March 3, 1995), and derivatives, salts and combination of these agents. Alternatively, the agent may be a hematopoietic protein such as erythropoietin steel factor, insulin, an interleukin, a growth factor, hormones such as activin or inhibin, disclosed in U.S. Patent Nos. 5,032,507 and 4,997,815, and active fragments and combinations of these proteins either with each other or with other chemical compounds. Such composition may have additive or synergistic effects.

In another embodiment, compositions of the invention may contain one or more chemical compounds that increase the extent or magnitude of hematopoiesis, increase the proliferation of hemoglobin expressing and other cells, increase or balance the expression of globin proteins or increase or stimulate the specific expression of functional globin protein such as γ-globin. Stimulation of specific gene expression involves activation of transcription or translation promoters or enhancers, or alteration of the methylation pattern or histone distribution along the gene to promote expression. Expression may also be stimulated by inhibition of specific transcription or translation repressors, activation of specific transcription or translation activation factors, or activation of receptors on the surface of particular populations of cells. Stimulation may recruit additional cells to marrow, reprogram differentiated cells to express hemoglobin or switch to the expression of an embryonic, fetal or other globin-like peptide. Stimulation may also activate a previously dormant or relatively inactive genes which substitutes for the defective or damaged gene products such as, for example, the post-natally suppressed genes which encode e, δ or γ globin, which can substitute for adult β globin, or ζ globin which can substitute for a defective or deficient α globin.

Alternatively, compositions may be used to turn down the expression of those genes whose products are being over expressed and thereby disrupting the balanced production of normal globin proteins. Genes whose expression or whose balanced expression can be effected by the compositions include the globin genes such as the various forms of the ζ-type genes, the €-type genes, the α-type genes, the β-type genes, the δ-type genes, the γ-type genes and at least partially functional pseudo-globin genes.

In another embodiment, compositions of the invention comprise an inducing agent to induce expression of a gene product in a virus-infected cell and an anti-viral agent whose anti-viral activity relates to or is directed to the expressed product. Preferably, the gene product expressed is a viral enzyme or a cellular enzyme or activity that is largely expressed in virus-infected cells. Expression products that can be targeted include enzymes involved with DNA replication, which may be either for repair or replication of the genome, assembly of complete virus particles, generation of viral membrane or walls, RNA transcription or protein translation or combinations of these activities. Interference with these process can be performed by inducing and then acting on an enzyme and, preferably, a critical enzyme in the process.

Inducing agents of the invention include retinoic acid, retinol, cytosine arabinoside, phorbols such as the phorbol diester 12-0-tetradecanoylphorbol 13-acetate (TPA), teleocidine B, indole alkaloids, cytotoxin, plant lectins from *Streptomyces*, glucocorticoids such as estrogen and progesterone, phytohemagglutinin (PHA), bryostatin, growth factors (*e.g*. PDGF, VEGF, EGF, FGF, NGF, TGF, BCGF), anti-sense nucleic acids (*e.g*. DNA, RNA or PNA), aptamers (nucleic acid oligonucleotides that form secondary or tertiary structures which bind with high affinity and selectivity to a target molecule), erythropoietin (EPO), the interleukins (IL-1, IL-2, IL-3, etc.), cAMP and cAMP analogs such as dibutyrl cAMP, activin, inhibin, steel factor, interferon, the bone morphogenic proteins (BMBs), hydroxyurea and dimethyl sulfoxide (DMSO). Other inducing agents include interferons (*e.g*. α-, β-, γ-interferon), cytokines such as tumor necrosis factor (TNF), cell receptors and growth factor antagonists, which may be purified or recombinantly produced.

Anti-viral agents useful in compositions of the invention include substrates and substrate analogs, inhibitors and other agents that severely impair, debilitate or otherwise destroy virus-infected cells. Substrate analogs include amino acid and nucleoside analogs. Substrates may be conjugated with toxins or other viricidal substances. Inhibitor include integrase inhibitors, protease inhibitors, polymerase inhibitors and transcriptase inhibitors such as reverse transcriptase inhibitors. Examples of nucleoside analogs include acyclovir (ACV), ganciclovir (GCV), famciclovir, foscamet, ribavirin, zalcitabine (ddC), zidovudine (AZT), stavudine (D4T), larnivudine (3TC), didanosine (ddI), cytarabine, dideoxyadenosine, edoxudine, floxuridine, idozuridine, inosine pranobex, 2'-deoxy-5-(methylamino)uridine, trifluridine and vidarabine. Examples of a few protease inhibitors that show particular promise in human therapy include saquinivir, ritonavir and indinavir. Other anti-viral agents include interferons (*e.g.* α-, β-, γ-interferon), cytokines such as tumor necrosis factor (TNF), cell receptors and growth factor antagonists, which may be purified or recombinantly produced.

The particular combination of inducing agent with antiviral agent that is most effective against a specific disorder can be determined by one of ordinary skill in the art from empirical testing and, preferably, from a knowledge of each agent's mechanism of action. Three such examples are as follows. First, many of the RNA viruses such as HIV and other retroviruses require a reverse transcriptase to transcribe their genome into DNA. A few of the agents that induce expression or activity of retroviruses and their encoded genes, such as, for example, reverse transcriptase, are known to those of ordinary skill in the art. Anti-viral agents such as nucleoside analogs can be administered to the patient. Those substrate analogs will be specifically recognized by the reverse transcriptase that, when incorporated into the infected-cell genome, result in cell death. Second, many viruses require an active protease to assemble virus capsids to be packaged with viral genome. Protease inhibitors or proteases that alter cleavage patterns so that packaging cannot occur can be specifically targeted with an anti-viral agent that comprises an amino acid analog or toxic conjugate. Third, arginine butyrate and isobutyramide enhance expression of viral thymidine kinase in EBV-infected lymphocytes. Ganciclovir or famciclovir, in the presence of the viral thymidine kinase, destroys the infected cell. Treatment of infected cells with both agents, according to the invention, will selectively destroy EBV virus-infected cells. Both inducing and antiviral agents can be purchased commercially and prepared as a mixed composition using techniques well-known to those of ordinary skill in the art.

Compositions of the invention may be administered by oral, parenteral, sublingual, rectal or enteral administration, or pulmonary absorption or topical application. Compositions can be directly or indirectly administered to the patient. Indirect administration is performed, for example, by administering the composition to cells *ex vivo* and subsequently introducing the treated cells to the patient. The cells may be obtained from the patient to be treated or from a genetically related or unrelated patient. Related patients offer some advantage by lowering the immunogenic response to the cells to be introduced. For example, using techniques of antigen matching, immunologically compatible donors can be identified and utilized.

Direct administration of a composition may be by oral, parenteral, sublingual, rectal such as suppository or enteral administration, or by pulmonary absorption or topical application. Parenteral administration may be by intravenous injection, subcutaneous injection, intramuscular injection, intra-arterial injection, intrathecal injection, intra peritoneal injection or direct injection or other administration to the site of the neoplasm. Injectable forms of administration are sometimes preferred for maximal effect. When long term administration by injection is necessary medi-ports, in-dwelling catheters, or automatic pumping mechanisms are also preferred wherein direct and immediate access is provided to the arteries in and around the heart and other major organs and organ systems.

An effective method of administration to a specific site may be by transdermal transfusion such as with a transdermal patch, by direct contact to the cells or tissue, if accessible, such as a skin tumor, or by administration to an internal site through an incisions or some other artificial opening into the body. Compositions may also be administered to the nasal passages as a spray. Diseases localized to the head and brain area are treatable in this fashion as arteries of the nasal area provide a rapid and efficient access to the upper areas of the head. Sprays also provide immediate access to the pulmonary system and are the preferable methods for administering compositions to these areas. Access to the gastrointestinal tract is gained using oral, enema, or injectable forms of administration. Compositions may be administered as a bolus injection or spray, or administered sequentially over time (episodically) such as every two, four, six or eight hours, every day (QD) or every other day (QOD), or over longer periods of time such as weeks to months.

Orally active compositions are preferred as oral administration is usually the safest, most convenient and economical mode of drug delivery. Oral administration is usually disadvantageous because compositions are poorly absorbed through the gastrointestinal lining. Compounds which are poorly absorbed tend to be highly polar. Consequently, compounds which are effective, as described herein, may be made orally bioavailable by reducing or eliminating their polarity. This can often be accomplished by formulating a composition with a complimentary reagent which neutralizes its polarity, or modifying the compound with a neutralizing chemical group. Oral bioavailability is also a problem because drugs are exposed to the extremes of gastric pH and gastric enzymes. These problems can be overcome in a similar manner by modifying the molecular structure to be able to withstand very low pH conditions and resist the enzymes of the gastric mucosa such as by neutralizing an ionic group, by covalently bonding an ionic interaction, or by stabilizing or removing a disulfide bond or other relatively labile bond.

Compounds may also be used in combination with other agents to maximize the effect of the compositions in an additive or synergistic manner. Cytokines which may be effective in combination with the compositions of the invention include growth factors such as B cell growth factor (BCGF), fibroblait-derived growth factor (FDGF), granulocyte/macrophage colony stimulating factor (GM-CSF), granulocyte colony stimulating factor (G-CSF), macrophage colony stimulating factor (M-CSF), epidermal growth factor (EGF), vascular endothelial growth factor (VEGF), platelet derived growth factor (PDGF) nerve growth factor (NGF), stem cell factor (SCF), and transforming growth factor (TGF). These growth factors plus a composition may further stimulate cellular differentiation and/or the expression of certain MHC antigens or tumor antigens. For example, BCGF plus a composition may be effective in treating certain B cell leukemias. NGF plus a composition may be useful in treating certain neuroblastomas and/or nerve cell tumors. In a similar fashion, other agents such as differentiating agents may be useful in combination with a composition of the invention to prevent or treat a neoplastic disorder. Other differentiating agents include B cell differentiating factor (BCDF), erythropoietin (EPO), steel factor, activin, inhibin, the bone morphogenic proteins (BMPs), retinoic acid or retinoic acid derivatives such as retinol, the prostaglandins and TPA.

Alternatively, other cytokines and related antigens in combination with a composition may also be useful to treat or prevent certain blood or cellular disorders, virus infections and other viral disorders. Potentially useful cytokines include tumor necrosis factor (TNF), the interleukins IL-1, IL-2, IL-3, IL-4, IL-5, IL-6, etc., recombinant IL receptors, growth factors, colony stimulating factors, erythropoietin (EPO), the interferon (IFN) proteins IFN-α, IFN-β, and IFN-γ; cyclic AMP including dibutyryl cyclic AMP, hemin, DMSO, hydroxyurea, hypoxanthine, glucocorticoid hormones and cytosine arabinoside. Therapies using combinations of these agents would be safe and effective therapies against malignancies and other forms of cancer. Combinations of therapies may also be effective in inducing regression or elimination of a tumor or some other form of proliferative disorder such as compositions of the invention plus radiation therapy, toxin or drug conjugated antibody therapy using monoclonal or polyclonal antibodies directed against the transformed cells, or specific anti-sense therapy. Effects may be additive, logarithmic or synergistic, and methods involving combinations of therapies may be simultaneous protocols, intermittent protocols or protocols which are empirically determined.

### Blood Disorders

Disorders of globin gene expression are extremely varied and produce a wide range of clinical manifestations. Consequences to the individual range from a mild weakness after exertion to a prolonged and protracted series of crises leading to an early death. Increased expression of the hemoglobin macromolecule or specific globin peptide chains has been shown to alleviate many of these manifestations, improving and prolonging the life of the afflicted individual. Some of the more successful treatments involve the administration of biologically active proteins or chemical compounds to promote hematopoiesis, to promote the proliferation of hemoglobin expressing cells or to increase or stimulate the expression of fetal globin protein. Although promising, these treatments have a number of drawbacks. Many substances are carcinogenic or mutagenic and prolonged use would pose serious risks to the patient Some require continuous use at fairly high doses while others have short effective half-lives. Tolerance to the active ingredient often develops rendering the composition functionally useless. In addition to problems associated with tolerance, the substances themselves or their metabolic by-products or carriers quickly reach toxic levels in the patient's system which slow or inhibit blood cell proliferation. Further, the chemical compounds themselves can be rapidly destroyed by catabolic enzymes, found in the cells and serum such as aminases, oxidases and hydrolases. Many of these enzymes are also found in hepatic cells, the principal sites for cleansing of the blood. Those able to survive cellular and hepatic catabolic processes are quickly eliminated from the patient's system by nephrotic cells of the kidneys. Consequently, *in vivo* retention times for active compounds are extremely short and the ability to achieve any sort of sustained biological effect becomes nearly impossible or, at least, impractical.

It has been discovered that a variety of chemicals including compositions of the invention useful for the treatment of blood and other disorders are more effective when administered to a patient in pulses. Pulse therapy is not a form of discontinuous administration of the same amount of a composition over time, but comprises administration of the same dose of the composition at a reduced frequency or administration of reduced doses. This mode of therapy has not been previously reported.

According to these methods, blood and other disorders can be effectively treated and without unnecessary adverse side effects to the patient. Although most compositions are generally safe and non-toxic at therapeutic doses, pulsed administration further reduces risks associated with, for example, toxicity, allergic reactions, the build-up of toxic metabolites and inconveniences associated with conventional treatment. In addition, chemical compositions, being useful at a reduced dose and frequency, have a substantially reduced risk of induced tolerance. Drugs are not inactivated by cellular enzymes or cleared from cells and organs prior to having the desired effect. Further, long-term therapy, typically required for the amelioration of many blood disorders, becomes possible. Consequently, doses necessary for maintaining a constant effect for the patient are steady and material costs and inconveniences associated with administration are substantially reduced.

Blood disorders that can be treated by the compositions and methods of the invention include any disease or malady which could be characterized as a direct or indirect consequence of a defect or disease of hemoglobin producing cells or the production of hemoglobin. For example, blood disorder may be associated with an anemia such as sickle cell anemia, hemolytic anemia, infectious anemia, aplastic anemias, hypoproliferative or hypoplastic anemias, sideroblastic anemias, myelophthisic anemias, antibody-mediated anemias, anemias due to enzyme-deficiencies or chronic diseases, anemias due to blood loss, radiation therapy or chemotherapy, thalassemias including α-like and β-like thalassemias. Treatable blood disorders also include syndromes such as hemoglobin C, D and E disease, hemoglobin lepore disease, and HbH and HbS diseases. Treatment ameliorates one or more symptoms associated with the disorder. Symptoms typically associated with blood disorders include, for example, anemia, tissue hypoxia, organ dysfunction, abnormal hematocrit values, ineffective erythropoiesis, abnormal reticulocyte (erythrocyte) count, abnormal iron load, the presence of ring sideroblasts, splenomegaly, hepatomegaly, impaired peripheral blood flow, dyspnea, increased hemolysis, jaundice, anemic crises and pain such as angina pectoris.

The mechanism of action of many of the chemical compounds or active ingredients of compositions for the treatment of blood disorders involves effecting one or more of the processes of cell proliferation, cell recruitment, specific hemoglobin expression, heme synthesis or globin chain synthesis. Cell proliferation may be increased, for example, by stimulating stem cells, CFUs, BFUs, megakaryocytes, myeloid cells, platelets, white blood cells or pro-erythrocyte colony growth, or decreased, for example, by effecting a cell's period in or ability to transverse a stage (S, G₀, G₁, M) of the cell cycle. Cell recruitment may be promoted through the expression of specific cytokines such as cell surface receptors or secreted factors. Hemoglobin expression can be increased or decreased by affecting heme expression, globin peptide expression, heme/globin peptide assembly, globin peptide glycosylation or globin transport through the golgi apparatus. Globin expression can be increased or decreased by altering chromatin and/or nucleosome structure to render a genetic element more or less susceptible to transcription, by altering DNA structure, for example, by methylation of G residues, by affecting the activity of cell-specific transcription or translation factors such as activators or repressors, or by increasing the rate of transcription or translation. For example, useful chemical compounds include phenoxyacetic acid, methoxyacetic acid, butyric acid ethyl ester, cinnamic acid, hydrocinnamic acid, α-methyl cinnamic acid and α-methylhydrocinnamic acid (αMHCA) stimulate alterations in binding or removal of transcription factors from the proximal promoter region of certain genes of the γ- and β-globin gene clusters and thereby increase post-natally suppressed gene expression.

Chemical compounds preferably increase the expression of hemoglobin, increase the expression of one or more embryonic or fetal globin genes or increase the number of hemoglobin expressing or fetal globin expressing reticulocytes. Preferably, compositions increase embryonic or fetal globin gene expression or embryonic or fetal reticulocyte counts greater than about 2%, more preferably greater than about 5%, and even more preferably greater than about 9%. For comparative purposes, a 4% increase in fetal globin gene expression equates to about 20% to 25% rise or increase in fetal globin in peripheral blood samples. Consequently, an increase of greater than about 1% fetal globin expression, preferably greater than about 3%, or about 1% fetal globin expressing cells, preferably greater than about 3%, can alleviate symptoms associated with beta globin disorders.

Hemoglobin expression, globin expression and cell proliferation can be assayed by measuring fold increases in expressed amounts of specific protein or numbers of specific cells in treated samples as compared to untreated controls. Utilizing this criteria, compositions preferably increase the amount of hemoglobin expression, the amount of globin expression, the number of hemoglobin expressing cells or the number of globin expressing cells by greater than or equal to about twofold, preferably about four-fold and more preferably about eight-fold.

### Viral and Cellular Disorders

The traditional technique for treating a virus infection, whether active or latent, is to destroy the virus or the virus-infected cells with anti-viral agents. Anti-viral agents are chemical compounds or biological products that are lethal to the virus or the virus-infected cell. This technique is designed to eradicate diseased cells and thereby prevent further infections.

Treatments that destroy the infected cell typically target a basic cellular process such as replication or expression. These treatment regimens are most effective when targeting a specific enzyme such as a polymerase or reverse transcriptase. For example, inhibition of reverse transcription frustrates viral replication and prevents further propagation of the virus. Infected cells are killed by cell lysis or indirectly by the accumulation of viral products. As no infectious virus particles are produced, there are also no subsequent infections.

Unfortunately, anti-viral agents can have a detrimental effect on uninfected cells. Dosages required to destroy the virus-infected cells often effect related and even unrelated activities in otherwise normal cells. Tolerance and cellular accommodation to the single agent or drug is also quite common. Drug transport across the cellular membrane can increase, rendering the administered dose ineffective, and other cellular activities can compensate or substitute for the inhibited function. In addition, many infections are latent with no discernable manifestations. Symptoms can flare periodically over long periods of time. Treatment options are limited to continued amelioration of symptoms without any sort of real cure of the underlying infection. As a consequence, anticipated benefits of these agents are often unattainable or unrealized.

Viruses convert at least some portion of the molecular machinery of the infected cell to their own use. In doing so, the virus causes the cell to express viral or cellular products that allow the body to recognize that cell as infected. Infected cells, once recognized, can be targeted for elimination by the organism's own immune system. Problems arise when the immune system functions poorly or not at all and when the product is transiently expressed or rapidly mutates. These problems are typically associated with latent infections which are much more difficult to treat.

It has been discovered that viral infections can be effectively treated by taking advantage of a molecular process, preferably a necessary enzyme activity, that is uniquely viral (i. e. largely restricted to the virus or the virus-infected cell), and that can be acted upon by an antiviral agent. The first step is not to inhibit that uniquely-viral process, as occurs with conventional therapy, but to actively promote that process. Virus-infected cells are than specifically targeted for destruction, not by the immune system, but by administration of the anti-viral agent whose ability to destroy infected cells relates to the viral activity that has been induced. This treatment combination has the surprising effect of forcing infected cells to become susceptible to selective killing. As a result, infected cells can be specifically targeted for elimination and previously untreatable infections arrested. Further, marginally or non-effective antiviral agents can now be successfully administered because the therapeutically effective dose has been substantially reduced. The concentrations or frequencies of administration of effective compositions can also be reduced to lessen any harmful side-effects without reducing effectiveness against the virus-infected cells.

Another embodiment of the invention is directed to a method for destroying, killing or otherwise severely cripple virus-infected cells by treating said cells with an inducing agent, to induce the activity of a gene product, and an anti-viral agent whose anti-viral activity is directed to the activity of the gene product induced. Preferably, the gene product is a viral enzyme that relates to a basic and necessary process of the virus such as virus adsorption, cell penetration, fusion, uncoating, reverse transcription, integration, DNA replication, viral interference, viral transcription, the switch from early to late expression, the latent or lytic phases, the switch from a latent to lytic phase, defective-interfering particle production, virus assembly, capsid packaging, the generation of virus-specific membrane, virus budding and virus secretion. The activity of one or more of these processes may be enhanced by one or more of the inducing agents and the enhanced activity targeted by one or more antiviral agents. The combination treatment is more effective than conventional treatment with anti-viral agents alone or simply allows for the administration of therapeutically effective amounts of the anti-viral agent which are less than what would be considered effective with conventional treatment regiments.

Agents that induce expression may act directly on the viral genome or indirectly through a cellular factor required for viral expression. For example, viral gene expression can be regulated through the regulation of the expression of thymidine kinase, AP-1, AP-2, Sp1, NF-κB and other transcriptional activators and/or repressors (factors), oncogenes or proto-oncogenes, or protein kinase C. These proteins act to regulate and thereby control expression of specific viral and/or other cellular genetic elements. According to the methods of the invention control over their expression can lead to control over the infection. Other gene products, both viral and cellular in origin, whose expression can be regulated with inducing agents include proteases, polymerases, reverse transcriptases, cell-surface receptors, major histocompatibility antigens, growth factors and combination of these products.

Additional genes whose expression or transcriptional regulation are altered in the presence of butyric acid includes the oncogenes *myc, ras, myb, abl* and *src.* The activities of these gene products as well as the activities of other oncogenes are described in J.D. Slamon et al. (Science 224:256-62, 1984). Anti-proliferative activity also includes the ability to repress tumor angiogenesis through the blockade of angiogenesis factor activity, production or release, transcriptional regulation, or the ability to modulate transcription of genes under angiogenesis or growth factor or hormonal control. Either would be an effective therapy particularly against both prostatic neoplasia and breast carcinomas. Further activities which effect transcription and/or cellular differentiation include increased intracellular cAMP levels, inhibition of histone acetylation, and inhibition of genomic methylation. Each of these activities are directly related to gene expression and increased expression can sensitize infected cells to a specific anti-viral agent.

Two of the preferred inducing agents are arginine butyrate and isobutyramide. Arginine butyrate induces EBV-TK activity in EBV-immortalized B-cells and patient-derived tumor cells. As latently-infected B-cells do not express TK, exposure of these cells to agents like arginine butyrate results in a modest induction of lytic replication and TK expression. Surprisingly, TK expression can be used as a point for attack by anti-viral agents allowing for treatment of latent infections.

Although there are numerous case reports of administration of inducing agents such as butyrates to patients for the treatment of malignancies (A. Novogrodsky et al., Cancer 51:9-14, 1983; A.A. Miller et al., Eur. J. Cancer Clin. Oncol. 23:1283-89, 1987), and for the administration of anti-viral agents for the treatment of viral disorders, there are no treatments directed to the administration of both agents. Arginine butyrate has been administered to adults and children over extended periods of time without major side effects (S.P. Perrine et al., Br. J. Haematol., 1994; S.P. Perrine et al., N. Engl. J. Med. 328:81-86, 1993). These drugs were recently approved for human studies to induce fetal Hb in children with sickle cell anemia and β-thalassemia. Preliminary *in vitro* studies demonstrate that induction of EBV-TK activity in EBV-immortalized B-cells and patient-derived tumor cells using these drugs is possible, and that these previously-resistant cells are rendered susceptible to ganciclovir therapy. Treatment of patients with viral-associated tumors such as EBV with inducing agents such as arginine butyrate, to induce the expression of EBV-TK, and GCV, to eliminate EBV-TK expressing tumor cells, is an effective, nontoxic therapy. This therapeutic regiment does not depend on the associated viral genome being the cause of the tumor. Just the presence of the EBV genome in latent form would be predicted to make the tumor susceptible to this combination protocol.

Preferably, the inducing agent is butyric acid in the form of arginine butyrate or isobutyramide and the antiviral agent is a nucleoside analog. Butyric acid is one many naturally-occurring short-chain fatty acids that are generated in the small and large bowel by metabolism of carbohydrates. Butyrate is a four-carbon fatty acid with weakly acidic properties, and is rapidly absorbed and metabolized. Butyrates have shown significant anti-tumor effects. Sodium butyrate (NAB) has been used clinically in patients with acute myelogenous leukemias and there has now been extensive experience with arginine butyrate, a salt of butyrate, in clinical studies for the treatment of β-hemoglobinopathies, and more recently with refractory solid neoplasms (F.M. Foss et al., Proc. ASCO 13:162 1994; D.A. Sanders et al., Proc. ASCO, 1995). Butyrate, and derivatives of butyrate including arginine butyrate, have demonstrated several effects upon transformed cell lines *in vitro* which include decreased DNA replication leading to arrest of cell division in the G₁ phase, modification of cellular morphology and alteration of gene expression consistent with differentiation of a given cell type examined (D. Klehr et al., Biochemistry 31:3222-29, 1992). For example, human tumor cell lines as diverse as colon, breast, melanoma, hepatoma, squamous cell carcinoma of the cervix, endometrial, adenocarcinoma, teratocarcinoma cell lines, leukemic cells (HL -60) and normal human keratinocytes can all be induced to differentiate in the presence of butyrate concentrations ranging from 2-5 mM (S.P. Perrine et al., Biochem. Biophys. Res. Commun. 148:694-700, 1987).

Multiple mechanisms of action for butyrate have been postulated. Butyrates have been shown to induce differentiation of tumor cell lines. The mechanism(s) of action proposed for these effects upon differentiation are varied, and are not fully understood. Butyrate inhibits histone (nuclear) deacetylase, which results in hyperacetylation of histones H3 and H4. When histories are acetylated, they have a reduced affinity for chromatin, thus allowing for chromosomal unfolding, and possibly enhancement of expression of certain genes. It is postulated that butyrate acts to prevent histones from binding to key regulatory regions on chromatin designated as nuclear scaffolding attached regions, and nuclear matrix-attached regions, with a net result that promoter regions of certain genes are exposed for expression.

Butyrate-associated induction of genes have been characterized for various cell types, and the genes are consistently in the class of differentiation markers of a cell. For example, in colon cancer cell lines, morphologic changes observed in the presence of butyrate correlate with increased expression of alkaline phosphatase, plasminogen activator and CEA, all markers of differentiation. Hepatoma cell lines increase expression of alpha fetoprotein. Breast cancer cell fines express milk-related glycoproteins, epithelial membrane antigens and increased lipid deposition. Sodium butyrate can also induce expression of cellular proteins associated with converting basal keratinacytes into committed epithelial cells.

Alteration of expression of certain transcription factors that regulator gene expression and regulation of the cell cycle. In the breast cancer cell line MCF-7, butyrate induces a block in cellular proliferation which is associated with decreased expression of estrogen and prolactin hormone receptor mRNA expression, thus blocking the potential growth stimulation by estrogen and prolactin. These effects are associated with increased expression of the EGF receptor. Butyrate also has been shown to induce down regulation of c*-myc* and p53 mRNA, and up-regulate expression of the c*-fos* transcription factor. In mouse fibroblasts, butyrate will block the cell cycle in the G1 phase. When these cells are stimulated to proliferate with serum, TPA or insulin, the immediate-early response transcription factors c*-myc* and c-*jun* are unregulated. However, the late G1 phase downstream gene marker cdc-2 mRNA is not expressed and cells are prevented from entering S phase.

Butyrate is an effective cell cycle blocker, associated with a putative restriction point, related to termination of expression of a labile protein. It is generally thought to block cell cycle progression in G1, but might also inhibit some cell types at a point in G2 (J.H. Bruce et al., Neurochem. Res. 17:315-20, 1992). Decreased p53 levels in correlation with butyrate treatment and the inhibition of polyomavirus DNA replication in p53 and Rb-knockout primary mouse fibroblasts in response to butyrate appear to rule out a direct involvement of these gene products individually in the mechanism of butyrate and imply that a putative control point perhaps lies at a later step in the cell cycle. It therefore appears that the butyrate effect is likely to involve a mechanism fundamentally conserved among cell types, but it does not appear to be exerted directly via the Rb or p53 gene product.

The herpes virus family members are capable of bypassing the butyrate-mediated block, which is probably due to the role of viral early genes in DNA synthesis, such as the viral DNA polymerase, DNA-binding protein and helicase genes (F.F. Shadan et aL, J. Virol. 68:4785-96, 1994). Butyrate treatment has been reported to result in the induction of the major CMV major immediate-early protein (IEP) by activating the IEI promoter via cellular factors in a human epithelial thyroid papilloma carcinoma cell line, and in cultured endothelial cells 149 under conditions that are conducive to terminal differentiation (L.P. Villarreal, Microbiol. Rev. 55:512-42, 1991). Similarly, EBV early antigen is induced by butyrate in the P3HR-1 cell line as well as Raji and NC37 cell lines. These results indicate that butyrate exerts some of its effects on viral growth at the level of gene transcription. This conclusion is also supported by the observation that butyrate activates the long terminal repeat-directed expression of human immunodeficiency virus and induces the Moloney murine sarcoma virus via a putative butyrate response enhancer-promoter element (C. Bohan et al., Biochem. Biophys. Res. Commun. 148:899-907, 1987; D.C. Tang et al., Biochem. Biophys. Res. Commun. 189:141-47, 1992; A. Yeivin et al., Gene 116:159-64, 1992). Therefore, butyrate appears associated with a general induction of early viral proteins. Butyrate has been reported to exert additional cytostatic effects such as G2/M blockage and anti-viral activity against RNA viruses.

One of the more difficult viral disorders to treat is a herpes virus infection. These viruses manifest distinct pathologies for both the active or lytic phase, and the latent phase. Many herpes-family virus-infected cells, including many cells infected by HSV and CMV, can be killed by nucleoside analog antiviral drugs like ganciclovir and acyclovir. Epstein-Barr virus (EBV), a typical herpes virus, is at best slightly susceptible to anti-viral drugs that inhibit replication of the herpes viruses. These drugs are not effective in limiting the progress of the disease, and, moreover, do not cure the underlying infection. Infectious particles remain in local regions of the body and susceptibility to anti-viral drugs is too difficult to assess as there is no adequate *in vitro* system for studying lytic replication. There are also no plaque assays for EBV and efficient *in vitro* infection of epithelial cells followed by lytic replication does not occur.

Unlike other members of the herpesvirus family, EBV is resistant to the antiviral agent ganciclovir, because of low levels of viral thymidine kinase. Acyclovir and ganciclovir have also been used to treat AIDS patients, many of whom had an active EBV infection. During treatment, regression of hairy leukoplakia, an EBV disorder, was inadvertently observed while latent EBV infection was unaffected. Additional studies demonstrated that even when virus production is minimal, expression of many EBV genes, active during the lytic cycle such as thymidine kinase, can be induced. Therefore, exposure of EBV-transformed B-cells or tumor cells to arginine butyrate induces EBV-TK and renders them sensitive to ganciclovir.

Like herpes simplex virus (HSV) and varicella-zoster virus (VZV), EBV encodes a thymidine kinase enzyme localized to the *BamH* I, X fragment of the genome. In a rate-limiting step, the TK converts nucleoside analogs to their monophosphate form. Cellular enzymes complete their conversion to biologically-active triphosphates. A viral DNA polymerase preferentially incorporates the toxic metabolites into viral DNA, leading to premature termination of the nascent DNA. ACV is a purine nucleoside analog with a linear side chain replacing the cyclic sugar of guanosine. GCV differs from ACV in the addition of a hydroxymethyl group to the side chain. However, ACV and GCV differ in functional assays. Whereas HSV TK preferentially phosphorylates ACV, EBV-TK preferentially phosphorylates GCV. Furthermore, because GCV triphosphate accumulates to higher levels and persists for longer periods in infected cells than ACV, GCV produces more interference with cellular DNA synthesis than occurs with ACV. In one study, selective toxicity of GCV for cells expressing HSV-TK was utilized to promote tumor killing in the CNS. Rapidly dividing murine glioma cells were infected *in vivo* with an amphotropic retrovirus containing HSV-TK. Animals were treated with GCV, which killed TK+ tumor cells, sparing adjacent normal cells that replicated too slowly for efficient infection and viral TK expression.

Types of virus infections and related disorders that can be treated include, for example, infections due to the herpes family of viruses such as EBV, CMV, HSV I, HSV II, VZV and Kaposi's-associated human herpes virus (type 8), human T cell or B cell leukemia and lymphoma viruses, adenovirus infections, hepatitis virus infections, pox virus infections, papilloma virus infections, polyoma virus infections, infections due to retroviruses such as the HTLV and HIV viruses, and infections that lead to cell proliferative disorders such as, for example, Burkitt's lymphoma, EBV-induced malignancies, T and B cell lymhoproliferative disorders and leukemias, and other viral-induced malignancies. Other neoplasias that can be treated include virus-induced tumors, malignancies, cancers or diseases which result in a relatively autonomous growth of cells. Neoplastic disorders include leukemias, lymphomas, sarcomas, carcinomas such as a squamous cell carcinoma, a neural cell tumor, seminomas, melanomas, germ cell tumors, undifferentiated tumors, neuroblastomas (which are also considered a carcinoma by some), mixed cell tumors or other malignancies. Neoplastic disorders prophylactically or therapeutically treatable with compositions of the invention include small cell lung cancers and other lung cancers, rhabdomyosarcomas, chorio carcinomas, glioblastoma multiformas (brain tumors), bowel and gastric carcinomas, leukemias, ovarian cancers, prostate cancers, osteosarcomas or cancers which have metastasized. Diseases of the immune system which are treatable include the non-Hodgkin's lymphomas including the follicular lymphomas, adult T and B cell lymphoproliferative disorders such as leukemias and lymphomas, hairy-cell leukemia, hairy leukoplakia, acute myelogenous, lymphoblastic or other leukemias, chronic myelogenous leukemia and myelodysplastic syndromes. Additional diseases which can be treated include breast cell carcinomas, melanomas and hematologic melanomas, ovarian cancers, pancreatic cancers, liver cancers, stomach cancers, colon cancers, bone cancers, squamous cell carcinomas, neurofibromas, testicular cell carcinomas, and adenocarcinomas.

Viruses may exist in infected cells as autonomous particles, or be integrated as, for example, latent infections. Latent infections may be periodic, such as HSV I and II, or be continuously productive of virus or virus products, but at fairly low levels. Infections may also be lytic with infectious particles secreted or otherwise extruded or expelled (virus burst) from cells. Infections may also be of parts of a virus such as, for example, by viral genes or by defective-interfering particles which are incapable of productive replication on their own, but may be capable of causing disease.

Cells that can be treated include any cell that becomes infected with a virus or a part of a virus and, preferably, infected by integration. Such cells include cells of the hematopoietic system such as lymphocytes, erythrocytes and mylocytes, neural cells and neural-supporting cells, cells of the digestive system, cells of the epithelial system. Cells that contain integrated viral genomes or only parts of viral genomes may also be effectively treated.

Administration of the inducing agent and the antiviral agent may be to cells or directly to a patient for prophylaxis or therapeutic treatment of a confirmed or suspected viral disorder. The patient may be a domesticated animal or mammal such as a dog, cat, horse, cow, steer, pig, sheep, goat or chicken, or a wild animal, but is preferably a human. Administration may be to an adult, an adolescent, a child, a neonate, an infant or *in utero.*

Administration of the inducing agent and the anti-viral agent may be staggered over time or simultaneous in a single composition. Further, the anti-viral agent may be administered before, after or simultaneously with the inducing agent. Administration of either agent may be short term, continuous or sporadic as necessary. Patients with a suspected or diagnosed viral-associated disorders may only require treatment for short periods of time or until the disorder has been effectively overcome.

Compositions and methods for the treatment of viral disorders, and particularly viral proliferative disorders, by augmenting the treatment methods of the invention with conventional chemo-therapy, radiation therapy, antibody therapy, and other forms of therapy. Some conventional chemotherapeutic agents which would be useful in combination therapy with methods and compositions of the invention include the cyclophosphamide such as alkylating agents, the purine and pyrimidine analogs such as mercaptopurine, the vinca and vinca-like alkaloids, the etoposides or etoposide like drugs, the antibiotics such as deoxyrubocin and bleomycin, the corticosteroids, the mutagens such as the nitrosoureas, antimetabolites including methotrexate, the platinum based cytotoxic drugs, the hormonal antagonists such as antiinsulin and antiandrogen, the antiestrogens such as tamoxifen an other agents such as doxorubicin, L-asparaginase, DTIC, mAMSA, procarbazine, hexamethylmelamine and mitoxantrone. These agents could be given simultaneously or alternately as defined by a protocol designed to maximize effectiveness, but minimize toxicity to the patient's body.

Virus-infected cells may also be treated *in vivo* by administering the inducing agent and the anti-viral agent directly to the patient. For example, patients exposed to mutagens, carcinogens, radiation, or other cancer producing agents may be continuously treated with compositions to inhibit the expected development of a neoplastic condition. Patients who have been genetically screened and determined to be at high risk for the future development of a neoplasia may also be administered compositions, possibly beginning at birth and possibly for life. Both prophylactic and therapeutic uses are readily acceptable because these compounds are generally safe and non-toxic at effective dosages.

Another embodiment of the invention is directed to a method for treating a viral disorder in a patient comprised of administering an activator and an anti-viral agent to the patient wherein the activator is administered in an amount sufficient to activate expression of a latent virus integrated into proliferating cells of the patient. Useful activators include phorbol ester, an oxidized phorbol ester, ceramide, bryostatin, an inducing agent or a combination thereof. Activator should be administered in an amount sufficient to activate, for example, protein kinase C, an oncogene, thymidine kinase, AP-1, AP-2, Sp-1 or NF-κB.

### Administration Therapy

Another embodiment of the invention is directed to the pulsed administration of pharmaceutical compositions for the treatment or prevention of a disorder. Pulsed administration is surprisingly more effective than continuous treatment as pulsed doses are often lower than would be expected from continuous administration of the same composition. Each pulse dose can be reduced and the total amount of drug administered over the course of treatment to the patient is minimize.

In traditional forms of therapy, repeated administration is designed to maintain a desired level of an active ingredient in the body. Very often, complications that develop can be attributed to dosage levels that, to be effective, are near toxic or otherwise harmful to normal cells. In contrast, with pulse therapy, *in vivo* levels of drug drop below that level required for effective continuous treatment. Therefore, pulsing is not simply the administration of a sufficiently large bolus such that there will be therapeutically sufficient drug available for a long period of time. Pulsed administration can substantially reduce the amount of the composition administered to the patient per dose or per total treatment regimen with an increased effectiveness. This represents a significant saving in time, effort and expense and, more importantly, a lower effective dose substantially lessens the number and severity of complications that may be experienced by the patients. As such, pulsing is surprisingly more effective than continuous administration of the same composition.

Preferably, compositions contain chemicals that are substantially non-toxic. Substantially non-toxic means that the composition, although possibly possessing some degree of toxicity, is not harmful to the long-term health of the patient. Although the active component of the composition may not be toxic at required levels, there may also be problems associated with administering the necessary volume or amount of the final form of the composition to the patient. For example, if the composition contains a salt, although the active ingredient may be at a concentration that is safe and effective, there can be a harmful build-up of sodium, potassium or another ion. With a reduced requirement for the composition or at least the active component of that composition, the likelihood of such problems can be reduced or even eliminated. Consequently, although patients may have minor or short term detrimental side-effects, the advantages of taking the composition outweigh the negative consequences.

Methods for the pulsed administration of compositions of the invention are preferably used for the treatment of blood disorders such as hemoglobinopathies (*e*.*g*. sickle cell anemia, thalassemia), neoplastic diseases including tumors, leukemias, lymphoproliferative disorders and metastases, and cell proliferative disorders such as viral-induced malignancies (*e.g.* latent virus infections) and cytopenia including red and white blood cell anemia, leukopenia, neutropenia and thrombocytopenia. Compositions most effective at pulsed administration are typically nontoxic or non-cytotoxic chemicals without any substantial proteinaceous active component at the therapeutically effective pulsed dose. Preferably, treatment does not stimulate apoptosis in the cells being directly treated or in the otherwise normal cells of the body which will also be exposed to the composition.

Individual pulses can be delivered to the patient continuously over a period of several hours, such as about 2, 4, 6, 8, 10, 12, 14 or 16 hours, or several days, such as 2, 3, 4, 5, 6, or 7 days, preferably from about 1 hour to about 24 hours and more preferably from about 3 hours to about 9 hours. Alternatively, periodic doses can be administered in a single bolus or a small number of injections of the composition over a short period of time, typically less than 1 or 2 hours. For example, arginine butyrate has been administered over a period of 4 days with infusions for about 8 hours per day or overnight, followed by a period of 7 days of no treatment. This has been shown to be an effective regimen for many thalassemic disorders. Fetal hemoglobin levels rise substantially and there is a significant rise in the number of both adult and fetal hemoglobin expressing cells. Substantially means that there are positive consequences that raise the patient's standard of living such as, for example, increased activity or mobility, fewer side-effects, fewer hospital stays or visits to the physician, or fewer transfusions.

The interval between pulses or the interval of no delivery is greater than 24 hours and preferably greater than 48 hours, and can be for even longer such as for 3, 4, 5, 6, 7, 8, 9 or 10 days, two, three or four weeks or even longer. As the results achieved may be surprising, the interval between pulses, when necessary, can be determined by one of ordinary skill in the art. Often, the interval between pulses can be calculated by administering another dose of the composition when the composition or the active component of the composition is no longer detectable in the patient prior to delivery of the next pulse. Intervals can also be calculated from the *in vivo* half-life of the composition. Intervals may be calculated as greater than the *in vivo* half-life, or 2, 3, 4, 5 and even 10 times greater the composition half-life. For compositions with fairly rapid half lives such as arginine butyrate with a half-life of 15 minutes, intervals may be 25, 50, 100, 150, 200, 250 300 and even 500 times the half life of the chemical composition.

The number of pulses in a single therapeutic regimen may be as little as two, but is typically from about 5 to 10, 10 to 20, 15 to 30 or more. In fact, patients can receive drugs for life according to the methods of this invention without the problems and inconveniences associated with current therapies. Compositions can be administered by most any means, but are preferable delivered to the patient as an injection (*e*.*g*. intravenous, subcutaneous, intraarterial), infusion or instillation, and more preferably by oral ingestion. Various methods and apparatus for pulsing compositions by infusion or other forms of delivery to the patient are disclosed in U.S. patent numbers 4,747,825; 4,723,958; 4,948,592; 4,965,251 and 5,403,590.

Compositions administered in pulses have the surprising benefit of reducing the overall load of drug on the patient as the total amount of drug administered can be substantially less than that amount that has been therapeutically administered by conventional continuous therapy. For example, arginine butyrate has been shown to be effective at continuous administration at about 2000 mg/kg patient weight. Doses of between about 400 to 1500 mg/kg, preferably from about 600 to 1000 mg/kg and more preferably from 700 to 800 mg/kg, when administered in pulses, are surprisingly more beneficial as measured by a rise in fetal hemoglobin levels in thalassemic patients. Typical pulsed amounts of arginine butyrate are from about 2 to about 20 g/kg/month, and preferably from about 3 to about 10 g/kg/month wherein the patient receives a total of less than about 20 kg per month, preferably less than about 15 kg per month and more preferably less than about 10 kg per month. The amounts administered per pulse as well as the total amount of the composition received by the patient over the regimen is substantially reduced. Preferably, the therapeutically effective pulsed dose is less than the continuous dose, or less than one half, one third, one quarter, one fifth, one tenth or even one twentieth of the therapeutic continuous dose of the same composition or even less.

A treatment regimen can be considered effective if it stimulates globin chain expression or the proliferation of erythroblasts or other erythroid progenitor cells, for example with hemoglobinopathy patients, the proliferation of cells such as white blood cells or platelet forming cells, or reduces the number of proliferating cells in, for example, a tumor or other malignancy. Cell numbers are usually most easily determined from peripheral blood sampling or from calculations of tumor size.

Another embodiment of the invention is directed to methods for the pulsed administration of compositions to a patient along with the pulsed or non-pulsed administration of other compositions or therapies for the treatment or amelioration of a disorder. Pulsing of either or both of the compositions can, in part, synchronize cell development, as there is an increased proliferation of erythrocytes and an increased expression of hemoglobin, specifically, fetal hemoglobin. Compositions and therapies which can be pulsed include most of the known or conventional or already well-known treatment regimens. One preferable treatment involves the pulsed or continuous administration of erythropoietin, or another bone marrow cell stimulant, followed by the pulsed administration of a chemical composition of the invention. This regimen has the beneficial effect of stimulating the process of E/Mega cell to erythrocyte development and proliferation which can be followed by stimulation of fetal globin gene expression from the newly proliferated cells. Following such treatments, fetal globin levels in the body rise substantially and much higher than would have been expected from conventional continuous therapy.

Another embodiment of the invention is directed to methods for the treatment of patients with blood disorder comprising the pulsed administration of one or more compositions. Compositions to be administered contain a therapeutically effective pulsed amount of a chemical compound or proteinaceous agent. A therapeutical effective pulsed amount in that amount which has a beneficial effect to the patient by alleviating one or more symptoms of the disorder or simply reduce premature mortality. For example, a beneficial effect may be a decrease in pain, a decrease in duration, frequency or intensity of crises, an increased hematocrit, an improved erythropoiesis, a reduced or eliminated necessity for chelation therapy, an increased reticulocyte count, an increased peripheral blood flow, a decreased hemolysis, decreased fatigue or an increased strength. Preferably, a therapeutic amount is that amount of chemical compound or agent that stimulates or enhances the expression of non-adult globin such as embryonic or fetal globin, or the proliferation of embryonic, fetal or adult globin expressing cells. A therapeutically effective amount for continuous therapy is typically greater than a therapeutically amount that is effective in pulsed therapy. Consequently, pulsed therapy exposes the patient to lower levels of the composition and/or the active ingredient than would be needed with non-pulse therapy.

Compositions provided to the patient may include any combination of the proteins or chemical compounds described herein or known to those of ordinary skill in the art. The patient may be a domesticated animal such as a dog, cat, horse, cow, steer, pig, sheep, goat or chicken, or a wild animal, but is preferably a human or another primate. Administration may be to an adult, an adolescent, a child, a toddler, a neonate or an infant, or administered *in utero.* Administration of the composition may be short term continuous or sporadic as necessary. Patients with a suspected or diagnosed with a blood disorder may only require composition treatment for short periods of time or until symptoms have abated or have been effectively eliminated.

Treatments to the patient may be therapeutic or prophylactic. Therapeutic treatment involves administration of one or more compositions of the invention to a patient suffering from one or more symptoms of the disorder. Symptoms typically associated with, for example, blood disorders include, for example, anemia, tissue hypoxia, organ dysfunction, abnormal hematocrit values, ineffective erythropoiesis, abnormal reticulocyte count, abnormal iron load, splenomegaly, hepatomegaly, impaired peripheral blood flow, dyspnea, increased hemolysis, jaundice, anemic crises and pain such as angina pectoris. Relief and even partial relief from one or more of these symptoms corresponds to an increased life span or simply an increased quality of life. Further, treatments that alleviate a pathological symptom can allow for other treatments to be administered.

Prophylactic treatments involve pulsed administration of a composition to a patient having a confirmed or suspected blood disorder without having any overt symptoms. For example, otherwise healthy patients who have been genetically screened and determined to be at high risk for the future development of a blood disorder may be administered compositions of the invention prophylactically. Administration can begin at birth and continue, if necessary, for life. Both prophylactic and therapeutic uses are readily acceptable because these compounds are generally safe and non-toxic.

Another embodiment of the invention is directed to a method for regulating the expression of a globin gene in a mammalian cell. Briefly, the cell is exposed to an effective amount of a composition. A poorly expressed or quiescent globin gene of the cell is stimulated to increase the expression of its protein product. An effective amount of the composition is that amount which increases the extent or magnitude of hematopoiesis, increases the proliferation of hemoglobin expressing cells, increases, decreases or balances expression from one or more globin genes, or increases or stimulates the specific expression of one or more globin genes such as an alpha (α) globin gene, a zeta (ζ) globin gene, an epsilon (e) globin gene, a beta (β) globin gene, a delta (δ) globin gene, a gamma (G-γ or A-γ) globin gene, or an, at least, partly functional pseudo-globin gene. Cells can be treated in culture or *in vivo.* Cultures of treated cells will produce increased amounts of hemoglobin and preferably embryonic or fetal globin. This hemoglobin can be harvested for introduction to a patient or the stimulated cells themselves can be administered to the patient. Alternatively, recombinant cells containing a globin gene which can be stimulated by compositions of the invention can be utilized. These recombinant cells may be heterologous or homologous natural cells, or synthetically created cells such as a lipid vesicles.

Another embodiment of the invention is directed to a method for regulating the proliferation of red blood cells and, preferably, specifically regulating the expression of fetal hemoglobin. As above, an effective amount of a composition is administered in pulses to, for example, a cell population obtained from stem cells, bone marrow, cord blood, yolk sac cells, or fetal cells such as fetal liver cells, or combinations thereof, *ex vivo.* The pulse-treated cells, or purified products harvested from these cells, are then administered to a patient *in vivo.* This method can be utilized to treat blood disorders in patients by increasing the amount of one or more different types of globin or hemoglobin expressing cells can alleviate symptoms associated with a blood disorder. Cells can be obtained from volunteers or the patients to be treated. Alternatively, treated cells or products derived from treated cells can be harvested, purified by, for example, column chromatography, and utilized for other medical applications such as diagnostic or other treatment monitoring screening kits.

Another embodiment of the invention is directed to a method for ameliorating a blood disorder by administering a therapeutically effective amount of a pharmaceutical composition containing an agent that stimulates the expression of a globin gene or stimulates the proliferation of hemoglobin expressing cells wherein the composition does not significantly decrease viability of the cell being treated or a normal cell. The therapeutically effective amount is that amount which ameliorates one or more symptoms of the blood disorder or reduces premature mortality. A normal cell is a relatively healthy mammalian cell that is not otherwise infected or transformed. Viability can be assayed by determining the effect of the composition on cell division, protein or nucleic acid synthesis, biochemical salvage pathways, amino acid or nucleotide transport processes, nucleic acid fragmentation or apoptosis and comparing the effects observed to control cells. Pulsing, according to the described treatment regimens, can also be used to administer these and other compositions of the invention and their effects tested in tissue culture, *in vivo* or by cell counting.

Patients with blood disorders are typically quite infirm with, for example, iron damaged organs and systems. Most treatments further tax the patient's already frail health in an effort to combat the disorder. This is true for both arginine butyrate and isobutyramide which decrease cell viability as determined in DNA fragmentation assays. To decrease cell viability is not desired for the treatment of blood disorders and may even be harmful. Surprisingly, many of the pulsed compositions maintain or, preferably, increase cell viability. This is a great benefit in the treatment of blood disorders and can significantly increase the chances for a successful outcome for the patient. For example, the pulsed administration of phenoxyacetic acid or butyric acid ethyl ester both reduce DNA fragmentation in fragmentation assays, and phenoxyacetic acid and α-methyl hydrocinnamic acid do not significantly alter system A transport of amino acids.

As such, pulsed composition can be used to treat or prevent iron overloaded or iron deficient systems such as occurs in transfused patients and anemic patients with thalassemia or sickle cell anemia. As chemicals of the compositions of the invention regulate systems that exploit iron, the amount of free and the amount of available iron in a patients's system can be regulated and carefully controlled. Chelation therapy, often the only conventional treatment available for iron overloaded transfusion patients, may be lessened or avoided entirely. As chelation therapy is often uncertain and with some risk of its own, the long-term prognosis for these patients is greatly improved.

Another embodiment of the invention is directed to a method for increasing fetal hemoglobin comprising the pulsed administration of a composition to a patient. For example, hemoglobin F content of blood so treated is increased greater than about 2%, preferably greater than about 5% and more preferably greater than about 10%. Patients which can be treated include any mammal such as a human. Chemical compounds which could be utilized include phenoxy acetic acid, butyric acid ethyl ester, cinnamic acid, hydrocinnamic acid, α-methyl hydrocinnamic acid, methoxy acetic acid, 2,2-dimethylbutyric acid, 2,2-diethylbutyric acid, phenyl butyric acid, thiophenoxy acetic acid, phenoxy propionic acid, succinamide, or a derivative or modification thereof. Such methods are useful to treat or prevent blood disorders in the same or a different patient. For example, to treat the same patient, the compound can be pulse administered for a therapeutically effective period of time to allow the hemoglobin content of just the globin protein content to rise. Alternatively, the patient can be treated and the patient's blood collected at peak times of hemoglobin or globin production, collected and stored, and administered to another patient or re-administered to the same patient. Such treatments would be useful therapies for those being treated with radiation therapy, chemotherapy, bone marrow transplants, blood diseases, such as sickle cell disease and thalassemia, and other disorders which would be alleviated with an increased blood hemoglobin content.

### Treatments for Infections and Neoplasia

Another embodiment of the invention is directed to methods for the treatment of a patient with an infection or a neoplastic disorder comprising the pulsed administration of a therapeutically effective composition. Treatable infectious diseases include bacterial infections such as sepsis and pneumonia, infections caused by bacterial pathogens such as, for example, *Pneumococci, Streptococci, Staphylococci, Neisseria, Chlamydia, Mycobacteria, Actinomycetes* and the enteric microorganisms such as enteric *Bacilli*; viral infections caused by, for example, a hepatitis virus, a retrovirus such as HIV, an influenza virus, a papilloma virus, a herpes virus (HSV I, HSV II, EBV), a polyoma virus, a slow virus, paramyxovirus and corona virus; parasitic diseases such as, for example, malaria, trypanosomiasis, leishmania, amebiasis, toxoplasmosis, sarcocystis, pneumocystis, schistosomiasis and elephantitis; and fungal infections such as candidiasis, phaeohyphomycosis, aspergillosis, mucormycosis, cryptococcosis, blastomycosis, paracoccidiodomycosis, coccidioidomycosis, histomycosis, actinomycosis, nocardiosis and the *Dematiaceous* fungal infections.

Anti-neoplastic activity includes, for example, the ability to induce the differentiation of transformed cells including cells which comprise leukemias, lymphomas, sarcomas, neural cell tumors, carcinomas including the squamous cell carcinomas; seminomas, melanomas, neuroblastomas, mixed cell tumors, germ cell tumors, undifferentiated tumors, neoplasm due to infection (*e.g*. viral infections such as a human papilloma virus, herpes viruses including Herpes Simplex virus type I or II or Epstein-Barr virus, a hepatitis virus, a human T cell leukemia virus (HTLV) or another retrovirus) and other malignancies. Upon differentiation, these cells lose their aggressive nature, no longer metastasize, are no longer proliferating and eventually die and/or are removed by the T cells, natural killer cells and macrophages of the patient's immune system. The process of cellular differentiation is stimulated or turned on by, for example, the stimulation and/or inhibition of gene specific transcription. Certain gene products are directly involved in cellular differentiation and can transform an actively dividing cell into a cell which has lost or has a decreased ability to proliferate. An associated change of the pattern of cellular gene expression can be observed. To control this process includes the ability to reverse a malignancy. Genes whose transcriptional regulation are altered in the presence of compositions of the invention include the oncogenes *myc*, *ras*, *myb*, *jun*, *fos*, *abl* and *src.* The activities of these gene products as well as the activities of other oncogenes are described in J.D. Slamon et al. (Science 224:256-62, 1984).

Another example of anti-neoplastic activity includes the ability to regulate the life cycle of the cell, the ability to repress angiogenesis or tissue regeneration through the blockade or suppression of factor activity, production or release, the ability to regulate transcription or translation, or the ability to modulate transcription of genes under angiogenesis, growth factor or hormonal control. These activities are an effective therapy particularly against prostatic neoplasia and breast carcinomas. Additional anti-neoplastic activities include the ability to regulate the cell cycle for example by effecting time in and passage through S phase, M phase, G₁ phase or Go phase, the ability to increase intracellular cAMP levels, the ability to inhibit or stimulate histone acetylation, the ability to methylate nucleic acids and the ability to maintain or increase intracellular concentrations of anti-neoplastic agents.

The neoplastic disorder may be any disease or malady which could be characterized as a neoplasm, a tumor, a malignancy, a cancer or a disease which results in a relatively autonomous growth of cells. Neoplastic disorders prophylactically or therapeutically treatable with compositions of the invention include small cell lung cancers and other lung cancers, rhabdomyosarcomas, chorio carcinomas, glioblastoma multiformas (brain tumors), bowel and gastric carcinomas, leukemias, ovarian cancers, prostate cancers, osteosarcomas or cancers which have metastasized. Diseases of the immune system which are treatable by these compositions include the non-Hodgkin's lymphomas including the follicular lymphomas, Burkitt's lymphoma, adult T-cell leukemias and lymphomas, hairy-cell leukemia, acute myelogenous, lymphoblastic or other leukemias, chronic myelogenous leukemia, and myelodysplastic syndromes. Additional diseases treatable by the compositions include virally-induced cancers wherein the viral agent is EBV, HPV, HIV, CMV, HTLV-1 or HBV, breast cell carcinomas, melanomas and hematologic melanomas, ovarian cancers, pancreatic cancers, liver cancers, stomach cancers, colon cancers, bone cancers, squamous cell carcinomas, neurofibromas, testicular cell carcinomas and adenocarcinomas.

### Treatment Aids

Another embodiment of the invention is directed to aids for the treatment of human disorders such as infections, neoplastic disorders and blood disorders. Aids contain compositions of the invention in predetermined amounts which can be individualized in concentration or dose for a particular patient. Compositions, which may be liquids or solids, are placed into reservoirs or temporary storage areas within the aid. At predetermined intervals, a set amount of one or more compositions are administered to the patient. Compositions to be injected may be administered through, for example, mediports or in-dwelling catheters. Aids may further comprise mechanical controls or electrical controls devices, such as a programmable computer or computer chip, to regulate the quantity or frequency of administration to patients. Examples include both single and dual rate infusers and programmable infusers. Delivery of the composition may also be continuous for a set period of time. Aids may be fixed or portable, allowing the patient as much freedom as possible.

The following examples are offered to illustrate embodiments of the present invention, but should not be viewed as limiting the scope of the invention.

### Examples - Hemoglobinopathy Studies

### Example I Fetal Globin Production in Erythroid Progenitor Cells.

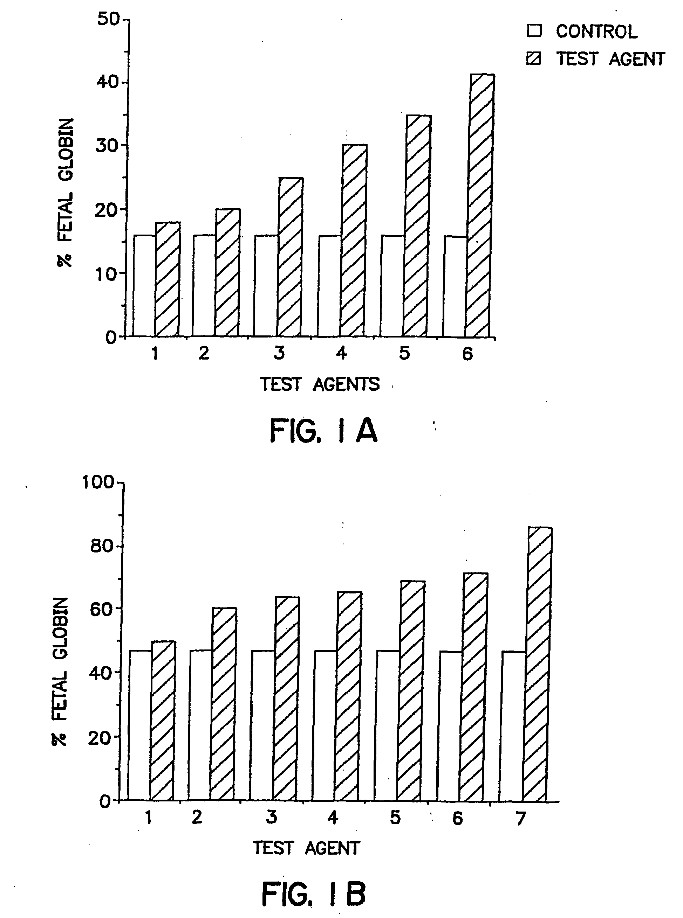

Fetal globin production was determined in ery-throid progenitor cells cultured in the presence or absence of six test compositions (Figure 1A). Blood was obtained from a normal individual and erythroid cells, purified and plated semi-solid medium containing the chemical compositions (lane 1) α-methyl-cinnamic acid; (lane 2) 3-phenylbutyrate; (lane 3) α-methylhydrocinnamic acid; (lane 4) 2,2-dimethylbutyric acid; (lane 5) phenoxyacetic acid; and (lane 6) thiophenoxyacetic acid. All test compositions were at a concentration of 0.2 mm and cultures were incubated for 10-14 days. As cultures were grown on semi-solid medium, neither the test composition or culture medium was replenished over time.

Proliferation of erythroid colonies (Bfu-e or CFU-e) is believed to be maximal in the presence of IL-3, SCF and EPO. Colonies from samples treated with growth factors alone (GM-CSF, SCF, EPO, IL-6, transferrin, insulin and IGF-1) are shown in white. Dark shaded bars 1-6 represent fetal globin produced in Bfu-e, treated with the respective composition in addition to the panel of maximal growth factors. Significant increases in fetal globin were observed with five of the six compositions tested (p < 0.05 paired t-test). Absolute levels of 16% γ-glonin (non-α globin) were observed in the controls, while 20-40% γ-globin (non-α globin) was expressed in Bfu-e cultured with the last five compounds.

Another experiment was performed using blood samples obtained from a patient with sickle cell anemia and seven different composition were tested and shown in Figure 1B: (lane 1) methoxyacetic acid; (lane 2) α-methylcinnamic acid; (lane 3) α-methylhydrocinnamic acid; (lane 4) 3-phenylbutyrate; (lane 5) 2,2-dimethylbutyrate; (lane 6) ; and (lane 7) thiophenoxyacetic acid. As shown, similar results were obtained. Increases in fetal globin were observed with all of the compositions tested (p < 0.05 paired t-test). Absolute levels of 48% of non-α globin were observed in the controls, while 51% to 83% of non-α globin was expressed in Bfu-e cultured with the compositions. Specifically, methoxyacetic acid showed a marginal 3% increase over controls; α-methylcinnamic acid showed a 12% increase over controls; α-methylhydro cinnamic acid produced an 18% increase over controls; 3-phenylbutyrate produced a 21% increase over controls; 2,2-dimethylbutyrate produced a 24% increase over controls; phenoxyacetic acid produced a 34% increase over controls; and thiophenoxyacetic acid produced a 38% increase over controls.

### Example 2 Erythroid Proliferation in the Presence of GM-CSF, SCF and EPO.

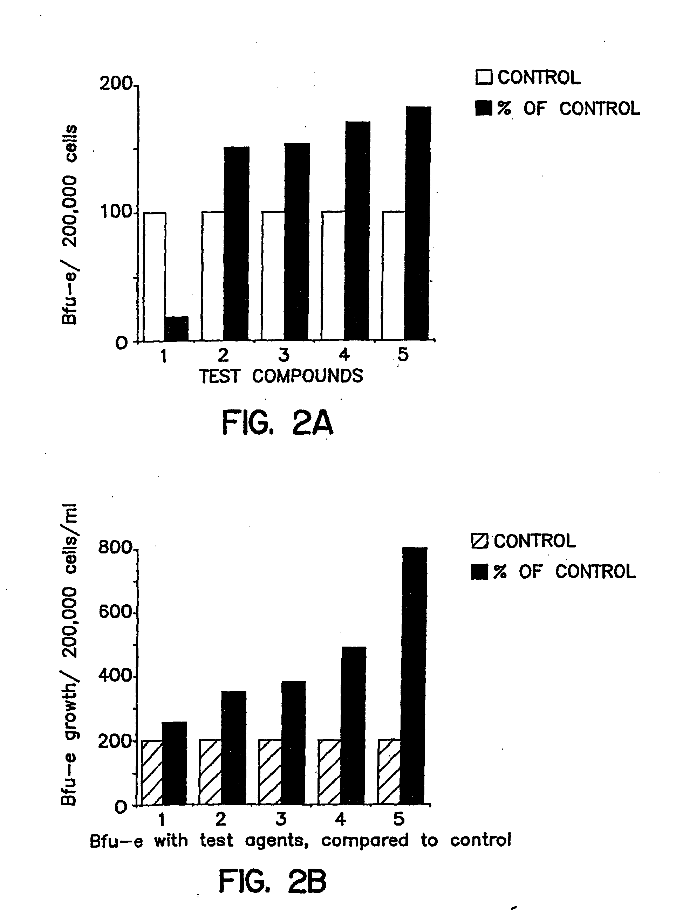

Erythroid cells were obtained, as described in Example 1, from a normal subject and cultured on semi-solid medium in the presence of a maximal amount of erythroid growth factors including IL-6, GM-CSF, SCF and EPO. Erythroid proliferation in the presence of these factors is expected to be maximal and, therefore, any increases observed would be significant. As shown in Figure 2A, colonies were grown in the presence or absence of: lane 1, 0.1 mM arginine butyrate (16% of control); lane 2, 0.5 mM 2-(4'-methoxyphenoxy) propionic acid; lane 3, α-methylhydrocinnamic acid; lane 4, 0.2 mM phenoxyacetic acid; and lane 5, 0.2mM 4-chlorophenoxy-2-propionic acid. As shown, all of these test compositions were able to further increase Bfu-e growth.

In a second experiment, erythroid progenitor cultures were established from a patient with sickle cell anemia and plated with maximal growth factors and the compositions. As shown in Figure 2B, responses to this set of compositions were similar to the effects observed with normal human Bfu-e: Lane 1, α-methylhydrocinnamic acid; Lane 2, 2-(4'-methoxyphenoxy)propionic acid; Lane 3, 2,2-dimethylbutyric acid; Lane 4, phenoxyacetic acid; and Lane 5 phenoxyacetic acid. Agents were tested at between 0.1 mM and 0.2 mM. Thus, effects could not be attributable solely to the influence of the erythroid growth factors added.

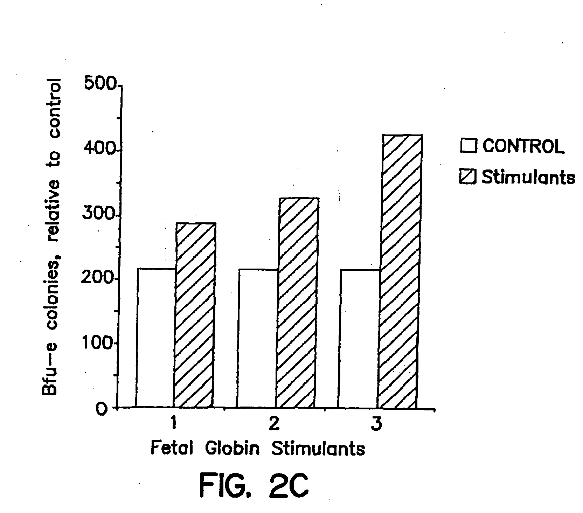

In a third experiment, human fetal liver cells were also tested. As fetal cells are known not to respond to the presence of the growth factors, any increases observed are highly significant. Erythroid progenitor cultures were established from fetal liver and plated with maximal amounts of growth factors. To these plates were added; lane 1, 2-(4'-methoxyphenoxy) propionic acid; lane 2, phenoxyacetic acid; and lane 3, α-methylhydrocinnamic acid; all at a concentration of 0.2 mM. As shown in Figure 2C, all plates showed increased proliferation of colonies and increases were determined to be significant (p <0.025).

In a fourth experiment, erythroid cells were obtained from a sickle cell patient. These cells were established in culture both with and without test a variety of test compounds. Cultures were incubated for 14 days in methylcellulose media with maximal concentrations of all hematopoietic growth factors (GM-CSF, SCF, EPO). Colonies were analyzed using fluorescent monoclonal antibodies against fetal globin to determine the proportion of erythroid cells that expressed fetal globin. These results are shown in Table I.

**Table I**

| **Proportion of Fetal Globin-expressing Cells in Culture** | | |
|---|---|---|
| Test Agent | Increase in γ-Globin Expressing Cells | |
| Controls (growth factors only) | | 40.7% |
| Phenoxyacetic Acid (0.2mM) | | 54.6% |
| 4-chloro-2-phenoxypropionic acid (0.5 mM) | | 59.7% |
| Fumaric Acid Monoethyl Ester | | 59.1% |
| Alpha-methylhydrocinnamic Acid | | 64.4% |
| 2,2-dimethylbutyric Acid | | 70.1% |

As shown, increases in fetal globin expressing cells were detected from 13.9% to 29.4% above control colonies from the same subject.

### Example 3 Pulse Administration of Phenoxyacetic Acids and Phenylakylacids.

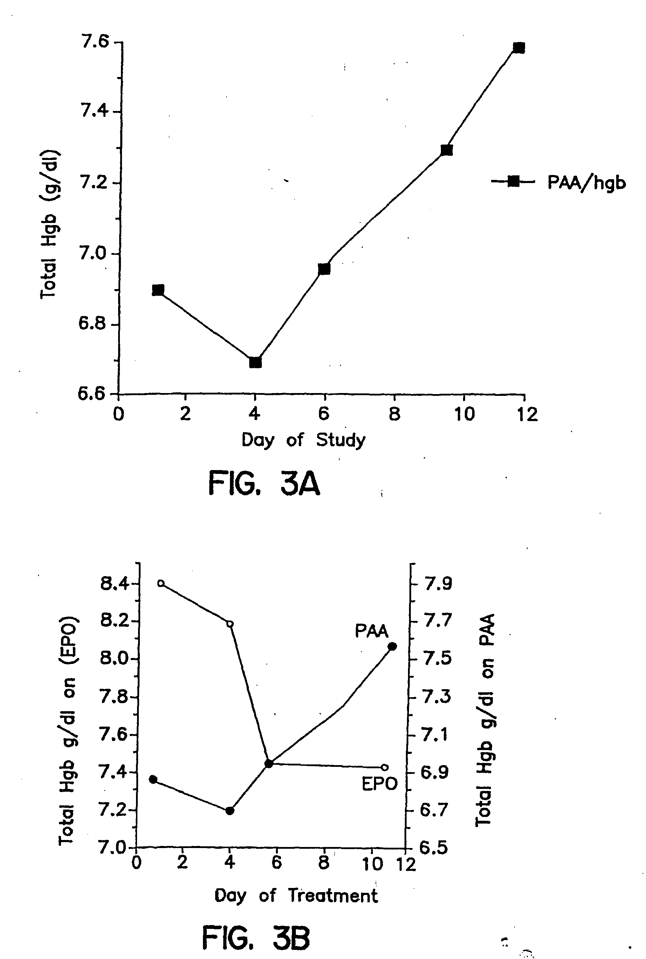

As discussed, compositions were more effective given as pulse therapy than as continuous therapy in baboons. Therapy was administered to anemic baboons who were being phlebotomized 5-10 ml/kg of blood per day on a continuous basis. EPO was administered daily at 300 U/kg/dose. Arginine butyrate was administered daily at 700 mg/kg/day for 5 days and phenoxyacetic acid at 500 mg/kg/day daily over 8 hours and at 1000 mg/kg/day for 24 hours/day daily for five days each. Total hemoglobin on these four continuous treatment schedules did not increase in the presence of anemia due to phlebotomy. As shown in Figure 3B, with continuous treatment with EPO, hemoglobin declined from 8.2 to 6.9 gm/dl. This same baboon showed no rise when treated with EPO at 300 units/kg 3 times per week. With continuous arginine butyrate treatment, hemoglobin remained essentially stable at 6.7 to 6.9 gram/dl. With continuous treatment with phenoxyacetic acid, hemoglobin levels remained stable at 7.0 gram/dl to 6.9 gram/dl during 8 hour therapy and declined from 6.8 to 4.5 when the drug was given for 24 hours per day for 5 days.

In contrast, administration of pulse doses of phenoxyacetic acid (2 oral doses per week, given as 500 mg/kg on days 1 and 4, resulted in rises in total hemoglobin from 6.9 to 7.6 gram/dl in one week (Figure 3A), a time frame which is consistent with the development of red blood cell precursors in the baboon. These results show that pulse therapy of phenoxyacetic acid (PAA) is superior to continuous delivery of this same compound. In a similar experiment, platelet counts increased from 240,000 to 767,000 with pulse administration of dihydrocinnamic acid. Treatment courses demonstrate that "less is more" or pulse therapy is surprisingly effective in an animal model very close to humans. Furthermore, the pulsed method of administering PAA was more effective than was administration of EPO at 300 U/Kg/dose, which is considered therapeutic at this dose for many forms of anemia.

### Example 4 Hematological Efficacy of Pulse Therapy.

Increased expression of fetal hemoglobin (Hb F) to adequate levels can ameliorate sickle cell disease. While any increase in Hb F can have ameliorating effects, and 8.5% Hb F prevents early mortality, 20% Hb F or more is generally considered necessary to ameliorate clinical symptomatology. This 20% Hb F level was achieved in 8% of subjects receiving hydroxyurea treatment on a national study, but remains a therapeutic goal for the majority of patients.

To determine if arginine butyrate can be used for therapeutic benefit, two low-dose regimens of butyrate were studied. Treatment were given intermittently either weekly, or in a pulse fashion, at 2-3 week intervals. Twenty-two treatment courses were examined with 300+ weeks of patient observation, including 18 months of home therapy.

Weekly therapy produced biochemical (Hb F) responses in 8/10 patients and increases in total Hb by 1-4 gram/dl above baseline levels, in 6/10. However, Hb F responses were in the 4-10% range, and total Hb levels declined to half the initial rise with continued weekly therapy.

In contrast, pulse therapy produced the desired hematologic responses in 8 of 12 sickle cell and beta thalassemia patients. Hb F levels increased 3-to 5-fold above baseline to greater than 20% in 4/6 adult sickle cell patients. A 55-year old patient reached 29% Hb F, and severe symptoms abated. The baseline Hb F level in responders was 2-6%. Total Hb levels increased by 2-3 grams/dl to >9.0 gm/dl in 4/6 severe thalassemia patients. The pulse regimen eliminated previous transfusion-dependency in thalassemia major and reduced iron stores, perhaps from mobilization of available stored iron into healthy red blood cells.

These results demonstrate that butyrates can be titrated to produce desired therapeutic goals for amelioration of beta globin disorders, and the fetal globin genes can be induced to high level expression at any age, from baseline levels of 2% or more.

### Example 5 Long Term Pulse Therapy.

Patients were treated by pulse therapy for 4-18 months with arginine butyrate. Patient's fetal globin genes were found to be reactivated to a degree that significantly compensated hematologically for defective adult β globin genes in greater than fifty percent of patients (8 out of 12). It is preferred to avoid prolonged treatment with high doses of butyrate, which cause cellular growth arrest in G1, and can enhance the accelerated apoptosis of beta thalassemia. Partial tolerance observed with long-term weekly therapy appears related to erythroid cell growth arrest and a decline in EPO levels as Hgb and Hct rise and depletion of available iron stores. Treatment with additional doses of iron supplements can overcome some of the anti-proliferative activities of butyrate *in vitro.* Time required on therapy has been only 25%-35% of the treatment days per month necessary for effective transfusion and iron chelation. This has improved the quality of life of some patients.

### Example 6 Comparison of Conventional verses Pulse Treatment.

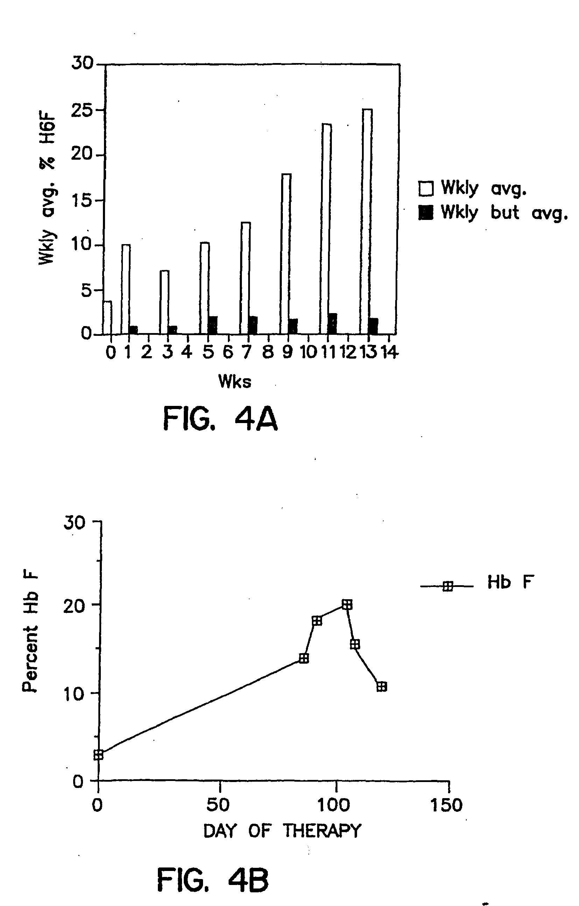

Conventional treatment: A 44-year old patient with sickle cell disease was tested with arginine butyrate at 750 mg/kg. Drug was administered by infusion for 10-16 hours per day and 5 days/week. As shown in Figure 4A, percent total HbF increased from 3% to about 15-18%. After 150 days, total HbF declined to about 10%.

Pulse treatment: Treatment of the same sickle cell patient as above was changed from weekly to pulse administration of arginine butyrate about one year later (Figure 4B). Arginine butyrate was administered by infusion at 500 to 750 mg/kg for 4 days for about 10-16 hours per day. After the 4 day regimen, drug was withheld for 10 days before treatment was repeated. This regimen was continued for about 5 months and percent total fetal hemoglobin increased to over 20%. This level was maintained until treatment was discontinued. Hemoglobin F levels above 20% are generally considered to be sufficient to eliminate symptoms associated with sickle cell disease.

### Example 7 Stimulation of Fetal Globin Production without Cellular Growth Arrest.

Stimulation of fetal globin production can ameliorate β-globin disorders, however butyrate can also induce cellular growth arrest. To determine if these potentially opposing actions can be balanced for therapeutic effect, two intermittent schedules of butyrate were compared in 21 treatment courses in 14 patients ages 3 to 55 years, with sickle cell disease or β thalassemia. An optimal dose was identified on an individual patient regimen and continued on a weekly basis in eleven patients. Providing 7 to 14 days without any drug exposure to facilitate proliferation of treated cells, an alternate week regimen or pulse therapy, was administered to twelve patients (see Tables II, III and IV).

A series of ten patients with sickle cell anemia and β-thalassemia were treated with continuous therapy with arginine butyrate. In 3 out of 4, transfusion requirement was diminished. Four patients had a decline or plateau following this initial rise in total hemoglobin. The mean peak in total HgB which was detectable in the ten patients over their baseline was 1.6 gm/dl.

**Table II**

| **Weekly Therapy** | | | | | | | |
|---|---|---|---|---|---|---|---|
| Age (yrs) | sex | Condition | F-retics | | Total Hgb | | |
| | | | weeks | Base | On | Increase | Comments |
| 34 | F | β IVS II-745 | 7 | 4 | 16 | 3 | six weeks therapy |
| 3 | M | β (-39-39) | 8 | 60 | 77 | ND | AIHA, trans. cont. |
| 4 | M | β IVS I, 1-6 | 40 | 56 | 75 | +4.3 | response declined |
| 40 | M | frameshift 5 | 5 | 0.5 | 1 | +1.0 | response declined |
| 42 | F | Hb SS | 24 | 12.7 | 35.3 | +2.0 | response declined |
| 25 | M | β-39,-39 | 8 | 76 | 68 | 1.5 | plateaued |
| 7 | F | β-39-87 | 16 | 64.7 | 67.3 | +2.3 | changed to pulse |
| 21 | F | HbS, β-39 | 6 | 4 | 20 | ND | transfusions cont. |
| 3 | F | HbE/? | 9 | 42 | 52.7 | ND | transfusions cont. |
| 31 | F | HbSS | 4 | 14 | 30.7 | +1.0 | response declined |

As shown in Table II, increases in total Hb occurred in some patients. However, in most cases hemoglobin levels declined to half of the initial increase above baseline when therapy was continued daily and weekly.

Patients were next administered a pulse therapy regimen. Each session comprised eight to twelve hours of nightly infusions. Patients received treatments for two to three nights followed by 7 to 21 days without any therapy. Total increases in Hgb were sustained as high as 3.0 g/dl above baseline levels with a mean increase of 2.1 g/dl in 8 out of 12 patients (p < 0.05, paired t-test, chi square). Importantly, these responses did not decline over time.

**Table III**

| **Pulse Therapy** | | | | | | | | | | |
|---|---|---|---|---|---|---|---|---|---|---|
| Age (yrs) | Sex | Cond. | F-cells | | | F-retics | | Percent HbF Inc. | | |
| | | | weeks | Base | On | Base | On | Base | On | Total Hgb |
| 44 | F | HbSS | 11 | 28 | 58 | 18 | 25 | 3 | 24 | +1.0 |
| 55 | F | HbSS | 28 | 42 | 56 | 18 | 36 | 6 | 29 | +1.0 |
| 30 | F | HbSS | 18 | 50 | 65 | 35 | 46 | 8 | 35 | +2.0 |
| 29 | M | HbSS | 15 | 23 | 59 | 12.6 | 24 | 6.9 | 22 | +2.0 |
| 34 | F | HbSS | 7 | 11 | 8 | 8 | 9.3 | 0.5 | 1.5 | ND |
| 18 | F | HbSS | 9 | 15 | 0.5 | 0.7 | 6.7 | 0.5 | 1.0 | ND |

**Table IV**

| **Pulse Therapy** | | | | | | | | | |
|---|---|---|---|---|---|---|---|---|---|
| Age (yrs) | Sex | Cond. | F-cells | | | Non-α/α* | | | Increase in |
| | | | Weeks | | Base | On | Base | On | Total Hgb |
| 25 | M | β-39 | | 7 | 100 | 100 | 0.4 | 0.9 | 3.0 |
| 7 | F | β-39-87 | | 68 | 29 | 100 | 0.6 | 1.1 | 3.6 |
| 27 | M | β IVS I-1 | | 4 | 100 | 100 | 0.5 | 0.9 | 2.1 |
| 26 | M | HbE/? | | 15 | 79 | 84 | 0.17 | 0.25 | 1.7 |
| 43 | M | frshft-5 | | 24 | 100 | 100 | 0.5 | 0.75 | |
| 17 | F | β IVS II | | 33 | 10 | 16 | 0.5 | 0.7 | ND |

| | | | | | | | | | |
|---|---|---|---|---|---|---|---|---|---|
| * = non-α:α globin chain synthesis ratios | | | | | | | | | |

As shown in Tables III and IV, pulse therapy results were significantly improved compared to weekly therapy. Percent HbF levels increased to above 20% for 4 of 6 patients treated and non-α/α globin chain ratios were at or near 1.0. These results are sufficient to overcome symptoms in the responsive sickle cell patients as well as in the same proportion of thalassemia patients (about 2/3).

Pulse regimens differ from other reported sickle cell therapy regimens in requiring smaller doses and less onerous delivery methods. Delivery in regimen one and regimen two is limited to 4 to 7 grams of butyrate per kilogram body weight per month while other therapy regimens require the delivery of 40 to 48 grams per kilogram body weight per month. Thus, the total amount of drug delivered is only ten to fifteen percent of the doses used in other treatment methods.

Benefits of the pulse treatment include reduced dose and reduced treatment time. Pulse treatments only require the delivery of 4 to 7 grams per kilogram body weight per month compared to 40 to 48 grams per kilogram body weight per month. Furthermore, pulse treatments only require 6 to 10 hour nightly infusions for a total of six to nine days per month while other methods require 24 hour infusions for 20-24 days per month. Surprisingly, pulse therapies avoid erythroid cell growth arrest, which is a problem with conventional therapies.

Weekly therapy produced biochemical increases in fetal globin expression in 8/11 patients and rises in total hemoglobin by a mean of 1.6 gm/dl in and 2.4 gm/dl in the 6 responsive patient (6/9 treated). However, hematologic effects frequently declined to half the initial rise when weekly treatment was continued Pulse therapy resulted in a 2- to 5-fold increase above baseline HbF levels to the desired 20% Hb F threshold, or higher, in 4/6 sickle cell patients and produced a further rise in total hemoglobin above baseline in 4/6 beta thalassemia patients. Transfusion-dependency was eliminated and iron stores were depleted in two patients with previously transfusion-dependent thalassemia major. No significant adverse side effects were observed with up to 17 months of home therapy and 350 weeks of patient treatment.

These results demonstrate that pulse administration of relatively low doses of a natural fatty acid can stimulate γ globin production to a therapeutic degree in two-thirds of patients with beta globin gene disorders.

Continuous 24 hour per day infusions of was administered in another study to 10 patients with sickle cell disease and beta thalassemia and produced rises in fetal globin in a lower proportion of patients, 40%. Many of these responses were not sustained.

Therefore, the same infusion rate given for 24 hours/day results in a lower response rate than does 8-16 hour/day treatment at the same infusion rate. A lower proportions of patients respond hematologically with continuous 24 hours therapy (1/10 patients) than respond to intermittent pulse therapy 8/10 patients. Hematologic responses are higher with pulse therapy (2-5) gram/dl above baseline levels compared to no responses in patients treated with 24 hour therapy.

### Example 8 Pulse Therapy Produces a Sustained Response.

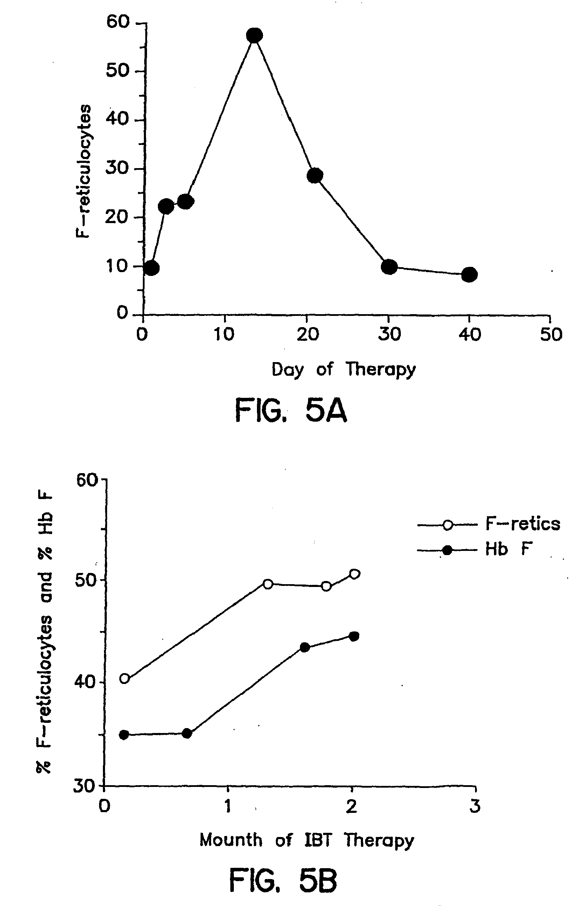

A comparison of continuous daily therapy and intermittent pulse therapy of with isobutyramide oral solution demonstrated that continuous daily therapy results in drug tolerance or a loss of drug effect. This is illustrated in an adult and in a child with sickle cell anemia who were receiving continuous daily isobutyramide oral solution at escalating doses from 25 to 300 mg/kg/day. The response of induction of fetal hemoglobin producing reticulocytes, or new red blood cells, rose and then declined back to baseline on continued therapy (Figure 5A). In contrast, in a 25 year old male patient with beta thalassemia treated with pulse therapy of isobutyramide oral solution (at 40 mg/kg dose) as 3 pulses per week, the F-reticulocytes increased progressively and did not decline (Figure 5B).

### Example 9 Effects of Continuous Therapy.

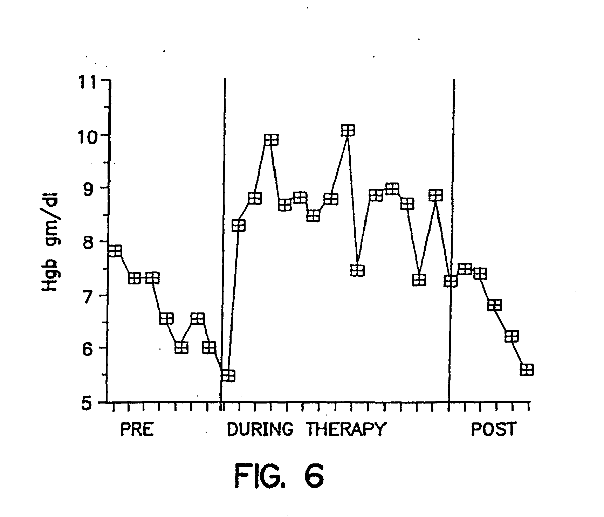

An example of the effects of continuous therapy is shown in a 4-year old child with beta thalassemia treated for 10 months with continuous therapy (Figure 6). Treatment period, the ten month duration of butyrate therapy, is shown between the vertical lines. A rise from a total hemoglobin from a baseline of 5.6 grams/dl to a peak of 9.9 gm/dl occurred, then plateaued at 8.0-8.8 gm/dl for 2 months. After 4 months of continuous weekly treatment, total hemoglobin levels declined to 7.5-8.4 gm/dl, suggesting a growth arrest affect or drug tolerance. When treatment was discontinued, total hemoglobin declined back to the patient's baseline of 5.6 gm/dl.

### Example 10 Examples of Pulse Therapy.

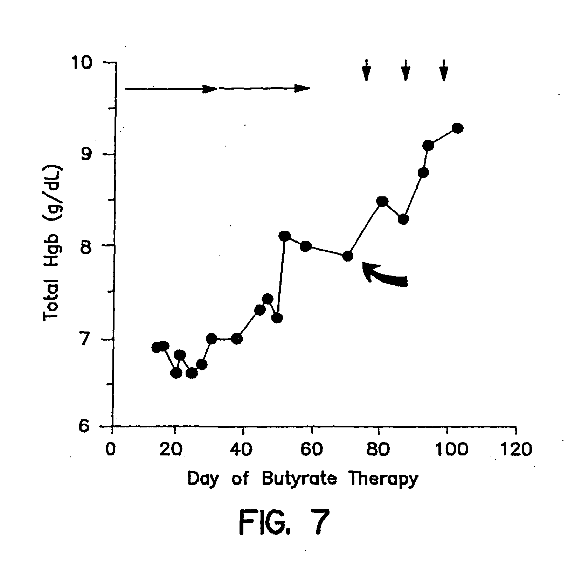

A plateau or even a decline in hematologic response has been commonly observed when butyrate therapy is given continuously. This is illustrated in a patient with β° thalassemia, who responded to weekly butyrate treatment with an increase in fetal globin synthesis and improved globin chain balance from 0.5 to 0.85 non-α to α-globin chain ratios (Figure 7). A 3-fold increase in γ globin mRNA was detected 24-36 hours after therapy. A 1-1.5 gram/dl rise in total hemoglobin followed the increase in γ globin synthesis and mRNA, but total Hgb then plateaued and even declined slightly (solid line). A decline in ferritin is also frequently observed (lower panel), even though the patient received single daily supplements.

When therapy was decreased to shorter pulses for 2-3 days on alternate weeks, total hemoglobin rose another 2 grams/dl. Increased iron supplements were required to support the active erythropoiesis, despite the presence of iron overload.

This figure illustrates the improved therapeutic effect between continuous therapy (to day 60) shown by the horizontal lines, and pulse therapy, shown by the vertical lines in a patient with beta thalassemia (Figure 7).

The therapeutic rise in total hemoglobin plateaued or declined on weekly therapy. With a change to pulse therapy, shown by the curved arrow, there was a further rise in total Hgb to 9.6 gm/dl, a level that no longer requires transfusions.

### Example 11 Reduced Infusion Rates Minimize Adverse Effects of Treatment.

Infusion rates were varied between 20 to 150 mg per kg per hour in several patients who have large proportions of circulating nucleated erythroblasts. Globin chain synthesis was assayed at different time points before and following drug infusions.

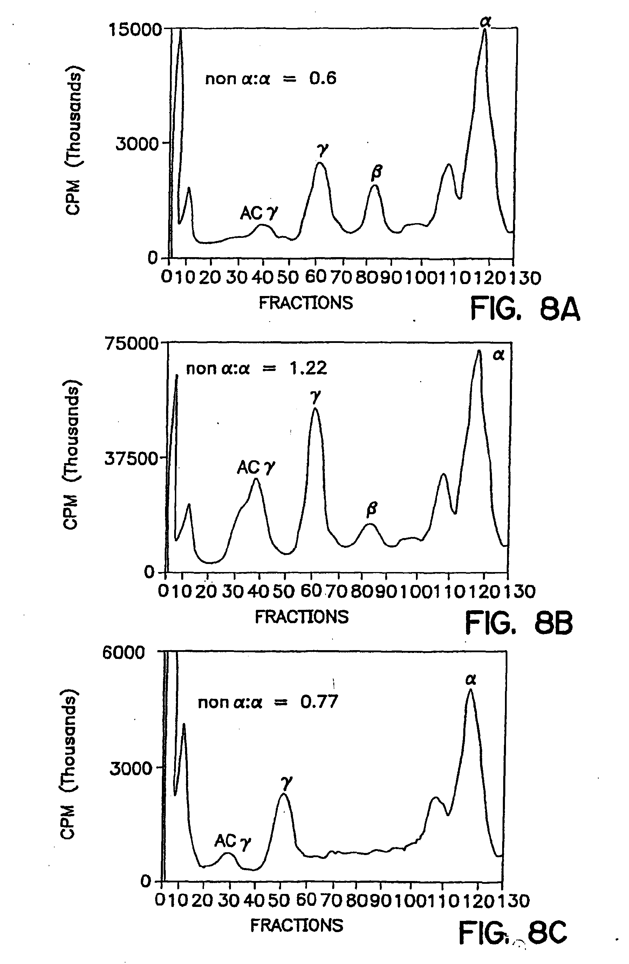

In a patient with β+ thalassemia, high doses of butyrate suppressed both γ and β globin chain synthesis (Figure 8B), causing an overall globin balance worse than the original baseline (Figure 8A). In contrast, lower infusion rates increased γ globin synthesis and corrected globin chain ratios close to a normal range.

Similar results were observed in another patient. Infusion at 150 mg per kg per hour completely suppressed β globin synthesis, although total globin chain balance was still improved (0.77 non-α globin to α globin ratio) over the patient's baseline level of 0.6 (Figure 8C). After infusion at moderate rates, normal globin chain ratios of 0.95 to 1.19 were observed at 12 hours. Thus, a reduced dose, while still stimulating fetal globin, is more effective in increasing total hemoglobin and hematocrit than doses typically used for continuous therapy.

### Example 12 Short Pulses of Butyrate do not Aggravate Apoptosis.

Peripheral blood from a patient with Hb-E beta thalassemia has 1200 NRBCs per 100 white blood cells which allows for rapid monitoring for apoptosis. This is demonstrated by the red stain which detects fragmented DNA. Apoptosis in cells after high-dose butyrate is demonstrated by red staining of most cells. After low-dose pulse therapy, no red-staining erythroblasts are observed. Short pulses of butyrate do not aggravate the tendency to accelerated apoptosis that is characteristic of thalassemic erythroblasts. A plateau or even a decline in hematologic response has been observed when butyrate therapy is given continuously.

This effect was observed in a patient with β° thalassemia, who responded to weekly pulse butyrate with an increase in fetal globin synthesis and im proved globin chain balance from 0.5 to 0.85 non-α/α globin chain ratios. A three fold increase in γ globin mRNA was detected 24 to 36 hours after therapy.

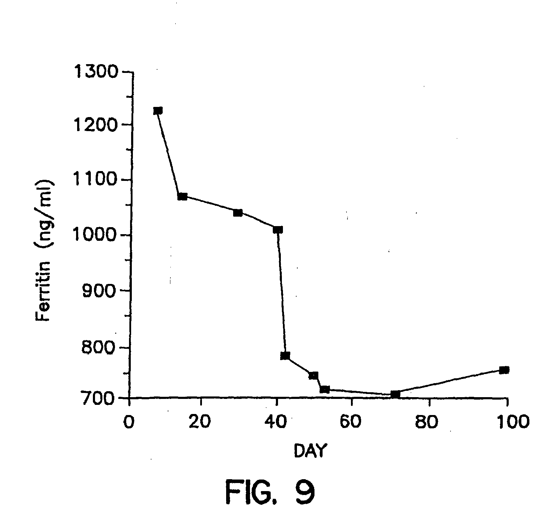

A 1.0 to 1.5 gram per deciliter rise in total hemoglobin followed the increase in γ globin mRNA synthesis for 30 to 50 days, but total Hgb plateaued and even declined slightly (Figure 7). A decline in ferritin was also frequently observed (Figure 9) even though the patient received single daily supplements.

When therapy was decreased to shorter pulses for two to three days on alternate weeks, total hemoglobin rose another two grams per deciliter for 50+ days (Figure 7). Increased iron supplements were required to support active erythropoiesis, despite iron overload (Figure 9).

### Examples - Viral Studies

### Example 1 Study Synopsis.

An intra-patient dose escalation study was designed to enroll up to 10 patients with EBV+ neoplasms. The major objectives are to evaluate the safety and pharmacology of arginine butyrate given in combination with ganciclovir (GCV). Because of the paucity of patients with EBV malignancies, this study employees intra- rather than inter-patient dose escalation. Because of the clinical experience already available with arginine butyrate, it is felt that this design will permit selection of a safe and effective dose regimen for a study in the future.

*In vitro*, peaks induction of EBV-TK plateaus at serum level of around 1 mM. In Phase I studies, doses of 1,000 mg/kg/day have resulted in serum levels of 0.5 to 0.75 mM. Therefore, dose levels up to 2,000 mg/kg/day, were evaluated in an attempt to reach serum levels of 1 mM. Treatment cycles were of 4 week duration, with 3 weeks on treatment and one week off. Patients were given the standard dose of GCV throughout each 21 day course of arginine butyrate, starting, however, on day 0. Arginine butyrate was given by continuous IV infusion at 500 mg/kg/day for 2 days, at 1,000 mg/kg/day for 2 days, at 1,500 mg/kg/day for 2 days, and thereafter at 2,000 mg/kg/day, or as tolerated, for the rest of the 21 day course. Cycle 2 was begun on Day 29. Patients were given a complete tumor evaluation after every course with subsequent cycles emitted unless disease progressed.

### Example 2 Patient Selection.

All patients selected had a microscopically documented neoplasm, (which if B-cell lymphoid, was monoclonal or oligoclonal, but not polyclonal), and EBV (+) as determined by immunohistochemistry [EBNA-2(+) and/or LMP-1(+)] and/or *in situ* hybridization [EBER + (*EcoR* I J] or [internal repeat + *BamFH* W(+)] or [EBNA-1 + (*BamM* K(+)]. Serology for EBV was obtained from each patient (and preferably from donor in the transplant setting), but need not be EBV(+). Patients with evaluable tumor are preferred. Patients with EBV-LPD who have not had prior cytotoxic chemotherapy or radiotherapy for this disease are preferred. Since there is no effective therapy for EBV-LPD, prior therapy was not required.

With respect to other EBV-associated malignancies, all patients had been refractory to at lease one combination of chemotherapy regimen, and were considered incurable by standard therapy. Patients should have recovered from prior chemotherapy or radiotherapy. At least 3 weeks elapsed since the last course of chemotherapy (6 weeks for nitrosoureas or mitomycin C). Minimum hematologic requirements were a platelet count greater than 30,000/mm³. Patients had a serum bilirubin less than 3.0 mg/dl, serum aminotransferase less than 2 times normal, and serum creatinine of less than 3.0 mg/dl. The calculated creatinine clearance was greater than 30 ml/minute. Patients had not had an acute myocardial infarction, or onset of atrial fibrillation within 6 months of study, were HIV negative and had the ability to give informed consent

### Example 3 Treatment Plan Including Dosing.

Eligible patients were provided central venous access via Porta-Cath or Ffickman line because of the hyperosmolarity of the arginine butyrate solution. On days 3 to 1 treatment with ganciclovir alone at standard doses (5 mg/kg IV over 1 hour bid) were given (unless already ongoing) and were continued through day 21. On day 1, infusion of arginine butyrate were begun at a total starting dose of 500 mg/kg/day to be infused continuously. In the absence of intolerable toxicity, dose escalation of arginine butyrate was according to the following scheme:
Level 1: 500 mg/kg/day IV (20.8 mg/kg/hr) for 2 days.
Level 2: 1000 mg/kg/day IV (41.6 mg/kg/hr) for 2 days.
Level 3: 1500 mg/kg/day IV (62.5 mg/kg/hr) for 2 days.
Level 4: 2000 mg/kg/day IV (83.2 mg/kg/hr) as tolerated and continued for the remainder of the 21 day treatment cycle.

Serum was obtained pretreatment and 4 hours into infusion on days 1, 3, 5, 7, 14 and 21 of treatment for measurement of butyrate levels. Serum samples were obtained at 30 minutes, 1 hour, 2 hours, 4 hours, 8 hours and 24 hours after discontinuation of infusion. Pretreatment and on days 2, 4, 6 and 8 of treatment, urine were collected for measurement of butyrate levels.

Arginine butyrate infusion was continued to and throughout day 21 and was discontinued as well GCV. Patients were examined on day 21 and serum was obtained for renal, hepatic and hematologic function. Repeat staging and biopsy (if tumor peripherally accessible) was done during the fourth week of each cycle. For patients that demonstrated stable disease, up to three cycles of treatment were given. If there is disease progression, or intolerable toxicity, the patient will be removed from the protocol. If a patient experiences a PR, up to six cycles may be given. If a patient experiences a CR, additional cycles may be given. Therapy in individual patients will be discontinued at any dose level if unacceptable drug-related toxicity occurs. Adverse drug reaction guidelines will be observed for reporting toxicity.

### Example 4 Clinical Pharmacology.

All patients entered undertook a pharmacokinetic study. This was to detect whether dose-dependent pharmacokinetics occur and whether drug levels or area under the curve correlate with toxicity. Blood samples of approximately 2 ml were collected in heparinized tubes. These samples were collected during the first cycle at pretreatment and on days 1, 3, 5, 7, 14 and at the end of infusion and at 30 minutes, 1 hour, 2 hours, 4 hours, 8 hours and 24 hours post-treatment. Plasma from these samples were separated by centrifugation, immediately frozen and stored at -20 ° C until analyzed.

Aliquots of urine (approximately 25 ml) were collected during the first cycle pretreatment, and days 2, 4, 6 and 8 of treatment. Urine was promptly frozen and stored at -20°C until analyzed.

At the discretion of the principal investigator tumor tissue samples from effusions, malignant ascites, blood or easily accessible tumor tissue were obtained pre-treatment and post-treatment (as standard care of routine evaluation and staging of patients with cancer).

### Example 5 Response Criteria.

All tumor measurements were recorded in centimeters and consisted of the longest tumor diameter and the longest perpendicular diameter. Liver size measurements were recorded at the xiphoid line and in the right MCL. Definitions of response:
Complete Emnse (CM Disappearance of all evidence of active disease for a minimum of 4 weeks. The patient must be free of any symptoms related to cancer. AU lytic lesions must have remineralized. No new lesions can have occurred.

Partial response (PR): 50% or greater decrease in the sum of the products of perpendicular diameters of measurable lesions for a minimum of 4 weeks. No simultaneous increase in size of any lesion or appearance of any pew lesion may occur. For tumors which can only be measured in one dimension, a greater than 50% shrinkage in largest dimension will quality as a PR.

Minor response (MR): Objective regression of measurable lesions of greater than 25%, but less than 50% for a minimum of 4 weeks. No simultaneous increase in size of any lesion or appearance of any new lesion may occur.

Stable disease (SD): Steady state of disease; less than minor response, without progression. There may be no appearance of new lesions or worsening of cancer-related symptoms. This state must be maintained for a minimum of eight weeks to qualify for disease stability.

Progressive disease (PD): Increase of at least 25% in the size of measurable lesions, or clearly progressive skeletal involvement manifested by new lytic lesions. For patients in partial remission, an increase of 25% or more in the sum of the products of the diameters of all measured tumors over that which was obtained at the time of maximum response or the appearance of any new lesions.

Relapse: The development of any new lesions or regrowth of old lesions in patients who achieved a complete response.

Duration of response (CR and PR) will be measured from the beginning of treatment to the first sign of progression or relapse. Duration of survival shall be determined from the time of the tissue diagnosis.

### Example 6 Toxicity and Pharmacology.

No organ specific toxicity was recorded by any principal investigator. There were also no drug-related deaths or life-threatening events (grade 4) due to drug administration. Pre-clinical data on the antineoplastic properties of arginine butyrate was divided into three areas: 1) induction by arginine butyrate of gene products in neoplastic cells which lead to differentiation of neoplastic cells, and thus, inhibition of their growth, or induction of gene products which lead to increased susceptibility to therapy directed to the induced gene product; 2) direct cytotoxic activity of arginine butyrate and cytotoxic activity in combination with chemotherapy to human tumor lines which display acquired drug resistance; 3) cytotoxic activity of combination therapy with arginine butyrate and ganciclovir on EBV+ tumors.

### Example 7 Induction of Differentiation and Gene Induction by Arginine Butyrate.

One current theory about the biology of cancer is that it represents an arrest in the development of the cell. The cancer cell remains in a relatively immature state and continues to be capable of growth and replication. A normal cell would mature fully into a functional bowel cell, blood cell, lung cell, etc, and would no longer be capable of growth. This process is called differentiation, and a cancer cell would therefore be a cell which has not differentiated fully (possibly as a result of oncogene activation). In theory, agents which can force the cancer cell to complete differentiation would render it incapable of further growth. Agents which can force differentiation of leukemia cells *in vitro* (cytosine arabinoside, retinoic acids, etc.) have recently been used effectively in patients to turn leukemia cells into normal, mature-appearing cells with a concordant slowing of the course of the disease.

Butyric acid is known for its ability to force differentiation of (human) erythroleukemia cells, chronic myelogenous leukemia cells (A. Novogrodsky et al., Cancer 51:9-14, 1983; Y.S. Chung et al., Cancer res. 45:2976-82, 1985), bowel cancer cells (A.B. Awad et al., Nutr. Cancer 16:125-33, 1991; L. Gamet et al., Int. J. Cancer 52:286-89, 1992), keratinocytes, salivary adenocarcinoma cells, pancreatic adenocarcinoma cells, melanoma cells, ovarian adenocarcinoma cells, medullary thyroid carcinoma cells, uterine malignancy cells, Burkitt lymphoma cells, germ cell malignancies, breast cancer cells, astrocytoma cells, hepatoma and neuroblastoma cells. The mechanisms whereby butyrate forces differentiation of tumors and an arrest in their growth are not fully understood, but butyrate's activity in increasing intracellular levels of cAMP, inhibiting histone acetylation, and inhibiting methylation of genomic DNA may be related to its differentiation effects. In many cases, this differentiation is accompanied by down-regulation of activated oncogenes in the tumors.

Butyric acid has not been tested extensively in humans because of the lack of an acceptable formulation. Clinical use of sodium butyrate require large doses because of the short half-life; results in salt overload and causes venous irritation.

A formulation with arginine was developed that prevents the salt overload, decreases venous irritation and possesses a longer half-life. The ability of arginine butyrate to induce differentiation has been evaluated and is measured by inhibition of growth, in some of the neoplastic cell types previously reported to be sensitive to (sodium) butyric acid, and found arginine butyrate to be as potent as butyric acid We have also extended that work to additional cell types and found Arginine Butyrate to induce differentiation in these new cell types. Table 1 lists the cell types tested and the minimum inhibitory concentration (MIC) of sodium butyrate (NaB) and arginine butyrate (ArgB) on these cell lines. The MIC is the lowest dose that prevents all growth over the 7 days that the cells are maintained in culture, that is the minimum dose that prevents an increase in cell number. While the MICs of NaB and ArgB was above 5 mM on normal cells, their MIC was below 0.5 mM on 4 of 8 tumor lines, and below 5 mM on all but one tumor line. The WC of ArgB and NaB were equivalent on all cell lines tested.

**Table 1**

| **MIC of Sodium Butyrate (NaB) and Arginine Butyrate (ArgB) On** | | | |
|---|---|---|---|
| **Normal Human and Human Tumor Cell Line Proliferation** | | | |
| Cells and Cell Lines | | Compound | MIC |
| **Non-Tumor:** | | | |
| | Fibroblasts (normal human) | NaB | > 5,000µM |
| | | ArgB | > 5,000µM |
| | Fibroblasts (mouse BALB/c-3T3) | NaB | > 5,000µM |
| | | ArgB | > 5,000µM |
| **Human Tumors:** | | | |
| | Rhabdomyosarcoma | NaB | 156µM |
| | | ArgB | 156µM |
| | Osteosarcoma (HOS) | NaB | 312µM |
| | | ArgB | 312µM |
| | Choriocarcinoma (JAR) | NaB | 156µM |
| | | ArgB | 156µM |
| | Glioblastoma Multiforme | NaB | N.D. |
| | | ArgB | 1,000µM |
| | Colonic Adenocarcinoma (HT29) | NaB | 2,000µM |
| | | ArgB | 2,000µM |
| | Gastric Carcinoma (AGS) | NaB | 2,000µM |
| | | ArgB | 2,000µM |
| | Myeloid Leukemia (HL60) | NaB | 78µM |
| | | ArgB | 78µM |
| | Erythroleukemia (K562) | NaB | > 5,000µM |
| | | ArgB | 1,250µM |

Human neuroblastoma cell line SK-N-MC was studied under different conditions. In this case, the cells were grown for 4 weeks with continuous exposure to sodium butyrate or arginine butyrate, and the cell number was quantitated at the end of this time. As shown in Table 2, a dramatic decrease in cell growth was observed after treatment with arginine butyrate, at levels of drug readily achieved in plasma. The expression of the oncogene n-*myc* transcript was decreased approximately fold in neuroblastoma cells treated with arginine butyrate (1 mM) compared to normal cells.

**Table 2**

| **Effect of Arginine Butyrate on Proliferation of Human Neuroblastoma** | | | |
|---|---|---|---|
| **Cell Line, SK-N-MC** | | | |
| | Compound | Concentration | Cell Number |
| | No treatment | -- | 1 x 10⁸ |
| | Arginine Butyrate | 0.05 mM | 1 x 10⁵ |
| | Arginine Butyrate | 1.0 mM | 1 x 10³ |

Similar growth inhibition studies but over 6 days rather than 4 weeks, were performed on 3 other neuroblastoma cell lines. All three cells lines showed dose response growth inhibition to the two concentrations, 0.5 and 1.0 mM of arginine butyrate tested (RFU = relative fluorescent units which is proportional to the number of live cells as described in Ginis and Faller, Anal. Biochem. 219:288-96, 1994). Arginine butyrate also induces apoptosis in SKNSH cells and the SY5Y neuroblastoma cell line.

The effects of arginine butyrate compounds on differentiation of several human tumor cell lines were evaluated by visual inspection and by histochemical and pathological staining:
Myeloid Leukemia (HL60): These cells responded to arginine butyrate analogues by differentiation along myeloid lines, acquiring a number of characteristics of mature, terminally differentiated neutrophils, including morphology identical to a mature neutrophil, expression of myeloperoxidase, specific esterases and the capacity to generate an oxidative burst.
Erythroleukemia (K562): These cells acquired the capacity to synthesize globin mRNA and protein and hemoglobin, like a normal red blood cell, and became red in color.

Colonic Adenocarcinoma (HT 29): Arginine butyrate produced absolute growth arrest by day 3-4, with no further growth out to 25 days. Inhibition of DNA synthesis (as measured by ³H-thymidine incorporation) occurred by 6 hours. Expression of the proto-oncogene c-*myc* was decreased by 2 hours. Morphologically, the treated cells developed a mature brush border, and increased expression of sucrase/isomaltase.

Gastric Carcinoma (AGS): These cells began to express mucin after treatment with Arginine Butyrate, a marker of mature gastric cell. Although HLA antigens are expressed on normal, fully differentiated cells, they are not expressed or are minimally expressed on most neoplastic cells. In this way neoplastic cells escape recognition by the cellular arm of the immune system and are able to survive.

HLA Induction: Arginine butyrate was tested for its ability to induce expression of HLA antigens in K562 cells, a human erythroleukemia cell line. Induction of HLA antigens in K562 cells has been shown to make these tumor cells susceptible to both humoral and cellular immune recognition (R.T. Maziarz et al., Cell Immunol. 130:329-338, 1990). As determined by a fluorescent activated cell sorter (FACS), HLA expression on K562 cells, measured before and after one hour of exposure to 1 mM arginine butyrate as described in Maziarz (Cell. Immunol. 130:329-38, 1990), showed HLA increased more than 20 fold.

The IL-2 receptor is a target for therapies currently under development. For example, antibodies directed to the IL-2 receptor and conjugated to a toxin, are currently being tested in the clinic, and IL-2, itself conjugated to a toxin, is currently being tested in the clinic. Arginine butyrate has been shown to increase expression of the IL-2 receptor. This increased expression might potentiate the therapies under development.

Cytotoxic activity of arginine butyrate as a single agent and in combination with chemotherapy on cell lines with acquired drug resistance. Attempts at biomodulation of current cytotoxic chemotherapeutic agents have been described both *in vitro* and clinically. Agents used, such as leucovorin to increase the activity of 5-fluorouracil for colon, breast and head and neck cancer, 13-cis-retinoic acid to enhance the cytotoxicity of interferon alpha in squamous cell carcinomas of the head, neck and cervix, and calcium channel blockers to augment cytotoxicity of adriamycin in myeloma and renal cell cancer have resulted in increased tumor response rates. The mechanisms of action of these biomodulating agents include overcoming acquired/intrinsic drug resistance, cellular differentiation, and prolongation of cytotoxic drug availability. Arginine Butyrate, as a single agent, has demonstrated a marked absence of major toxicities to organ function such as the hemopoietic, hepatic, renal, pulmonary, cardiac and neurologic systems in animals and humans, which would make this an ideal compound to combine with cytotoxic agents.

Arginine butyrate was tested for cytotoxic activity alone and in combination with chemotherapeutic agents that are associated with acquired drug resistance.

When various human malignant cell lines from breast, colon, lung, prostate, ovarian and lymphoid sources were exposed to arginine butyrate cytotoxic, i.e. cell death was at 72 hours was seen at all concentrations tested between 0.1-5.0 mM.

In clinical trails with arginine butyrate, the range of serum concentrations that have been attained were between 0.05 to 1.0 mM. Combinations of arginine butyrate and chemotherapeutic agents were examined at the lower arginine butyrate concentrations.

As shown in Tables 3 and 4, the combination of arginine butyrate and cisplatin or adriamycin resulted in significant enhancement of cytotoxicity to various human tumor cell lines above that seem by each compound alone.

**Table 3**

| **Survival Fraction After 72 hours Exposure: AB and/or CP** | | | | | | |
|---|---|---|---|---|---|---|
| Tumor Line | Ctl | 0.5AB | 1 CP | 2 CP | 0.5 AB/1CP | 0.5 AB/2CP |
| PC-3 | 1.0 | 0.67* | 0.92 | 0.49* | 0.39** | 0.26** |
| A549 | 1.0 | 0.92 | 0.77* | 0.54* | 0.33** | 0.15** |
| OvCar | 1.0 | 0.86** | 0.91 | 0.45* | 0.53** | 0.35** |
| | | | | | | |
| | Ctl | 1.0AB | 1 CP | 2 CP | 1.0 AB/1CP | 1.0 AB/2CP |
| CaCo | 1.0 | 0.60* | 0.87* | 0.53* | 0.22** | 0.15** |

**Table 4**

| **Survival Fraction After 72 Hour Exposure: AB and/or A** | | | | | | | |
|---|---|---|---|---|---|---|---|
| Tumor Line | Ctl | 0.5AB | .05AB | 0.1 A | 0.5 A | 0.5 AB/0.1A | 0.5AB/0.5A |
| A549 | 1.0 | 0.76* | 0.71* | 0.59* | 0.59* | 0.50 | 0.53 |
| PC-3 | 1.0 | 0.74* | 0.82* | 0.72* | 4.68* | 0.48** | 0.56** |
| MCF-7 | 1.0 | 0.74* | 0.89* | 0.73* | 0.69* | 0.61** | 0.63 |
| CaCo | 1.0 | 0.67* | 0.81* | 0.68* | 0.62* | 0,44** | 0.55** |
| SW900 | 1.0 | 0.92 | 0.95 | 0.82* | 0.76* | 0.61** | 0.67** |
| OvCar | 1.0 | 0.88* | 0.96 | 0.83* | 0.81* | 0.66** | 0.83 |

| | | | | | | | |
|---|---|---|---|---|---|---|---|
| * = Statistically significant vs. control (p<0.05). ** = Statistically significant vs. 1 CP, 2 CP, 0.1A or 0.5A as appropriate (p<0.05). | | | | | | | |

Growth inhibition was measured by the MTT assay. Survival fraction is calculated from at least 3 experiments, and differences in survival fraction are statistically significant P<0.05, using the Paired Two-Sample For Means T-Test. Ctl = control; 0.5 AB = 0.5 mM Arginine Butyrate; 1.0 AB = 1.0 mM Arginine Butyrate; 1CP = 1.0.µg/ml cisplatin; 2 CP-2.0 µg/ml cisplatin; 0.05A = 0.05 µg/ml adriamycin; 0.1 A = 0.1 µg/ml adriamycin; 0.5A = 0.5 µg/ml adriamycin. PC-3 = prostate cancer; A549 = non-small cell cancer; OvCar = ovarian cancer; CaCo = colon cancer; MCF-7 = breast cancer; and SW900 = non-small cell lung cancer.

These results show that Arginine Butyrate increases the cytotoxic activity of cisplatin in tumor cell lines from the lung, prostate, ovary and colon, and to a lesser extent, of adriamycin, upon human tumor cell lines from the lung, prostate, ovary, breast and colon. Other chemotherapeutic agents with non-overlapping mechanisms of antiproliferative activity such as 5-fluorouracil, taxol and interferon alpha have been evaluated with arginine butyrate. There was enhancement of cytotoxicity observed only at high concentrations of taxol exceeding 10 mM for breast and colon lines, but not in any other combination.

Since arginine butyrate enhanced the cytotoxicity of both cisplatin and adriamycin. in drug resistance human tumor cell line, a generalized mechanism of action may explain these observations. It has been shown that sodium butyrate can induce apoptosis in human tumors or tumor cell lines (L.A. Noorduyn et al., Histopathology 21:59, 1992). *In vitro* evidence for DNA fragmentation and cellular shrinkage of leukemia cell lines induced by arginine butyrate supports these observations. This was observed by flow cytometry after exposure of K562 to arginine butyrate alone, or in combination with other chemotherapeutic agents. DNA fragmentation was measured as described in Ginis and Faller (Anal. Biochem. 219:288-96, 1994) and survival was measured by the MTT assay. The addition of arginine butyrate increased their apoptotic activity to all of the following compounds: etoposide, camptothecin, adriamycin, cyclohexamide, and actinomycin D. Lack of enhancement with cyclophosphamide and actinomycin D was perhaps due to maximal DNA fragmentation by these two compounds alone at the concentrations selected.

Although it has been shown that both cisplatin and adriamycin can induce apoptosis, evidence for DNA fragmentation or cellular shrinkage by flow cytometry was not observed after exposure to arginine butyrate alone, or in combination with cisplatin or adriamycin in tumor cell lines A549,PC-3 and CaCo. These results suggest that at the lower concentration of arginine butyrate (0.1-0.5 mM), apoptosis is not the mechanisms of action for enhanced cytotoxicity observed when combined with cisplatin or adriamycin in these cell lines.

However, when induction of apoptosis by arginine butyrate in the human neuroblastoma cell line, SY5Y, was measured, significant DNA fragmentation was found with both 0.5 and 1.0 mM concentrations.

### Example 8 Cytotoxic Activity of Arginine Butyrate and Ganciclovir on EBV+ Tumor Cells.

Neoplastic Cells Epstein-Barr virus (EBV) is a 172 kb herpesvirus. Virus tropism is determined by complement receptor type 2 which mediates attachment of the envelope protein gp350/250 to B and some T lymphocytes, follicular dendritic cells and epithelial cells. *In vivo* EBV undergoes lytic replication after initial infection of oropharyngeal epithelia. The genome in linear form is duplicated, packaged into the viral capsid and extruded from the cell by budding or lysis. One hundred viral proteins are synthesized during this lytic stage of the virus life cycle.

In contrast, normal B-cells incubated with EBV *in vitro* are efficiently immortalized and develop into continuously growing lymphoblastoid cell lines (LCLs). The cellular events regulating these distinct outcomes are not understood. In immortalized cells, the genome circularizes, amplifies and replicates coordinate with, and dependent upon, cell division. Because no viral particles are produced, infection is considered to be latent. EBV persists in B-cells for life. Outgrowth of latently infected B-cells is prevented by T-cell immune surveillance. In immortalizing latent infection, only 11 gene products are detected, including 6 nuclear antigens (EBNA-1, -2, -LP, -3A, -3B, -3C), 3 membrane proteins (LMP-1, LMP-2A, LMP-2B) and two small, non-poly (A) RNAs (EBER-1, EBER-2). In EBV+ tumors such as Burkitt's lymphoma, neoplastic genetic events have often superseded the requirements for viral immortalizing functions, and gene expression may be limited to EBNA-I.

EBV is a common and worldwide pathogen. Childhood infection is asymptomatic. Fifty percent of individuals with delayed exposure develop a self-limited lymphoproliferative syndrome, infectious mononucleosis. EBV is also detected in 2 endemic tumors: African Burkitt's lymphoma (BL) and nasopharyngeal carcinoma (NPC). Recently, some T-cell and B-cell lymphomas, as well as 50% of Hodgkin's lymphomas have been found to contain EBV.

The development of a large patient population with T-cell dysfunction in the 1980's, caused by profound iatrogenic immunosuppression required to facilitate solid organ and bone marrow transplantation and by the AIDS epidemic, led to the discovery of two additional EBV-related illnesses; hairy leukoplakia, and B-lymphoproliferative disease (EBV-LPD).

In cells productively infected with EBV, EBV thymidine kinase (TK) is expressed. This makes these cells susceptible to treatment with ganciclovir (GCV). GCV is phosphorylated by EBV-TK and the resulting accumulation of phosphorylated GCV inhibits synthesis of DNA, which in turn leads to cell death. *In vivo* efficacy of GCV was established in hairy leukoplakia, a lytic EBV disease, wherein the production of virus leads to cell lysis. However, most EBV+ neoplasms are not lytic, rather they are latent infections. In the absence of viral production, TK is either not produced or produced at low levels and therapy with GCV is therefore, not an effective treatment.

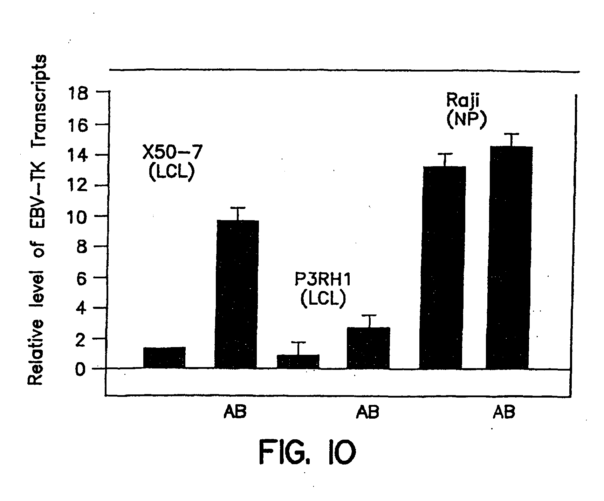

Because of arginine butyrate's ability to induce various genes, its ability to induce EBV-TK mRNA in human lymphoma cell lines was evaluated. Figure 10 provides bar graphs of the basal levels of TK and the levels following 12 hours of incubation with 3 mM arginine butyrate in 3 lymphoma cell lines: X50-7 a latent large cell lymphoma (B cell); P3RH1 an EBV B-cell producer line derived from Burkitt's lymphoma; and Raji a non-producer line derived from a Burkitt's lymphoma. EBV-TK levels were not increased in the EBV non-producer cell line, Raji, which has a high basal level of TK, but were increased approximately 4 fold in an EBV producer cell line and 10 fold in a non-producer cell line.

In some experiments sodium was used instead of arginine to neutralize butyric acid. In dose response experiments, levels of EBV-TK mRNA plateaus at around 1 mM to 3 mM sodium butyrate, inducing levels essentially equivalent to those induced by 1 mM arginine butyrate. However, arginine butyrate at 2 mM and 3 mM was cytocidal to most EBV+ and EBV- cell lines while 1 mM was cytostatic to some cell lines and permitted normal cell growth in others. Similarly, levels of GCV above 30 µM were generally cytocidal to both to EBV+ and EBV- cell lines. Cell lines used were X50-7, a latent EBV+ B-cell lymphoma; Akata, a producer EBV+ Burkitt's lymphoma; HPB-ALL, a T-cell line; and Nalm-6, an EBV- B-cell line.

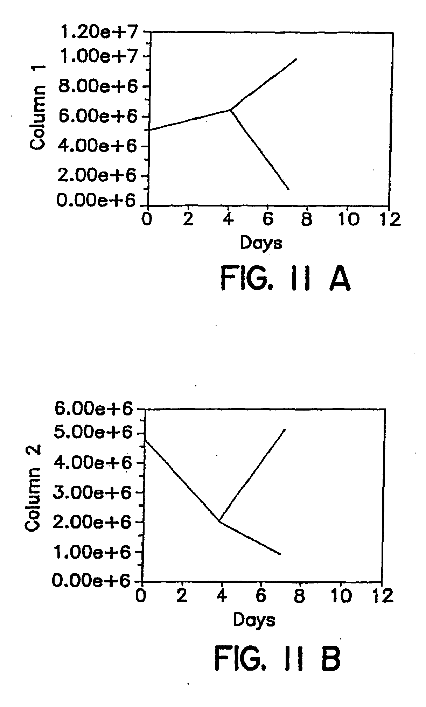

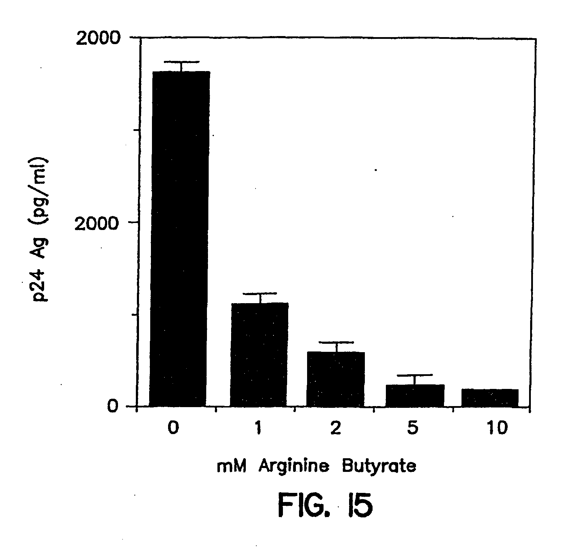

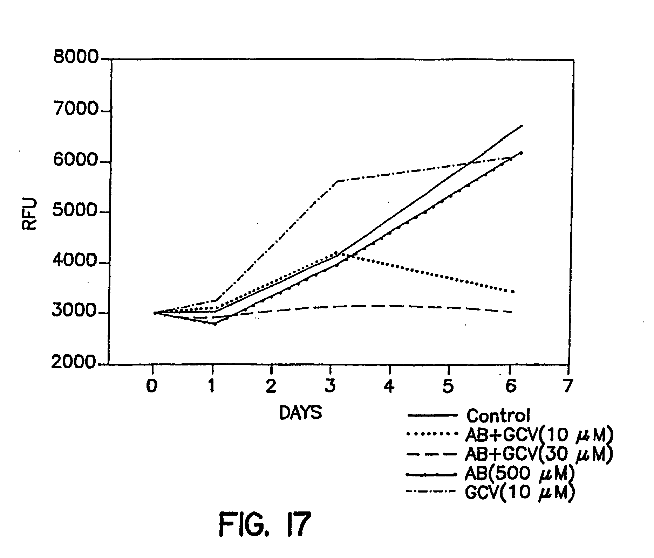

Levels at or below 1 mM arginine butyrate and 30 µM GCV were chosen to study in combination with each other for ability to kill EBV+ tumor cells. Figures 11-16 demonstrate the growth inhibition and cytotoxic effect of these agents used in combination at these doses, but not when used alone, on both EBV+ lymphoma lines (Figures 11a and 11b) and a fresh tumor isolate (Figures 15 and 17) of a patient concurrently treated with arginine butyrate and GCV.

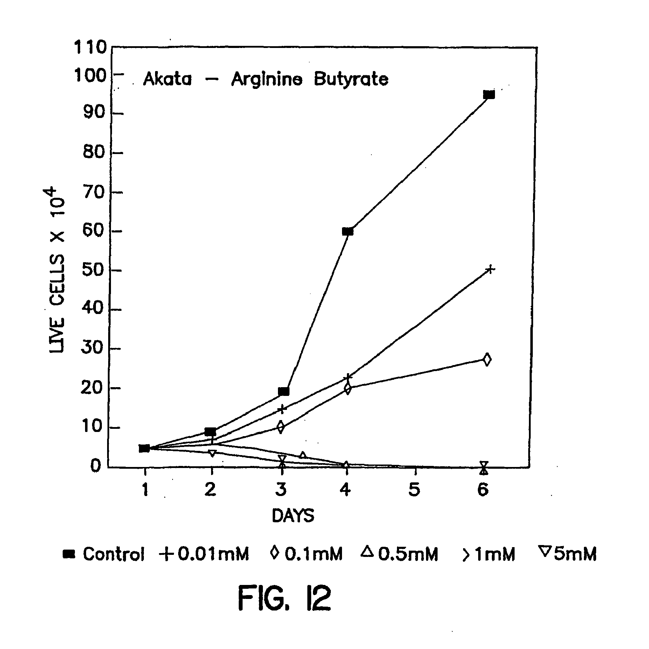

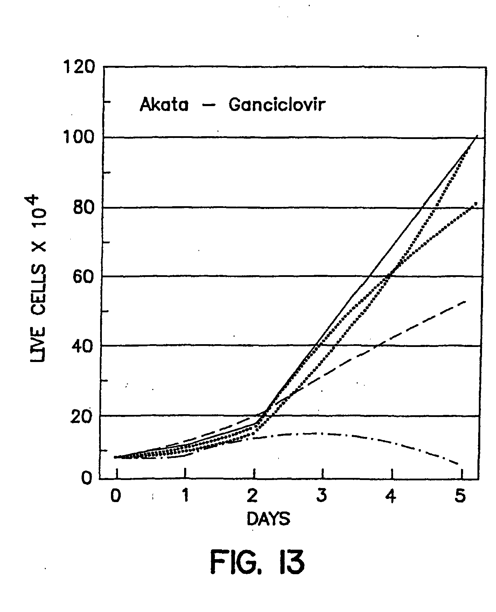

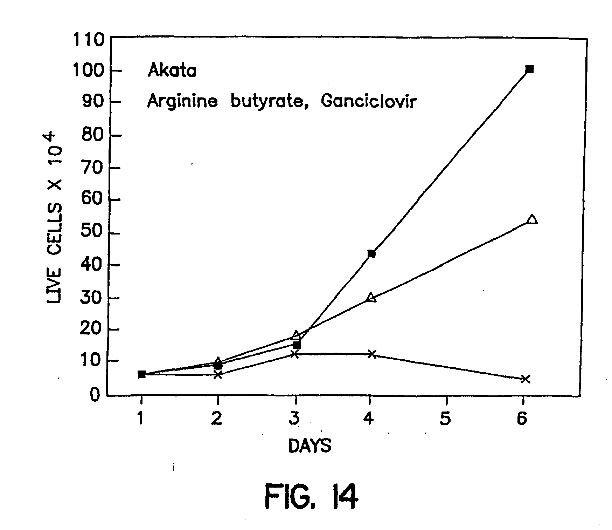

JY is an EBV+ B-cell LCL that is latent. The JY cells were incubated in the presence of 0.5mM (non-toxic) (Figure 11a), or 2.0 mM (non-toxic) arginine butyrate (Figure 11b) for 3 days, after which time Arginine Butyrate was washed out and GCV added to some plates (+) at 0.5 µM. Because of TK induction, JY cells were killed efficiently at the non-toxic doses of the individual drugs. EBV cells coincubated with arginine butyrate and then GCV at non-toxic doses were unaffected. Growth curves for the Akata cell line (a latent EBV+ cell LCL), grown in different concentrations of arginine butyrate are shown in Figure 12. Growth curves for the Akata cell line grown in the presence of different concentrations of GCV are shown in Figure 13. These cells were then cultivated in the presence or absence of non-toxic doses of arginine butyrate at 1.0 mM, 0.5 µM GCV or the combination of both arginine butyrate and GCV. Growth curves for the combination are shown in Figure 14. As can be seen, the combination was clearly much more toxic, and effective, that either agent alone.

There have been reports that butyrate induces activation/transcription from the HIV-1 promoter in transient-expression assays (C.A. Bohan et al., Virol. 172:573-83, 1989; C.A. Bohan et al., Biochem. Biophys. Res. Comm. 148:899-905, 1987; A. Yeivin et al., Gene 116:159-64, 1992; D.-C. Tang et al., Biochem. Biophys. Res. Comm. 169:141-47, 1992), and also that butyrate causes death of HIV-1 infected cells (M.R. Sadaie et al., Virol. 202:513-18, 1994). In addition, a recent report demonstrated that butyrate, in Kaposi's sarcoma-associated herpes virus patients, enhances Herpes virus type 6 gene expression (G. Miller et al., N. Engl. J. Med. 334:1292-98, 1996).

The effect of arginine butyrate on HIV p24 production could be easily studied in co-cultures of human PBMC and Ci11, an HIV infected cell line. As shown in Figure 15, the addition of arginine butyrate led to a significant decrease in p24 production. Despite these encouraging results with a more direct assay than those published previously, the potential risks to HIV+ patients of treatment with arginine butyrate have not been evaluated as these patients were excluded from the protocol (Figure 15). HIV p24 levels assessed by radio-immuno assay.

In summery, arginine butyrate has multiple properties each of which could presage success as an anti-cancer treatment. Arginine butyrate induces terminal differentiation in developmentally arrested human tumor cell lines. These now terminally mature cells are subject to the normal, restricted cell growth characteristic of fully differentiated cells and no longer proliferate in the uncontrolled fashion characteristic of malignant neoplastic cells.

Arginine butyrate induces cellular proteins which render the cell susceptible to immunotherapy or directed cytotoxicity. For example, one reason neoplastic cells escape immune surveillance is lack, of or under-expression of, HLA antigens. Arginine butyrate induces expression of HLA antigens on human tumor cells, making them susceptible to humoral and cellular immune recognition. Arginine butyrate also induces the expression of IL-2 receptors making them susceptible to directed cytotoxic therapy.

Arginine butyrate is directly cytotoxic to drug resistant cell lines. The addition of cisplatin or adriamycin produces a statistically significant increase in cytotoxic in the cell lines which are resistance to cisplatin or adriamycin alone.

Finally, arginine butyrate induces EBV-TK mRNA and combination treatment with arginine butyrate and GCV is cytotoxic to EBV+ lymphoma cell lines and fresh isolates of EBV+ lymphoma cells at serum levels achievable in humans.

### Example 9 Previous Human Experience.

Human experience with butyrate involves more than 25 patients with β-hemoglobinopathies, 9 patients in a traditional end-stage cancer patients and three compassionate patients with EBV+ lymphomas. Children with acute myelogenous leukemia and children with neuroblastoma have been treated with sodium butyrate. Doses of sodium butyrate ranging from 0.5 - 1.5 g/kg/day for 14 days, and 10 g/kg/day IV infusions were given without toxic side effects.

Eleven pediatric and twelve adult patients with β-hemoglobinopathies have been treated with IV arginine butyrate at a starting dose of 500 mg/kg/day for 2-7 weeks. Dose escalation at the rate of 250 mg/kg/day to a maximum of 2,000 mg/kg/day was permitted. There were no grade 3 or 4 toxicities and most patients showed response as judged by increase in fetal globin mRNA.

An inter-group dose-escalation study is being conducted in patients with refractory malignancies. There are 3 patients per group with the dose levels starting at 500 mg/kg/day, and increasing by 250 mg/kg/day per group, if toxicity permits. Doses are given by 8 hour IV infusion, daily, four 5 days times 2 weeks, with weekends off, for a total of 10 days of treatment per cycle.

In the first dose group, three patients with refractory neoplasms (breast, melanoma, adenocarcinoma of unknown primary) were enrolled and completed 1-2 cycles of arginine butyrate at a dose of 500 mg/kg/day given over 6-8 hours, by IV infusion, for 10 days over a 2 week period (F.M. Foss et al., Proc. ASCO 13:162, 1994; D.A. Sanders et al., Proc. ASCO, 1995; F.M. Foss et al., Proc. ASCO 13:162, 1995). There were no objective toxicities noted for the hepatic, renal, cardiac, pulmonary or hematopoietic systems. Two of the three patients experienced grade I nausea/vomiting which was controlled with oral anti-emetics. No objective tumor responses were noted.

In the second dose group, three patients (breast cancer, bowel cancer, Hodgkin's disease) have completed 2 cycles of arginine butyrate at a dose of 750 mg/kg/day for 10 days. One, of the three patients had grade I nausea/vomiting which was controlled with oral anti-emetics. One patient had a transient elevation of serum BUN, without elevation of serum Cr, and proteinuria measured by dipstick. These abnormalities resolved within 5 days of discontinuation of the drug. Otherwise, there were no other objective signs of organ toxicity. Two patients had stable disease after two cycles, with no progression. Tumors progressed after treatment was stopped. The patient with Hodgkin's disease had a partial response (>80%) reduction in tumor area) after the first cycle, with resolution of lymphadenopathy and leukocytosis, and complete resolution of back pain, with all narcotics stopped. He had further resolution of disease during the second cycle. Disease progression was noted one month after the second cycle was completed.

In the third dose group, three patients have been enrolled on study and are being treated at 1,000 mg/kg/day.

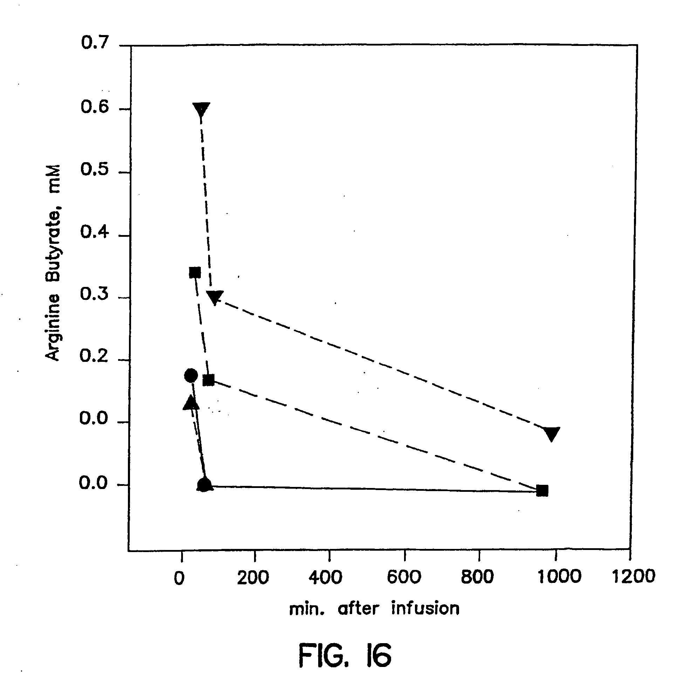

The pharmacokinetics in the cancer patients show dose response and are similar to the pharmacokinetics reported patients with β-hemoglobinopathies. Figure 16 shows a plot of the serum levels following the first dose for four patients in the oncology study.

Three cancer patients were treated on an emergency basis. Because of their significantly advanced condition they were treated up to 2,000 mg/kg/day by 12-24 hours. IV infusion, daily for 10 days, a dose felt to be safe and therapeutic. There were no untoward side effects noted in any of these patients.

Finally, two patients with post-transplant EBV-induced lymphomas have been treated on a compassionate basis witch IRB approval. Each patient is summarized below.

Patient one was a 38 year old male, 2 months post lung transplant. He was receiving cyclosporin to prevent impending rejection. He developed massive lymphadenopathy in mediastinum and hilum, with progressive respiratory failure. He was biopsy positive for EBV, and the tumor cells were monoclonal and of donor origin.

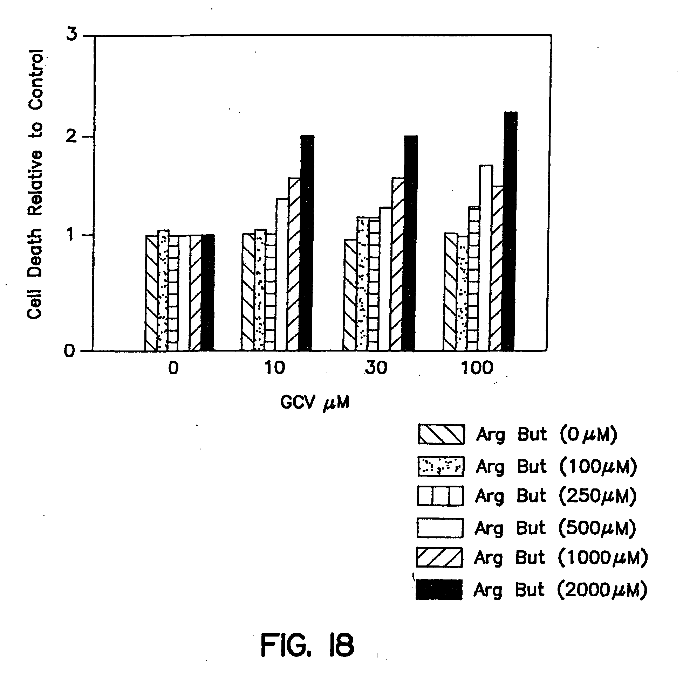

This patient's circulating tumor cells grew in culture and, since initiation of clinical treatment was urgent, were tested *in vitro* with arginine butyrate and GCV post start of *in vivo* therapy. As shown in Figures 17 and 18, treatment with arginine butyrate and GCV was cytotoxic to this patient's cells. The patient had been previously treated with and had no response to XRT, CHOP (developed severe neutropenia *and Aspergillus* septicemia), or α-IFN plus gamma globulin. Cell death in Figure 18 was measured by the uptake of ethidium homodimer.

He had been off all therapy, except ganciclovir, for 3 weeks when he started arginine butyrate at 1,000 mg/kg/day. He had been on GCV for 2 weeks, but showed no response. After starting arginine butyrate, he showed progressive resolution of tumor by radiography during the 21 day treatment. He eventually died from *Aspergillus* infection. At postmortem there was no evidence of lymphoma.

The second patient was a 23 year old female, 4 months post-allogeneic bone marrow transplant for leukemia. She was receiving cyclosporin to prevent rejection. She developed cervical, mediastinal and abdominal lymphadenopathy, with brain metastases. A biopsy was positive for EBV and tumor cells were monoclonal confirming the diagnosis of LPD.

She had been treated with and had no response to XRT, CHOP, or α-IFN plus gamma globulin. She had been off all therapy for 2 weeks and was cachectic, too weak to ambulate, paraplegic, in constant pain and had been admitted for terminal care, when she started arginine butyrate at 1,000 mg/kg/day and ganciclovir.

Within one day she began eating and walking. By day 14, she had experienced complete resolution of her systemic tumor and substantial resolution of CNS metastases, as documented by radiography. Her treatment continued for 21 days. Her CNS lesions are now stable by MRI. The residual findings may represent scarring, in which case this patient would be scored as a CR. She has been well and off therapy for longer than 9 months.

In total, more than 40 patients have been treated with arginine butyrate (>25 with β-hemoglobinopathies and >15 with neoplasia). Doses used were up to 2,000 mg/kg/day given over 8 to 24 hours per day, for more than 4 weeks in patients with β-hemoglobinopathies, with some of these patients having been treated for more than a year, and for up to 2 cycles of 10 days of treatment per cycle. Arginine butyrate has been well tolerated with no grade 3 and no grade 4 toxicities reported. Importantly, two patients with EBV-associated disease have been treated, not only safely, but with significant clinical improvement.

Other embodiments and uses of the invention will be apparent to those skilled in the art from consideration of the specification and practice of the invention disclosed herein. All U.S. patents, patent application and other documents cited herein, for whatever reason, are to be specifically incorporated by reference. The specification and examples should be considered exemplary only with the true scope and spirit of the invention indicated by the following claims.

The following clauses form part of the description
1. A pharmaceutical composition comprising an inducing agent to induce expression of a viral product in a virus-infected cell and an antiviral agent whose anti-viral activity is directed to the viral product expressed.
2. The composition of clause 1 wherein the inducing agent is a chemical compound of the structure R₁-R₂-R₃ wherein R₁ is CHₓ, Hₓ, NHₓ, OHₓ, SHₓ, COHₓ, CONTₓ, COOH or COSHₓ; R₂ is CHₓ or a branched or linear alkyl chain; R₃ is CONHₓ, COOH, COSHₓ, COOR₄, COR₄ or OR₄; R₄ is CHₓ, Hₓ, NHₓ, OHₓ, SHₓ or a branched or linear alkyl chain; and x is 0, 1, 2 or 3.
3. The composition of clause 1 wherein the inducing agent is propionic acid, butyric acid, isopropionic acid, 2,2- or 3,3-dimethyl or diethyl butyric acid, butyric acid ethyl ester or an amine, an amide, a salt or a combination thereof.
4. The composition of clause 3 wherein the amide is isobutyramide.
5. The composition of clause 3 wherein the salt is an arginine, lysine or choline.
6. The composition of clause 1 wherein the inducing agent is a chemical compound of the structure phenyl-R₅-R₆-R₇ wherein phenyl is a six carbon benzyl ring or a six carbon hydrogenated, hydroxylated or halogenated ring; R₅ is CHₓ, NHₓ, OHₓ or SHₓ; R₆ is CHₓ, NHₓ, OHₓ, SHₓ or a branched or linear alkyl chain; R₇ is CH_{x.} Hₓ, NHₓ, OHₓ, SHₓ, CONHₓ, COOH, COSHₓ, COOR₈, COR₈, or OR₈; R₈ is CHₓ, Hₓ, NHₓ, OHₓ, SHₓ or a branched or linear alkyl chain; and x is 0, 1, 2 or 3.
7. The composition of clause 6 wherein the inducing agent is methoxy acetic acid, methoxy propionic acid, methoxy butyric acid, phenoxy acetic acid, 4-chloro-2-phenoxy 2-propionic acid, 2- or 3-phenoxy butyric acid, phenyl acetic acid, phenyl propionic, acid, 3-phenyl butyric acid, ethyl-phenyl acetic acid, α-methyl-dihydrocinnamic acid, thiophenoxy acetic acid or an amine, an amide, a salt or a combination thereof.
8. The composition of clause 7 wherein the salt is an arginine, lysine or choline.
9. The composition of clause 1 wherein the anti-viral agent is an interferon, a nucleoside analog, an integrase inhibitor, a protease inhibitor, a polymerase inhibitor or a reverse transcriptase inhibitor.
10.The composition of clause 9 wherein the nucleoside analog is acyclovir (ACV), ganciclovir (GCV), famciclovir, foscarnet, ribavirin, zalcitabine (ddC), zidovudine (AZT), stavudine (D4T), larnivudine (3TC), didanosine (ddI), cytarabine, dideoxyadenosine, edoxudine, floxuridine, idozuridine, inosine pranobex, 2'-deoxy-5-(methylamino)uridine, trifluridine or vidarabine.
11. The composition of clause 9 wherein the protease inhibitor is saquinivir, ritonavir or indinavir.
12.A method for killing virus-infected cells by treating said cells with an inducing agent to induce expression of a gene product and an anti-viral agent whose anti-viral activity is directly proportional to activity of the gene product expressed in said cells.
13.The method of clause 12 wherein the virus-infected cells are lymphocytes.
14. The method of clause 12 wherein the virus-infected cells are treated *in vivo* by administering the inducing agent and the anti-viral agent to a patient.
15. The method of clause 12 wherein the virus-infected cells contain an integrated or episomal latent virus.
16. The method of clause 12 wherein the virus-infected cells contain a herpes virus, a human T cell or B cell leukemia virus, an adenovirus or a hepatitis virus.
17. The method of clause 16 wherein the herpes virus is an Epstein-Barr virus (EBV) or a Kaposi's-associated human herpes virus.
18. The method of clause 16 wherein the leukemia virus is a human immunodeficiency virus (HIV) or a human T-cell leukemia/lymphoma virus (HTLV).
19. The method of clause 12 wherein the gene product regulates viral gene expression.
20.The method of clause 12 wherein the gene product is a viral enzyme, an oncogene or proto-oncogene, a transcription factor, a protease, a polymerase, a reverse transcriptase, a cell surface receptor, a structural protein, a major histocompatibility antigen, a growth factor or a combination thereof.
21. The method of clause 20 wherein the viral enzyme is a thymidine kinase.
22. The method of clause 12 wherein the gene product expressed sensitizes the infected-cells to the anti-viral agent.
23. A method for treating a virus-induced, cell proliferative disorder comprised of administering an inducing agent and an anti-viral agent to a patient suspected of having said disorder wherein the inducing agent induces the expression of a gene product in proliferating cells associated with said disorder.
24. The method of clause 23 wherein the cell proliferative disorder is a neoplasia.
25. The method of clause 23 wherein the gene product is expressed from a latent virus.
26. The method of clause 5 wherein the latent virus is episomal or integrated into the genome of said proliferating cells.
27. The method of clause 26 wherein the latent virus is an RNA or a DNA virus.
28. The method of clause 27 wherein the latent virus is an Epstein-Barr virus (EBV), a Kaposi's-associated human herpes virus (human herpes virus 8), a human immunodeficiency virus (HIV), or a human T-cell leukemia/lymphoma virus (HTLV).
29. The method of clause 23 wherein the gene product is a viral enzyme, an oncogene or proto-oncogene, a transcription factor, a cell surface receptor, a protease, a polymerase, a reverse transcriptase, a major histocompatibility antigen, a growth factor or a combination thereof.
30. The method of clause 29 wherein the viral enzyme is a thymidine kinase.
31. The method of clause 23 wherein at least most of the viral genome is expressed.
32. The method of clause 23 wherein the gene product expressed sensitizes the proliferating cells to the anti-viral agent.
33. A method for treating a cell proliferative disorder in a patient comprised of administering an activator and an anti-viral agent to the patient wherein the activator is administered in an amount sufficient to activate expression of a latent virus episomal or integrated into proliferating cells of the patient.
34. The method of clause 33 wherein the activator is a phorbol ester, an oxidized phorbol ester, ceramide, bryostatin, an inducing agent or a combination thereof.
35. A method for treating an EBV-induced disorder comprised of administering an inducing agent, in an amount sufficient to induce expression of EBV-thymidine kinase, and a nucleoside analog to a patient suspected of having said disorder.
36. The method of clause 35 wherein the nucleoside analog is administered in an amount sufficient to kill EBV-infected cells.
37. A method for treating an HIV-induced disorder comprised of administering an inducing agent, in an amount sufficient to induce expression of HIV, and a nucleoside analog to a patient suspected of having said disorder.
38. A method for treating an HTLV-induced disorder comprised of administering an inducing agent, in an amount sufficient to induce expression of HTLV, and a nucleoside analog to a patient suspected of having said disorder.
39. A pharmaceutical composition comprising diethyl or dimethyl butyrate and a pharmaceutically acceptable carrier.
40. The composition of clause 39 wherein the dimethyl butyrate is 2,2-dimethyl butyrate.
41. A method for treating a human disorder comprising the administration of a substantially nontoxic chemical composition to a patient in a plurality of pulses with an interval between each pulse of greater than about 48 hours.
42. The method of clause 41 wherein the disorder is a cell proliferative disorder.
43. The method of clause 41 wherein the disorder is a cytopenia.
44. The method of clause 43 wherein the cytopenia is a red or white blood cell anemia, a leukopenia or a thrombocytopenia.
45. The method of clause 41 wherein the disorder is a hemoglobinopathy.
46. The method of clause 45 wherein the hemoglobinopathy is sickle cell anemia or thalassemia.
47. The method of clause 41 wherein the composition is not cytotoxic to the patient.
48. The method of clause 41 wherein the composition contains a chemical of the structure R₁-R₂-R₃ or R₁-C(O)-R₂-R₃ wherein R₁ is CHₓ, Hₓ, NHₓ, OHₓ, SHₓ, CONE COOH, COSHₓ, or COHₓ; R₂ is CHₓ or a branched or linear alkyl chain; R₃ is CHₓ, Hₓ NHₓ, OHₓ, SHₓ, CONHₓ, COOH, COSHₓ, COOR₄, COR₄ or OR₄; R₄ is CHₓ, Hₓ, NHₓ, OHₓ, SHₓ or a branched or linear alkyl chain; and x is 0, 1, 2 or 3; or an amine, an amide or a salt thereof.
49. The method of clause 48 wherein the chemical is propionic acid, butyric acid, isobutyramide, α-amino-n-butyric acid, fumaric acid monoethyl ester, dimethyl or diethyl butyric acid, o-benzoly lactate, n-dimethyl butyric glycine amide, o-dimethyl butyric lactate, diethyl butyric acid, isobutyric acid, isopropionic acid, trifluorobutyric acid, butyric acid ethyl ester or an arginine, lysine or choline salt thereof.
50. The method of clause 41 wherein the composition contains is a chemical of the structure phenyl-R₅-R₆-R₇ wherein phenyl is a six carbon benzyl or a six carbon hydrogenated, hydroxylated or halogenated ring; R₅ is CHₓ, NHₓ, OHₓ or SHₓ; R₆ is CHₓ, or a branched or linear alkyl chain; R₇ is CHₓ, Hₓ, NHₓ, OHₓ, SHₓ, CONHₓ, COOH, COSHₓ, COOR₈, COR₈ or OR₈; R₈ is CHₓ, Hₓ, NHₓ, OHₓ, SHₓ or a branched or linear alkyl chain; and x is 0, 1,2 gr 3; or an amine, an amide or a salt thereof.
51. The method of clause 50 wherein the composition contains methoxy acetic acid, phenyl acetic acid, phenoxy acetic acid, phenyl butyric acid, ethyl-phenyl acetic acid, cinnamic acid, hydrocinnamic acid, dihydrocinnamic acid, α-methyl-hydrocinnamic acid, 3-phenyl butyric acid, 4-chloro-2-phenoxy 2-propionic acid, 2- or 3-phenoxypropionic acid, 2, 2-dimethyl butyric acid, tributyrin, trifluoro butyric acid, thiophenoxy acetic acid, or an arginine, lysine or choline salt thereof.
52. The method of clause 41 wherein the composition is substantially non-proteinaceous.
53. The method of clause 41 wherein the plurality of pulses comprises from about 2 to about 10 pulses.
54. The method of clause 41 wherein the each pulse comprises delivery of a therapeutically effective pulsed dose of said composition over a period of time and the therapeutically effective pulsed dose comprises less of the composition than a therapeutic continuous dose administered over said period of time.
55. The method of clause 41 wherein the interval is from about 3 to about 21 days.
56. A method for expanding a cell population comprising treating said cell population with a composition in a plurality of pulses with an interval between each pulse of greater than about 48 hours.
57. The method of clause 56 wherein the cell population is obtained from bone marrow, stem cells, fetal liver, cord blood, yolk sac cells or combinations thereof.
58. The method of clause 56 wherein the expanded cell population is administered to a patient.
59. A method for treating a human disorder comprising the administration of a chemical composition to a patient in a plurality of pulses with an interval between each pulse which is greater than the *in vivo* half-life of the chemical composition.
60. The method of clause 59 wherein the interval between said each pulse is from about 10 to about 100 times the *in vivo* half-life of the chemical composition.
61. The method of clause 59 wherein the each pulse is administered periodically over a period of from about I to about 6 days.
62. The method of clause 59 wherein the each pulse is administered in a pluraity of doses over a period of from about 4 hours to about 4 days.
63. A method for treating a human disorder comprising the administration of a composition to a patient in a plurality of pulses wherein each pulse comprises delivery of a therapeutically effective pulsed dose of said composition followed by a period of no delivery wherein the therapeutically effective pulsed dose is less than a therapeutic continuous dose of said composition.
64. The method of clause 63 wherein the therapeutically effective pulsed dose is less than half of the therapeutic continuous dose.
65. The method of clause 63 wherein the therapeutically effective pulsed dose is less than a fifth of the therapeutic continuous dose.
66. The method of clause 63 wherein the composition is substantially undetectable in the patient prior to delivery of said each pulse.
67. The method of clause 63 wherein treatment increases the number of circulating red blood cells as determined from peripheral blood cell counts.
68. The method of clause 63 wherein treatment stimulates the number of circulating platelet cells or white blood cells as determined from peripheral blood cell counts.
69. A method for treating a globin disorder comprising the administration of a chemical composition to a patient in a plurality of pulses wherein each pulse comprises delivery of a therapeutic pulsed dose of said composition followed by a period of no administration of greater than about 48 hours.
70. The method of clause 69 wherein the composition is arginine butyrate, the therapeutically effective pulsed dose is from about 3 to about 10 g/kg/month.
71. The method of clause 69 wherein the therapeutically effective pulsed dose is delivered by infusion for between about 4 to about 16 hours per day for between about 1 to about 6 days.
72. The method of clause 69 wherein treatment stimulates expression of a fetal globin gene.
73. The method of clause 69 wherein treatment stimulates proliferation of erythroblasts or other erythroid or myeloid progenitor cells.
74. The method of clause 69 wherein treatment does not induce apoptosis of erythroblasts.
75. The method of clause 69 wherein the composition is administered by injection, infusion, instillation or oral ingestion.
76. The method of clause 69 wherein the therapeutically effective dose is that amount of the composition that specifically stimulates expression of a fetal globin gene or proliferation of fetal globin-expressing cells.
77. The method of clause 69 wherein the period of no administration is at least 10 times longer than the in vivo half-life of an active component of said composition.
78. The method of clause 69 wherein the therapeutically effective pulsed dose is less than half of a therapeutic continuous dose of said composition.
79. The method of clause 69 wherein the period of no administration is from about 6 to about 21 days.
80. The method of clause 69 further comprising the step of administering a second composition to the patient.
81. The method of clause 80 wherein the second composition is administered to the patient in pulses.
82. The method of clause 80 wherein the second composition contains erythropoietin and said second composition is administered to the patient prior to the administration of the chemical composition.

## Claims

1. A pharmaceutical composition comprising an inducing agent to induce expression of a viral product in a virus-infected cell and an anti-viral agent whose anti-viral activity is directed to the viral product expressed.

2. A composition according to claim 1 wherein the inducing agent is butyric acid, isopropionic acid, 2,2- or 3,3-dimethyl or diethyl butyric acid, butyric acid ethyl ester or an amine, an amide, a salt or a combination thereof.

3. A composition according to claim 1 or claim 2 wherein the anti-viral agent is an interferon, a nucleoside analog, an integrase inhibitor, a protease inhibitor, a polymerase inhibitor or a reverse transcriptase inhibitor.

4. A composition for use in killing virus-infected cells wherein said composition comprises an inducing agent to induce expression of a gene product that sensitizes the proliferating cells to an anti-viral agent, and the anti-viral agent whose anti-viral activity is directly proportional to activity of the gene product expressed in said cells.

5. A composition according to claim 4 wherein the virus-infected cells are lymphocytes.

6. A composition according to claim 4 or claim 5 wherein the gene product expressed is a viral enzyme, an oncogene or proto-oncogene, a transcription factor, a cell surface receptor, a protease, a polymerase, a reverse transcriptase, a major histocompatibility antigen, a growth factor or a combination thereof.

7. A composition according to any of claims 4 to 6 wherein the inducing agent is a phorbol ester, an oxidized phorbol ester, ceramide, bryostatin, or a combination thereof.

8. A composition according to any of claims 4 to 7 wherein at least most of the viral genome is expressed.

9. A composition for use in treating an EBV-induced disorder comprising an inducing agent, administered in an amount sufficient to induce expression of an EBV-thymidine kinase, and a nucleoside analog administered to a patient suspected of having said disorder in an amount sufficient to kill EBV-infected cells.

10. A composition for use in treating a HTLV-induced disorder comprising an inducing agent, administered in an amount sufficient to induce expression of HTLV, and a nucleoside analog administered to a patient suspected of having said disorder in an amount sufficient to kill HTLV-infected cells.

11. A pharmaceutical composition comprising diethyl or dimethyl butyrate.

12. A composition according to claim 11 wherein the diethyl or dimethyl butyrate is 2,2- or 3,3-dimethyl or diethyl butyric acid.

13. A composition for use in treating a human disorder comprising a substantially nontoxic chemical composition which is administered to a patient in a plurality of pulses with an interval between each pulse of greater than about 48 hours.
